# FASCICULE DE BREVET EUROPEEN

(11) **EP 1 814 879 B1**
(45) Date de publication et mention de la délivrance du brevet: **24.09.2008**
(21) Numéro de dépôt: 05814795.0
(22) Date de dépôt: 07.11.2005
(51) Int. Cl.: C07D 417/12, A61K 31/5377, A61K 31/496

(54) **DERIVES DE 4,7-DIOXOBENZOTHIAZOLE-2-CARBOXAMIDES, LEUR PREPARATION ET LEURS APPLICATIONS THERAPEUTIQUES**
DERIVATE VON 4,7-DIOXOBENZOTHIAZOL-2-CARBONSÄUREAMIDEN, VERFAHREN ZU DEREN HERSTELLUNG UND DEREN THERAPEUTISCHE ANWENDUNGEN
DERIVATIVES OF 4,7-DIOXOBENZOTHIAZOLE-2-CARBOXAMIDES, PREPARATION METHOD THEREOF AND THERAPEUTIC USES OF SAME

(30) Priorité: 05.11.2004 FR 0411802
(43) Date de publication de la demande: 08.08.2007
(73) Titulaire: Societe de Conseils de Recherches et d'Applications Scientifiques (S.C.R.A.S) SAS, 75016 Paris (FR)
(72) Inventeur: GALCERA CONTOUR, Marie-Odile, F-91070 Bondoufle (FR); PREVOST, Grégoire, F-92160 Antony (FR); SIDHU, Alban, F-91940 LES ULIS (FR)
(74) Mandataire: Bourgouin, André
(86) Numéro de dépôt international: PCT/FR2005/002763
(87) Numéro de publication internationale: WO 2006/051202

(56) Documents cités:
- WO-A-03/055868

## Description

La présente invention a pour objet des nouveaux dérivés de 4,7-dioxobenzothiazole-2-carboxamides, lesquels inhibent les phosphatases cdc25, en particulier la phosphatase cdc25-C.

Le contrôle de la transition entre les différentes phases du cycle cellulaire durant la mitose ou de la méiose est assuré par un ensemble de protéines dont les activités enzymatiques sont associées à des états différents de phosphorylation. Ces états sont contrôlés par deux grandes classes d'enzymes : les kinases et les phosphatases.

La synchronisation des différentes phases du cycle cellulaire permet ainsi la réorganisation de l'architecture cellulaire à chaque cycle dans l'ensemble du monde vivant (microorganismes, levure, vertébrés, plantes). Parmi les kinases, les kinases dépendantes des cyclines (CDKs) jouent un rôle majeur dans ce contrôle du cycle cellulaire. L'activité enzymatique de ces différentes CDKs est contrôlée par deux autres familles d'enzymes qui travaillent en opposition (Jessus et Ozon, Prog. Cell Cycle Res. (1995), 1, 215-228). La première regroupe des kinases telles que Weel et Mik1 qui désactivent les CDKs en phosphorylant certains acides aminés (Den Haese et coll., Mol. Biol. Cell (1995), 6, 371-385). La seconde regroupe des phosphatases telle que Cdc25 qui activent les CDKs en déphosphorylant des résidus tyrosine et thréonine de CDKs (Gould et coll., Science (1990), 250, 1573-1576).

Les phosphatases sont classifiées en 3 groupes : les sérines/thréonines phosphatases (PPases), les tyrosines phosphatases (PTPases) et les phosphatases à double spécificité (DSPases). Ces phosphatases jouent un rôle important dans la régulation de nombreuses fonctions cellulaires.

En ce qui concerne les phosphatases cdc25 humaines, 3 gènes (cdc25-A, cdc25-B et cdc25-C) codent pour les protéines cdc25. De plus, des variants issus de splicing alternatif du gène cdc25B ont été identifiés : il s'agit de cdc25B1, cdc25B2 et cdc25B3 (Baldin et coll., Oncogene (1997), 14, 2485-2495).

Le rôle des phosphatases Cdc25 dans l'oncogénèse est maintenant mieux connu et les mécanismes d'action de ces phosphatases sont illustrés en particulier dans les références suivantes : Galaktionov et coll., Science (1995), 269, 1575-1577 ; Galaktionov et coll., Nature (1996), 382, 511-517 ; et Mailand et coll., Science (2000), 288, 1425-1429.

En particulier, la surexpression des différentes formes de cdc25 est maintenant reportée dans de nombreuses séries de tumeurs humaines :
- Cancer du sein : cf. Cangi et coll., *Résumé 2984*, *AACR meeting San Francisco,* 2000) ;
- Lymphomes : cf. Hemandez et coll., Int. J. Cancer (2000), 89, 148-152 et Hemandez et coll., Cancer Res. (1998), 58, 1762-1767 ;
- Cancers du cou et de la tête : cf. Gasparotto et coll., Cancer Res. (1997), 57, 2366-2368.

Par ailleurs, le groupe de E. Sausville rapporte une corrélation inverse entre le niveau d'expression de cdc25-B dans un panel de 60 lignées et leurs sensibilités aux inhibiteurs de CDK, suggérant que la présence de cdc25 puisse apporter une résistance à certains agents antitumoraux et plus particulièrement aux inhibiteurs de CDK (Hose et coll., Proceedings of AACR, Abstract 3571, San Francisco, 2000).

Parmi d'autres cibles, l'industrie pharmaceutique recherche donc à présent des composés capables d'inhiber les phosphatases Cdc25 afin de les utiliser notamment comme agents anti-cancéreux.

Les phosphatases Cdc25 jouent également un rôle dans les maladies neurodégénératives telles que la maladie d'Alzheimer (cf. Zhou et coll., Cell Mol. Life Sci. (1999), 56(9-10), 788-806 ; Ding et coll., Am. J. Pathol. (2000), 157(6), 1983-90 ; Vincent et coll., Neuroscience (2001), 105(3), 639-50) de sorte que l'on peut aussi envisager d'utiliser des composés possédant une activité d'inhibition de ces phosphatases pour traiter ces maladies.

Un autre problème auquel s'adresse l'invention est la recherche de médicaments destinés à prévenir ou traiter le rejet de greffes d'organes ou encore à traiter des maladies auto-immunes. Dans ces désordres / maladies, l'activation non appropriée des lymphocytes et des monocytes/macrophages est impliquée. Or les médicaments immunosuppresseurs connus à ce jour ont des effets secondaires qui pourraient être diminués ou modifiés par des produits ciblant spécifiquement les voies de signalisation dans les cellules hématopoïétiques qui initient et maintiennent l'inflammation.

L'invention a tout d'abord pour objet de nouveaux inhibiteurs de phosphatases cdc25 (en particulier de la phosphatase cdc25-C), lesquels sont des dérivés de 4,7-dioxobenzothiazole-2-carboxamides et répondent à la formule générale **(I)** définie ci-après. Compte tenu de ce qui précède, ces composés sont susceptibles d'être utilisés comme médicaments, en particulier dans le traitement et / ou la prévention des maladies ou désordres suivants :
- l'inhibition de la prolifération tumorale seule ou en combinaison avec d'autres traitements ;
- l'inhibition de la prolifération des cellules normales seule ou en combinaison avec
- d'autres traitements ;
- les maladies neurodégénératives comme la maladie d'Alzheimer ;
- la prévention de l'alopécie spontanée ;
- la prévention de l'alopécie induite par des produits exogènes ;
- la prévention de l'alopécie radio-induite ;
- la prévention de l'apoptose spontanée ou induite des cellules normales ;
- la prévention de la méiose et / ou la fécondation ;
- la prévention de la maturation des oocytes ;
- toutes les maladies / tous les désordres correspondant à des utilisations rapportées pour les inhibiteurs de CDKs, et notamment les maladies prolifératives non tumorales (par exemple : angiogénèse, psoriasis ou resténose), maladies prolifératives tumorales, parasitologie (prolifération de protozoaires), infections virales, maladies neurodégénératives, myopathies ; et / ou
- toutes les maladies / tous les désordres correspondant à des applications cliniques de la vitamine K et de ses dérivés.

Par ailleurs, les composés de la présente invention sont également, du fait de leurs propriétés d'inhibition des phosphatases cdc25, susceptibles d'être utilisés pour inhiber ou prévenir la prolifération des microorganismes, notamment des levures. L'un des avantages de ces composés consiste en leur faible toxicité sur les cellules saines.

L'invention a tout d'abord pour objet les composés répondant à la formule générale **(I)** 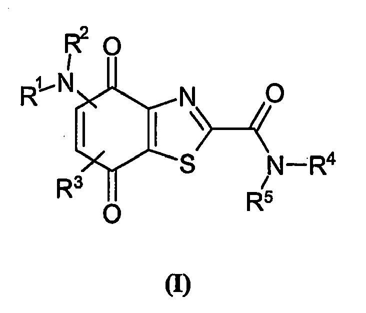 sous forme racémique, d'énantiomère ou toute combinaison de ces formes, dans laquelle :
R¹ représente un atome d'hydrogène ou un radical alkyle, alkoxyalkyle, alkylthioalkyle, cycloalkyle, -(CH₂)-X-Y, -(CH₂)-Z-NR⁶R⁷ ou un radical -CHR⁸R⁹,
X représentant une liaison ou un radical alkylène linéaire ou ramifié comptant de 1 à 5 atomes de carbone,
Y représentant un système cyclique carboné saturé comptant de 1 à 3 cycles condensés choisis indépendamment parmi des cycles de 3 à 7 chaînons, ou bien Y représentant un hétérocycle saturé comptant de 1 à 2 hétéroatomes choisis indépendamment parmi O, N et S et attaché au radical X par un chaînon N ou CH, ledit hétérocycle saturé comptant par ailleurs de 2 à 6 chaînons supplémentaires choisis indépendamment parmi -CHR¹⁰-, -CO-, -NR¹¹-, -O- et -S-, R¹⁰ représentant un atome d'hydrogène ou un radical alkyle et R¹¹ représentant un atome d'hydrogène ou un radical alkyle ou aralkyle, ou encore Y représentant un radical aryle carbocyclique ou hétérocyclique éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR¹² et un radical NR¹³R¹⁴, R¹² représentant un atome d'hydrogène ou un radical alkyle ou phényle, et R¹³ et R¹⁴ représentant indépendamment des radicaux alkyle,
Z représentant une liaison ou un radical alkylène linéaire ou ramifié comptant de 1 à 5 atomes de carbone,
R⁶ et R⁷ étant choisis indépendamment parmi un atome d'hydrogène, un radical alkyle, aralkyle ou -(CH₂)ₙ-OH dans lequel n représente un entier de 1 à 6,
ou R⁶ représentant un radical alkoxycarbonyle, haloalkoxycarbonyle ou aralkoxycarbonyle et R⁷ représentant un atome d'hydrogène ou un radical méthyle,
ou encore R⁶ et R⁷ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR¹⁵R¹⁶-, -O-, -S- et -NR¹⁷-, R¹⁵ et R¹⁶ représentant indépendamment à chaque fois qu'ils interviennent un atome d'hydrogène ou un radical alkyle, et R¹⁷ représentant un atome d'hydrogène ou un radical alkyle ou aralkyle, ou encore R¹⁷ représentant un radical phényle éventuellement substitué de 1 à 3 fois par des substituants choisis indépendamment parmi un atome halogène et un radical alkyle ou alkoxy,
R⁸ et R⁹ formant ensemble avec l'atome de carbone qui les porte un radical indanyle ou tétralinyle, ou encore R⁸ et R⁹ formant ensemble avec l'atome de carbone qui les porte un hétérocycle saturé comptant de 5 à 7 chaînons et de 1 à 2 hétéroatomes choisis parmi O, N et S, les atomes d'azote dudit hétérocycle étant éventuellement substitués par des radicaux choisis parmi les radicaux alkyle et le radical benzyle ;
R² représentant un atome d'hydrogène ou un radical alkyle ou aralkyle ;
ou encore R¹ et R² formant ensemble avec l'atome d'azote un hétérocycle de 4 à 8 chaînons comportant de 1 à 2 hétéroatomes, les chaînons nécessaires pour compléter l'hétérocycle étant choisis indépendamment parmi les radicaux -CR¹⁸R¹⁹-, -O-, -S- et -NR²⁰-, R¹⁸ et R¹⁹ représentant indépendamment à chaque fois qu'ils interviennent un atome d'hydrogène ou un radical alkyle, et R²⁰ représentant un atome d'hydrogène ou un radical alkyle ou aralkyle ;
R³ représente un atome d'hydrogène ou un atome halogène ;
R⁴ représente un radical alkyle, un radical haloalkyle, un radical cycloalkyle, un radical cycloalkylalkyle, un radical alkoxyalkyle, l'un des radicaux aryle carbocyclique ou hétérocyclique ou aralkyle carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂,
ou encore R⁴ représente l'un des radicaux -(CH₂)ₘ-[O-(CH₂)ₚ]_{q}-O-Alk, -(CH₂)ᵣ-[O-(CH₂)ₛ]ₜ-NR²¹R²² ou -(CH₂)ᵥ-A dans lesquels m, p et s sont chacun indépendamment un entier de 2 à 4, q est un entier de 1 à 4, t est un entier de 0 à 4, r est un entier de 2 à 12 (et de préférence un entier de 2 à 8 et notamment un entier de 2 à 6) et v est un entier de 1 à 12 (et de préférence un entier de 1 à 8 et notamment un entier de 1 à 6), Alk est un radical alkyle, R²¹ est un atome d'hydrogène ou un radical alkyle, alkoxycarbonyle ou aralkoxycarbonyle, R²² est un atome d'hydrogène ou un radical alkyle et A est un hétérocycle saturé comptant de 1 à 2 hétéroatomes choisis indépendamment parmi O, N et S et attaché au groupe -(CH₂)ᵥ- par un chaînon N ou CH, ledit hétérocycle saturé comptant par ailleurs de 2 à 6 chaînons supplémentaires choisis indépendamment parmi -CHR²³-, -CO-, -NR²⁴-, -O- et -S-, R²³ représentant un atome d'hydrogène ou un radical alkyle et R²⁴ représentant un atome d'hydrogène, un radical alkyle ou un groupement alkoxycarbonyle ou aralkoxycarbonyle,
ou encore R⁴ représente un radical de formule 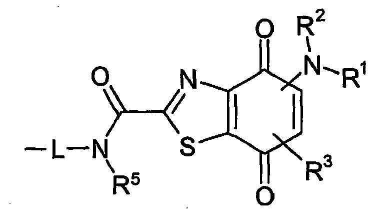 dans laquelle R¹, R², R³ et R⁵ sont identiques aux radicaux R¹, R², R³ mentionnés précédemment et R⁵ mentionnés ci-après, et L est choisi parmi les radicaux -(CH₂)_{g}-[O-(CH₂)_{w}]ₓ-[O-(CH₂)_{y}]_{z}- et -(CH₂)ₐ-Ω-(CH₂)_{b}- dans lesquels g, w et y sont des entiers de 2 à 4 (et de préférence 2 à 3), x est un entier de 1 à 3 et z est 0 ou 1, a et b sont indépendamment des entiers de 2 à 6 (et de préférence 2 à 4) et Ω est choisi parmi le groupe constitué par -O-, -S-, -NR²⁵-, -CO-, -CO-NR²⁶-, -CR²⁷R²⁸-, un radical cycloalkylène comptant de 3 à 7 atomes de carbone et enfin un radical aryle carbocyclique, R²⁵ représentant un radical alkyle, R²⁶ représentant un atome d'hydrogène ou un radical méthyle, R²⁷ et R²⁸ étant chacun choisis indépendamment parmi un atome d'hydrogène et un groupe méthyle ;
ou bien encore R⁴ représente un radical de formule 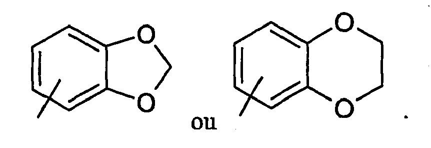
R⁵ représente un atome d'hydrogène ou un radical alkyle ou aralkyle, R⁵ pouvant également représenter un radical identique à R⁴ lorsque R⁴ représente un radical alkyle, haloalkyle, alkoxyalkyle ou aralkyle carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂;
ou encore R⁴ et R⁵ forment ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CR²⁹R³⁰-, -O-, -S- et -NR³¹-, R²⁹ et R³⁰ représentant un atome d'hydrogène ou un radical alkyle ou aralkyle et R³¹ représentant -COR³² ou -SO₂R³³,
R³² représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore R³² représentant un radical aryle hétérocyclique ou un hétérocycle saturé comptant de 5 à 7 chaînons et de 1 à 2 hétéroatomes choisis indépendamment parmi O, N et S (et notamment l'un des radicaux pipéridino, pipérazino, morpholino, thiomorpholino ou 2-tétrahydrofuryle),
R³³ représentant un atome d'hydrogène ou un radical alkyle,
ou enfin R⁴ et R⁵ forment ensemble avec l'atome d'azote qui les porte un radical aryle hétérocyclique choisi parmi les radicaux 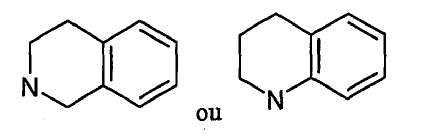 dont le noyau aromatique peut être substitué de 1 à 3 fois par des substituants choisis indépendamment parmi le groupe constitué par un radical alkyle et un radical alkoxy ;
et leurs sels.

La Demanderesse a découvert de façon surprenante que les composés ci-dessus répondant à la formule générale (I) sont de puissants inhibiteurs des phosphatases Cdc25 (et notamment de la phosphatase Cdc25C), ce qui les rend aptes à une utilisation en tant qu'agents anti-cancéreux.

L'invention concerne donc en premier lieu les composés de formule générale (I) définie précédemment et les sels de tels composés.

Par alkyle, lorsqu'il n'est pas donné plus de précisions, on entend un radical alkyle linéaire ou ramifié comptant de 1 à 12 atomes de carbone, de préférence de 1 à 10 atomes de carbone et plus préférentiellement 1 à 8 atomes de carbone (et notamment de 1 à 6 atomes de carbone).

Par alkylène, lorsqu'il n'est pas donné plus de précisions, on entend un radical alkyl saturé, linéaire ou ramifié, comptant de 1 à 5 atomes de carbone, par exemple un radical -CH₂- ou -CH₂-CH₂-.

Par alkoxy, lorsqu'il n'est pas donné plus de précisions, on entend un radical alkoxy linéaire ou ramifié comptant de 1 à 6 atomes de carbone (et notamment de 1 à 4 atomes de carbone).

Par alkoxycarbonyle, lorsqu'il n'est pas donné plus de précisions, on entend un radical de type -CO-O-alkyl.

Par haloalkoxycarbonyle lorsqu'il n'est pas donné plus de précisions, on entend un radical de type -CO-O-haloalkyl.

Par haloalkyle, on entend un radical alkyle dont au moins l'un des atomes d'hydrogène (et éventuellement tous) est remplacé par un atome halogène.

Par aralkoxycarbonyle lorsqu'il n'est pas donné plus de précisions, on entend un radical de type -CO-O-alkyl-aryl.

Par cycloalkyle, lorsqu'il n'est pas donné plus de précisions, on entend un radical cycloalkyle comptant de 3 à 7 atomes de carbone.

Par cycloalkylène, lorsqu'il n'est pas donné plus de précisions, on entend un radical cycloalkyle comptant de 3 à 7 atomes de carbone, par exemple un radical

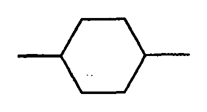

Par alkylthioalkyle, lorsqu'il n'est pas donné plus de précisions, on entend un radical de type -alkyl-S-alkyle.

Par aryle carbocyclique ou hétérocyclique, on entend un système carbocyclique ou hétérocyclique de 1 à 3 cycles condensés comprenant au moins un cycle aromatique, un système étant dit hétérocyclique lorsque l'un au moins des cycles qui le composent comporte au moins un hétéroatome (O, N ou S); lorsqu'un radical aryle carbocyclique ou hétérocyclique est dit substitué sans qu'il soit donné plus de précisions, on entend que ledit radical aryle carbocyclique ou hétérocyclique est substitué de 1 à 3 fois, et de préférence de 1 à 2 fois par des radicaux différents d'un atome d'hydrogène qui, s'ils ne sont pas précisés, sont choisis parmi un atome halogène et les radicaux alkyle ou alkoxy ; par ailleurs, lorsqu'il n'est pas donné plus de précisions, on entend par aryle un aryle carbocyclique exclusivement.

Par halogène ou atome halogène on entend un atome de chlore, de brome, de fluor ou d'iode.

Par radicaux cycloalkylalkyle, alkoxyalkyle, haloalkyle, haloalkoxy et aralkyle, on entend respectivement les radicaux cycloalkylalkyle, alkoxyalkyle, haloalkyle, haloalkoxy et aralkyle dont les radicaux alkyle, cycloalkyle et aryle ont les significations indiquées précédemment.

Lorsqu'il est indiqué qu'un radical est éventuellement substitué de 1 à 3 fois, il est de préférence éventuellement substitué de 1 à 2 fois et plus préférentiellement éventuellement substitué une fois.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par haloalkyle, on entend notamment le radical trifluorométhyle. Par haloalkoxy, on entend notamment le radical trifluorométhoxy. Par aryle carbocyclique, on entend en particulier les radicaux phényle et naphtyle. Par aralkyle, on entend en particulier les radicaux phénylalkyle, et notamment le radical benzyle. Par système cyclique carboné saturé comptant de 1 à 3 cycles condensés choisis indépendamment parmi des cycles de 3 à 7 chaînons, on entend en particulier les radicaux cyclopropyle, cyclobutyle, cyclohexyle et adamantyle. Par aryle hétérocyclique ou hétéroaryle, on entend en particulier les radicaux thiényle, furannyle, pyrrolyle, imidazolyle, thiazolyle, oxazolyle et pyridyle. Enfin, par halogène, on entend les atomes de fluor, de chlore, de brome ou d'iode.

Par sel d'un composé, on entend les sels d'addition dudit composé avec un acide organique ou inorganique ou, le cas échéant, avec une base, et notamment les sels pharmaceutiquement acceptables dudit composé.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

D'une façon générale, on préfère que l'hétérocycle saturé A soit tel qu'il comporte au maximum 2 chaînons -CO-, au maximum 2 chaînons choisis indépendamment parmi -NR²⁴-, -O- et -S- et au maximum un chaînon -CHR²³- dans lequel R²³ n'est pas un atome d'hydrogène. De même, on préfère, dans le cas où R⁴ et R⁵ forment ensemble avec l'atome d'azote qui les porte un hétérocycle saturé de 4 à 7 chaînons comportant de 1 à 2 hétéroatomes, que ledit hétérocycle saturé soit tel qu'il comporte au maximum deux chaînons -CR²⁹R³⁰ - dans lequel au moins l'un de R²⁹ et R³⁰ n'est pas un atome d'hydrogène.

Selon une variante de l'invention, les composés de formule générale **(I)** ou leurs sels seront tels que R⁴ ne représente pas un radical de formule 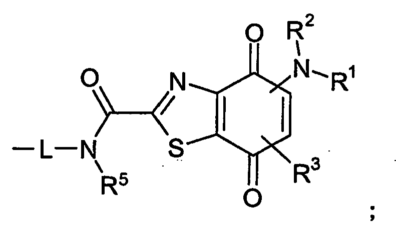 pour la suite de cet exposé, de tels composés seront appelés les "composés de sous-formule générale **(I**)**_{M}**".

Parmi les composés de sous-formule générale **(I)_{M}**, certains répondront à la sous-formule générale **(I)_{M5}** 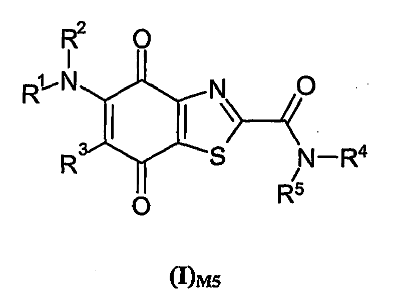 dans laquelle R¹, R², R³, R⁴ et R⁵ ont la même signification que dans la sous-formule générale **(I)_{M}**;
tandis que les autres répondront à la sous-formule générale **(I)_{M6}** 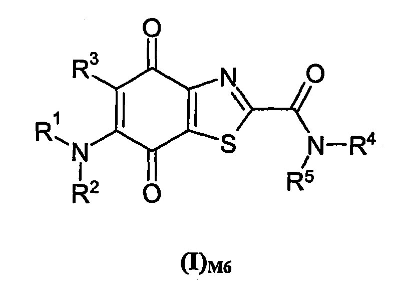 dans laquelle R¹, R², R³, R⁴ et R⁵ ont la même signification que dans la sous-formule générale **(I)_{M}**.

Selon une autre variante de l'invention, les composés de formule générale **(I)** ou leurs sels seront tels que R⁴ représente un radical de formule 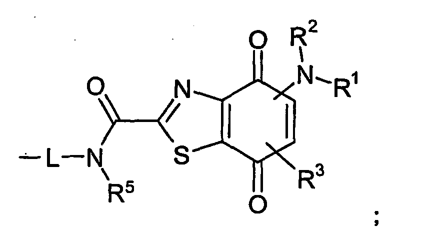 pour la suite de cet exposé, de tels composés seront appelés les "composés de sous-formule générale **(I)_{D}**".

Parmi les composés de sous-formule générale **(I)_{D}**, certains répondront à la sous-formule générale **(I)_{D5}** 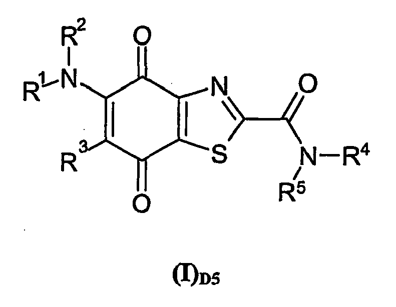 dans laquelle R¹, R², R³ et R⁵ ont la même signification que dans la sous-formule générale **(I)_{D}** et R⁴ représente un radical de formule 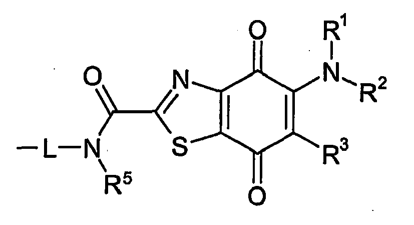 dans lequel L a la même signification que dans la sous-formule générale **(I)_{D}**;
tandis que les autres répondront à la sous-formule générale **(I)_{D6}** 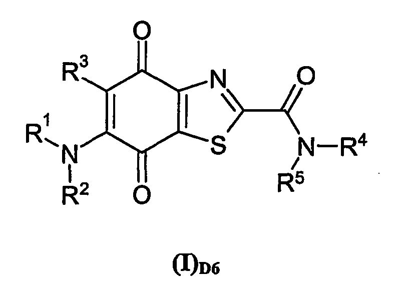 dans laquelle R¹, R², R³ et R⁵ ont la même signification que dans la sous-formule générale **(I)_{D}** et R⁴ représente un radical de formule 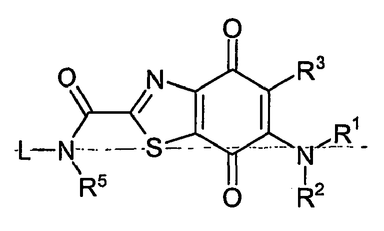 dans lequel L a la même signification que dans la sous-formule générale **(I)_{D}**.

Lorsque les composés de sous-formule générale **(I)_{D}** ou leurs sels seront tels que L représente -(CH₂)ₐ-Ω-(CH₂)_{b}-, Ω sera de préférence -O-, -CO-, -CR²⁷R²⁸- et plus préférentiellement -O-.

De préférence, les composés de formule générale **(I)** ou leurs sels seront tels qu'ils possèdent au moins indépendamment l'une des caractéristiques suivantes :
❖ R¹ représente un radical alkyle, cycloalkyle, alkoxyalkyle, -(CH₂)-X-Y, -(CH₂)-Z-NR⁶R⁷ ou-CHR⁸R⁹ ; ou
❖ R² représente un atome d'hydrogène ou le radical méthyle, éthyle ou benzyle ; ou
❖ R³ représente un atome d'hydrogène ; ou
❖ R⁴ représente un radical alkyle, un radical cycloalkyle, un radical cycloalkylalkyle, un radical alkoxyalkyle, l'un des radicaux aryle carbocyclique ou hétérocyclique ou aralkyle carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂,
   ou encore R⁴ représente l'un des radicaux -(CH₂)ₘ-[O-(CH₂)ₚ]_{q}-O-Alk, -(CH₂)ᵣ-[O-(CH₂)ₛ]ₜ-NR²¹R²² ou -(CH₂)ᵥ-A dans lesquels m, p et s sont chacun indépendamment 2 ou 3, q est un entier de 1 à 3, t est un entier de 0 à 3, r est un entier de 2 à 6 et v est un entier de 1 à 6, Alk est un radical alkyle, R²¹ est un atome d'hydrogène ou un radical alkoxycarbonyle, R²² est un atome d'hydrogène ou un radical méthyle et A est un hétérocycle saturé comptant de 1 à 2 hétéroatomes choisis indépendamment parmi O, N et S et attaché au groupe -(CH₂)ᵥ- par un chaînon N ou CH, ledit hétérocycle saturé comptant par ailleurs de 2 à 6 chaînons supplémentaires choisis indépendamment parmi -CHR²³-, -CO-, -NR²⁴-, -O- et -S-, R²³ représentant un atome d'hydrogène ou un radical méthyle et R²⁴ représentant un atome d'hydrogène, un radical alkyle ou un groupement alkoxycarbonyle ;
   ou bien encore R⁴ représente un radical de formule 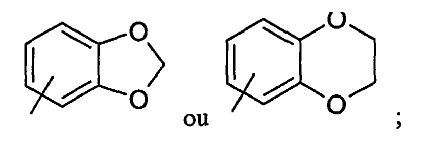 ou
❖ R⁵ représente un atome d'hydrogène ou un radical alkyle ou aralkyle, R⁵ pouvant également représenter un radical identique à R⁴ lorsque R⁴ représente un radical alkyle, alkoxyakyle ou aralkyle carbocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂ ;
❖ ou encore R⁴ et R⁵ forment ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CH₂-, -O-, -S- et -NR³¹-, R³¹ représentant -COR³²- ou -SO₂R³³-,
   R³² représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore R³² représentant un radical aryle hétérocyclique ou bien un hétérocycle saturé choisi parmi les radicaux pipéridino, pipérazino, morpholino, thiomorpholino et 2-tétrahydrofuryle,
   R³³ représentant un atome d'hydrogène ou un radical alkyle,
   ou encore R⁴ et R⁵ forment ensemble avec l'atome d'azote qui les porte un radical aryle hétérocyclique de formule 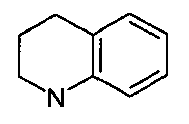 dont le noyau aromatique peut être substitué de 1 à 3 fois par un radical alkyle ou alkoxy.

Plus préférentiellement, les composés de formule générale **(I)** ou leurs sels seront tels qu'ils possèdent au moins indépendamment l'une des caractéristiques suivantes :
❖ R¹ représente un radical -(CH₂)-Z-NR⁶R⁷,
   Z représentant une liaison ou un radical alkylène linéaire ou ramifié comptant de 1 à 5 atomes de carbone,
   R⁶ et R⁷ étant choisis indépendamment parmi un atome d'hydrogène et un radical alkyle, ou encore R⁶ et R⁷ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CH₂-, -O-, -S- et -NR¹⁷-, R¹⁷ représentant un atome d'hydrogène ou un radical alkyle ; ou
❖ R² représente un atome d'hydrogène ou le radical méthyle ; ou
❖ R⁴ représente un radical alkyle, un radical cycloalkyle, un radical cycloalkylalkyle, un radical alkoxyalkyle ou un radical aryle carbocyclique ou hétérocyclique ou aralkyle carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂ (et de préférence choisis indépendamment parmi le groupe constitué par un atome halogène, un radical alkyle et un radical alkoxy),
   ou encore R⁴ représente l'un des radicaux -(CH₂)ₘ-[O-(CH₂)ₚ]_{q}-O-Alk, -(CH₂)ᵣ-[O-(CH₂)ₛ]ₜ-NR²¹R²² ou -(CH₂)ᵥ-A dans lesquels m, p et s sont chacun indépendamment 2 ou 3, q est un entier de 1 à 3, t est un entier de 0 à 3, r est un entier de 2 à 6 et v est un entier de 1 à 6, Alk est un radical alkyle, R²¹ est un atome d'hydrogène ou un radical alkoxycarbonyle, R²² est un atome d'hydrogène ou un radical méthyle et A est un hétérocycle saturé comptant de 1 à 2 hétéroatomes choisis indépendamment parmi O, N et S et attaché au groupe -(CH₂)ᵥ- par un chaînon N ou CH, ledit hétérocycle saturé comptant par ailleurs de 2 à 6 chaînons supplémentaires choisis indépendamment parmi -CHR²³-, -CO-, -NR²⁴-, -O- et -S-, R²³ représentant un atome d'hydrogène ou un radical méthyle et R²⁴ représentant un atome d'hydrogène, un radical alkyle ou un groupement alkoxycarbonyle ;
   ou bien encore R⁴ représente un radical de formule 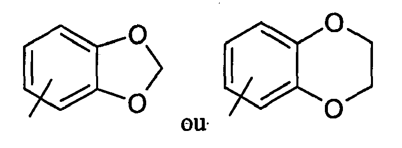
❖ R⁵ représente un atome d'hydrogène, R⁵ pouvant également représenter un radical identique à R⁴ lorsque R⁴ représente un radical alkyle, alkoxyakyle ou aralkyle carbocyclique dont le noyau aryle est éventuellement substitué de 1 à 3 fois par des substituants choisis indépendamment parmi le groupe constitué par un atome halogène, un radical alkyl, un radical alkoxy, un radical haloalkyle et un radical -SO₂-NH₂ ; ou
❖ ou encore R⁴ et R⁵ forment ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CH₂-, -O-, -S- et -NR³¹-, R³¹ représentant -COR³²- ou -SO₂R³³-,
   R³² représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore R³² représentant un radical aryle hétérocyclique choisi parmi les radicaux 2-furyle, 2-pyrrolyle et 2-thiényle, ou bien un hétérocycle saturé choisi parmi les radicaux pipéridino, pipérazino, morpholino, thiomorpholino et 2-tétrahydrofuryle,
   R³³ représentant un atome d'hydrogène ou un radical méthyle,
   ou encore R⁴ et R⁵ forment ensemble avec l'atome d'azote qui les porte un radical aryle hétérocyclique de formule 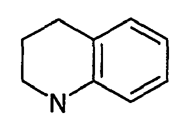 dont le noyau aromatique peut être substitué de 1 à 3 fois par un radical alkyle ou alkoxy (et de préférence par un radical alkoxy).

Encore plus préférentiellement, les composés de formule générale **(I)** ou leurs sels seront tels qu'ils possèdent au moins indépendamment l'une des caractéristiques suivantes :
❖ R¹ représentant un radical -(CH₂)-Z-NR⁶R⁷,
   Z représentant une liaison ou un radical alkylène linéaire ou ramifié comptant de 1 à. 3 atomes de carbone (et en particulier 1 atome de carbone),
   R⁶ et R⁷ étant choisis indépendamment parmi un atome d'hydrogène et un radical alkyle (et en particulier R⁶ et R⁷ représentant chacun un radical méthyle), ou encore R⁶ et R⁷ formant ensemble avec l'atome d'azote un cycle pyrrolidinyle ou pipéridinyle (et en particulier pyrrolidinyle) ;
❖ R² représentant un atome d'hydrogène ;
❖ R⁴ représentant un radical alkyle, un radical cycloalkyl, un radical cycloalkylalkyle, un radical alkoxyalkyle ou un radical aryle carbocyclique ou hétérocyclique ou aralkyle carbocyclique ou hétérocyclique dont le noyau aryle est éventuellement substitué de 1 à 2 fois par des substituants choisis indépendamment parmi le groupe constitué par un atome halogène, un radical alkyle ou un radical alkoxy,
   ou encore R⁴ représentant l'un des radicaux -(CH₂)ₘ-[O-(CH₂)ₚ]_{q}-O-Alk, -(CH₂)ᵣ-[O-(CH₂)ₛ]ₜ-NR²¹R²² ou -(CH₂)ᵥ-A dans lesquels m, p et s sont chacun indépendamment 2 ou 3, q est un entier de 1 à 3, t est un entier de 0 à 3, r est un entier de 2 à 6 et v est un entier de 1 à 6, Alk est un radical alkyle comptant de 1 à 3 atomes de carbone, R²¹ est un atome d'hydrogène ou un radical alkoxycarbonyle (et notamment *tert*-butoxycarbonyle), R²² est un atome d'hydrogène et A est un hétérocycle saturé comptant de 1 à 2 hétéroatomes choisis indépendamment parmi O, N et S et attaché au groupe -(CH₂)ᵥ- par un chaînon N ou CH, ledit hétérocycle saturé comptant par ailleurs de 2 à 6 chaînons supplémentaires choisis indépendamment parmi -CH₂-, -CO-, -NR²⁴-, -O- et -S-, R²³ représentant un atome d'hydrogène ou un radical méthyle et R²⁴ représentant un atome d'hydrogène, un radical alkyle ou un groupement alkoxycarbonyle ;
   ou bien encore R⁴ représente un radical de formule 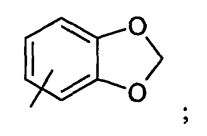
❖ R⁵ représentant un atome d'hydrogène ;
❖ ou encore R⁴ et R⁵ formant ensemble avec l'atome d'azote qui les porte un hétérocycle saturé de 5 à 7 chaînons comportant de 1 à 2 hétéroatomes en tout, les chaînons nécessaires pour compléter l'hétérocycle étant choisis indépendamment parmi les radicaux -CH₂-, -O-, -S- et -NR³¹-, R³¹ représentant -COR³²- ou -SO₂R³³-,
   R³² représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore R³² représentant un radical aryle hétérocyclique choisi parmi les radicaux 2-furyle, 2-pyrrolyle et 2-thiényle, ou bien un hétérocycle saturé choisi parmi les radicaux pipéridino, pipérazino, morpholino, thiomorpholino et 2-tétrahydrofuryle,
   R³³ représentant un radical alkyle ;
   ou encore R⁴ et R⁵ forment ensemble avec l'atome d'azote qui les porte un radical aryle hétérocyclique de formule 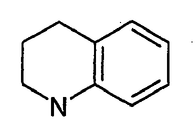 dont le noyau aromatique peut être substitué de 1 à 3 fois (et de préférence 1 fois) par un radical alkoxy (et de préférence par un radical méthoxy).

Plus particulièrement les composés de formule générale **(I)** ou leurs sels seront tels qu'ils possèdent au moins indépendamment l'une des caractéristiques suivantes :
❖ R¹ représente au moins un radical choisi parmi les radicaux suivants : -(CH₂)₂-N(CH₃)₂, ou -(CH₂)₂-O-CH₃, ou un radical 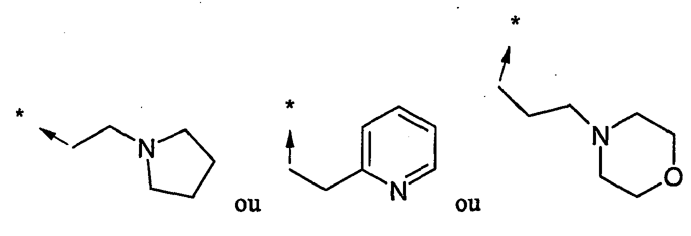 ou 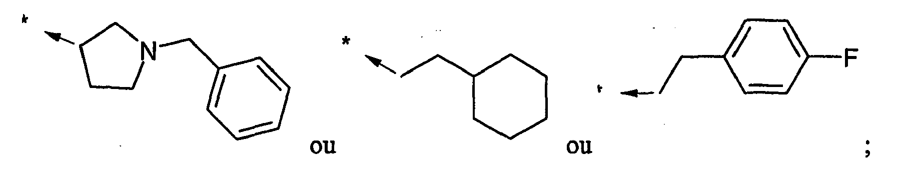
❖ Ou bien R¹R²N- représente un radical 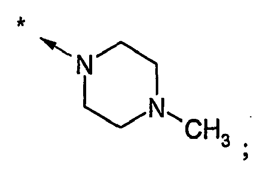
❖ Ou bien R² représente un atome d'hydrogène ou un radical méthyle ;
❖ Ou bien R³ représente un atome d'hydrogène ou de brome ;
❖ Ou bien R⁴ représente au moins un radical choisi parmi les radicaux suivants : 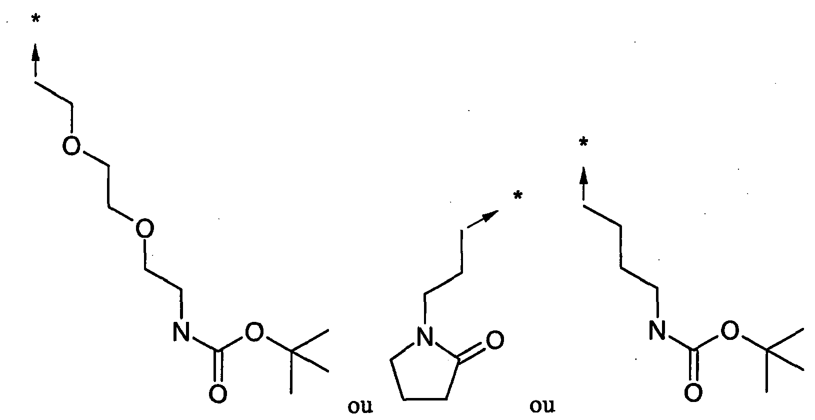 ou 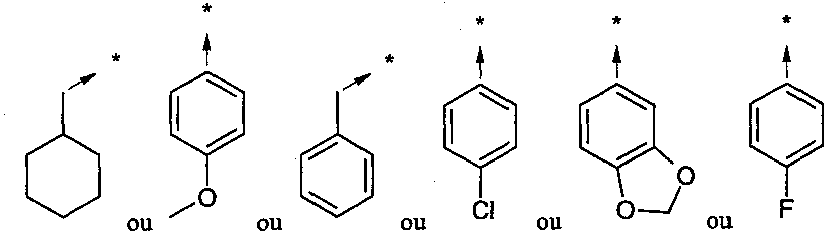 ou 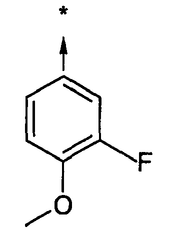 ou un radical éthyl, isobutyl, butyl ou -(CH₂)₂-O-CH₃,
   ou encore R⁴ représente un radical de formule 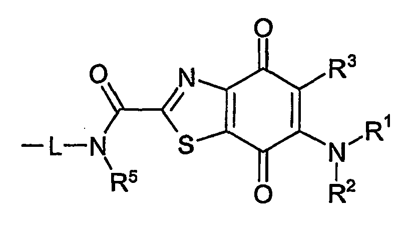 dans lequel L représente un radical choisi indépendamment parmi -(CH₂)₂-O-(CH₂)₂-O-(CH₂)₂- ou -(CH₂)₂-O-(CH₂)₂-, R⁵, R³ et R² représentent H et R1 représente -(CH₂)₂-N(CH₃)₂;
❖ Ou bien R⁵ représente un atome d'hydrogène ou un radical -(CH₂)₂-O-CH₃ ou un radical 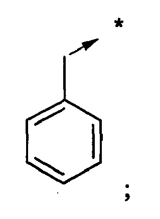
❖ ou encore R⁴ et R⁵ formant ensemble avec l'atome d'azote qui les porte un hétérocycle choisi parmi les hétérocycles suivants : 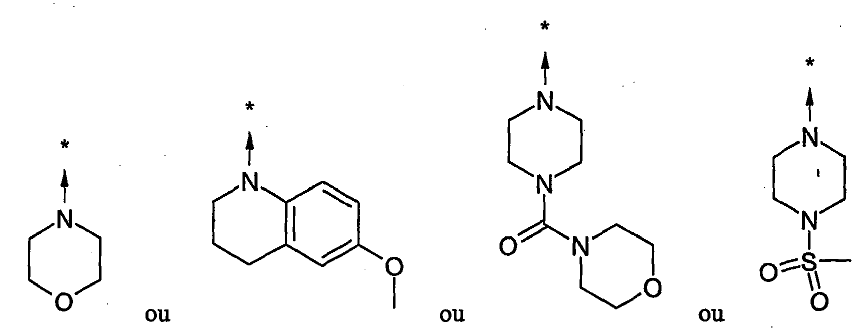 ou 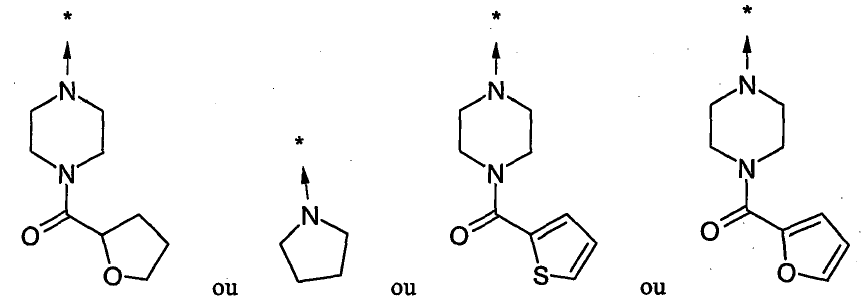 ou 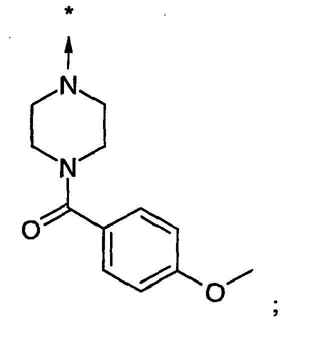 étant entendu → * que signifie le point d'attachement à la formule générale (I).

Parmi les composés de formule générale **(I)**, on préférera en particulier les composés suivants décrits dans les exemples :
- 5-{[2-(diméthylamino)éthyl]amino}-2-(morpholin-4-ylcarbonyl)-1,3-benzothiazole-4,7-dione ;
- {2-[2-(2-{[(5-{[2-(diméthylamino)éthyl]amino}-4,7-dioxo-4,7-dihydro-1,3-benzothiazol-2-yl)carbonyl]amino}éthoxy)éthoxy]éthyl}carbamate de *tert*-butyle ;
- *N,N*-dibenzyl-5-{[2-(diméthylamino)éthyl]amino}-4,7-dioxo-4,7-dihydro-1,3-benzothiazole-2-carboxamide ;
- 5-{[2-(diméthylamino)éthyl]amino}-*N,N-*bis(2-méthoxyéthyl)-4,7-dioxo-4,7-dihydro-1,3-benzothiazole-2-carboxamide ;
- 5-{[2-(diméthylamino)éthyl]amino}-4,7-dioxo-*N*-[3-(2-oxopyrrolidin-1-yl)propyl]-4,7-dihydro-1,3-benzothiazole-2-carboxamide ;
- 4,7-dioxo-*N*-[3-(2-oxopyrrolidin-1-yl)propyl]-5-[(2-pyrrolidin-1-yléthyl)amino]-4,7-dihydro-1,3-benzothiazole-2-carboxamide ;
- 5-{[2-(diméthylamino)éthyl]amino}-*N*-isobutyl-4,7-dioxo-4,7-dihydro-1,3-benzothiazole-2-carboxamide ;
- (4-{[(5-{[2-(diméthylamino)éthyl]amino}-4,7-dioxo-4,7-dihydro-1,3-benzothiazol-2-yl)carbonyl]amino}butyl)carbamate de *tert*-butyle ;
- 5-{[2-(diméthylamino)éthyl]amino}-2-{[4-(2-furoyl)pipérazin-1-yl]carbonyl}-1,3-benzothiazole-4,7-dione ;
- 5-{[2-(diméthylamino)éthyl]amino}-*N*-(2-méthoxyéthyl)-4,7-dioxo-4,7-dihydro-1,3-benzothiazole-2-carboxamide ;
- *N*-butyl-5-{[2-(diméthylamino)éthyl]amino}-4,7-dioxo-4,7-dihydro-1,3-benzothiazole-2-carboxamide ;
- *N*-benzyl-5-{[2-(diméthylamino)éthyl]amino}-4,7-dioxo-4,7-dihydro-1,3-benzothiazole-2-carboxamide ;
- *N*-(cyclohexylméthyl)-5-{[2-(diméthylamino)éthyl]amino}-4,7-dioxo-4,7-dihydro-1,3-benzothiazole-2-carboxamide ;
- *N,N'*-(oxydiéthane-2,1-diyl)bis(5-{[2-(diméthylamino)éthyl]amino}-4,7-dioxo-4,7-dihydro-1,3-benzothiazole-2-carboxamide) ;
- *N,N*-[éthane-1,2-diylbis(oxyéthane-2,1-diyl)]bis(5-{[2-(diméthylamino)éthyl]amino}-4,7-dioxo-4,7-dihydro-1,3-benzothiazole-2-carboxamide);
- 5-{[2-(diméthylamino)éthyl]amino}-2-{[4-(morpholin-4-ylcarbonyl)pipérazin-1-yl]carbonyl}-1,3-benzothiazole-4,7-dione ;
- 2-{[4-(morpholin-4-ylcarbonyl)pipérazin-1-yl]carbonyl}-5-[(2-pyrrolidin-1-yléthyl)amino]-1,3-benzothiazole-4,7-dione ;
- 5-{[2-(diméthylamino)éthyl]amino}-*N*-(4-méthoxyphényl)-4,7-dioxo-4,7-dihydro-1,3-benzothiazole-2-carboxamide ;
- 5-{[2-(diméthylamino)éthyl]amino}-2-{[4-(méthylsulfonyl)pipérazin-1-yl]carbonyl}-1,3-benzothiazole-4,7-dione ;
- 5-{[2-(diméthylamino)éthyl]amino}-2-{[4-(tétrahydrofuran-2-ylcarbonyl)pipérazin-1-yl]carbonyl}-1,3-benzothiazole-4,7-dione ;
- 6-bromo-5-{[2-(diméthylamino)éthyl]amino}-2-{[4-(tétrahydrofuran-2-ylcarbonyl)pipérazin-1-yl]carbonyl}-1,3-benzothiazole-4,7-dione ;
- 5-{[2-(diméthylamino)éthyl]amino}-2-(pyrrolidin-1-ylcarbonyl)-1,3-benzothiazole-4,7-dione ;
- 5-{[2-(diméthylamino)éthyl]amino}-2-{[4-(2-thiénylcarbonyl)pipérazin-1-yl]carbonyl}-1,3-benzothiazole-4,7-dione ;
- 5-{[2-(diméthylamino)éthyl]amino}-*N*-éthyl-4,7-dioxo-4,7-dihydro-1,3-benzothiazole-2-carboxamide ;
- *N*-(4-chlorophényl)-5-{[2-(diméthylamino)éthyl]amino}-4,7-dioxo-4,7-dihydro-1,3-benzothiazole-2-carboxamide ;
- *N*-1,3-benzodioxol-5-yl-5-{[2-(diméthylamino)éthyl]amino}-4,7-dioxo-4,7-dihydro-1,3-benzothiazole-2-carboxamide ;
- 5-[(2-méthoxyéthyl)amino]-2-{[4-(tétrahydrofuran-2-ylcarbonyl)pipérazin-1-yl]carbonyl}-1,3-benzothiazole-4,7-dione ;
- 5-(4-méthylpipérazin-1-yl)-2-{[4-(tétrahydrofuran-2-ylcarbonyl)pipérazin-1-yl]carbonyl}-1,3-benzothiazole-4,7-dione ;
- 5-[(2-pyridin-2-yléthyl)amino]-2-{[4-(tétrahydrofuran-2-ylcarbonyl)pipérazin-1-yl]carbonyl}-1,3-benzothiazole-4,7-dione ;
- 5-[(3-morpholin-4-ylpropyl)amino]-2-{[4-(tétrahydrofuran-2-ylcarbonyl)pipérazin-1-yl]carbonyl}-1,3-benzothiazole-4,7-dione;
- 5-[(1-benzylpyrrolidin-3-yl)amino]-2-{[4-(tétrahydrofuran-2-ylcarbonyl)pipérazin-1-yl]carbonyl}-1,3-benzothiazole-4,7-dione;
- 5-[(2-cyclohexyléthyl)amino]-2-{[4-(tétrahydrofuran-2-ylcarbonyl)pipérazin-1-yl]carbonyl}-1,3-benzothiazole-4,7-dione;
- 5-{[2-(diméthylamino)éthyl]amino}-*N*-(4-fluorophényl)-4,7-dioxo-4,7-dihydro-1,3-benzothiazole-2-carboxamide ;
- 6-{[2-(diméthylamino)éthyl]amino}-2-(pyrrolidin-1-ylcarbonyl)-1,3-benzothiazole-4,7-dione ;
- 5-{[2-(diméthylamino)éthyl]amino}-*N*-(3-fluoro-4-méthoxyphényl)-4,7-dioxo-4,7-dihydro-1,3-benzothiazole-2-carboxamide ;
- 5-{[2-(diméthylamino)éthyl]amino}-2-[(6-méthoxy-3,4-dihydroquinolin-1(2*H*)-yl)carbonyl]-1,3-benzothiazole-4,7-dione;
- 5-{[2-(diméthylamino)éthyl]amino}-2-{[4-(4-méthoxybenzoyl)pipérazin-1-yl]carbonyl}-1,3-benzothiazole-4,7-dione;
- 5-[[2-(diméthylamino)éthyl](méthyl)amino]-*N*-éthyl-4,7-dioxo-4,7-dihydro-1,3-benzothiazole-2-carboxamide ;
- *N*-éthyl-5-{[2-(4-fluorophényl)éthyl]amino}-4,7-dioxo-4,7-dihydro-1,3-benzothiazole-2-carboxamide;
et les sels de ces composés.

Seront plus particulièrement préférés les composés suivants :
- 5-{[2-(diméthylamino)éthyl]amino}-2-(morpholin-4-ylcarbonyl)-1,3-benzothiazole-4,7-dione;
- 5-{[2-(diméthylamino)éthyl]amino}-4,7-dioxo-*N*-[3-(2-oxopyrrolidin-1-yl)propyl]-4,7-dihydro-1,3-benzothiazole-2-carboxamide ;
- 5-{[2-(diméthylamino)éthyl]amino}-2-{[4-(2-furoyl)pipérazin-1-yl]carbonyl}-1,3-benzothiazole-4,7-dione ;
- 5-{[2-(diméthylamino)éthyl]amino}-*N*-(4-méthoxyphényl)-4,7-dioxo-4,7-dihydro-1,3-benzothiazole-2-carboxamide ;
- 5-{[2-(diméthylamino)éthyl]amino}-2-(pyrrolidin-1-ylcarbonyl)-1,3-benzothiazole-4,7-dione ;
- 5-{[2-(diméthylamino)éthyl]amino}-2-{[4-(2-thiénylcarbonyl)pipérazin-1-yl]carbonyl}-1,3-benzothiazole-4,7-dione ;
- *N*-1,3-benzodioxol-5-yl-5-{[2-(diméthylamino)éthyl]amino}-4,7-dioxo-4,7-dihydro-1,3-benzothiazole-2-carboxamide ;
- 5-{[2-(diméthylamino)éthyl]éthyl]amino}-*N*-(4-fluorophényl)-4,7-dioxo-4,7-dihydro-1,3-benzothiazole-2-carboxamide ;
- 6-{[2-(diméthylamino)éthyl]amino}-2-(pyrrolidin-1-ylcarbonyl)-1,3-benzothiazole-4,7-dione;
- 5-{[2-(diméthylamino)éthyl]amino}-*N*-(3-fluoro-4-méthoxyphényl)-4,7-dioxo-4,7-dihydro-1,3-benzothiazole-2-carboxamide;
- 5-{[2-(diméthylamino)éthyl]amino}-2-[(6-méthoxy-3,4-dihydroquinolin-1(2*H*)-yl)carbonyl]-1,3-benzothiazole-4,7-dione ;
- 5-{[2-(diméthylamino)éthyl]amino}-2-{[4-(4-méthoxybenzoyl)pipérazin-1-yl]carbonyl}-1,3-benzothiazole-4,7-dione;
et les sels de ces composés.

L'invention concerne également, à titre de médicaments, les composés de formule générale **(I)** mentionnés ci-dessus, ou leurs sels pharmaceutiquement acceptables.

L'invention a encore pour objet les compositions pharmaceutiques comprenant, à titre de principe actif, un composé de formule générale **(I)** ou un sel pharmaceutiquement acceptable d'un tel composé, avec au moins un excipient pharmaceutiquement acceptable.

Un autre objet de l'invention est l'utilisation des composés de formule générale **(I)** ou de leurs sels pharmaceutiquement acceptables pour préparer un médicament destiné à traiter une maladie ou un désordre choisi parmi les maladies suivantes ou les désordres suivants : les maladies prolifératives tumorales (et en particulier le cancer), les maladies prolifératives non tumorales, les maladies neurodégénératives, les maladies parasitaires, les infections virales, l'alopécie spontanée, l'alopécie induite par des produits exogènes, l'alopécie radio-induite, les maladies auto-immunes, les rejets de greffes, les maladies inflammatoires ou les allergies.

Tout particulièrement, les composés de formule générale **(I)** ou leurs sels pharmaceutiquement acceptables pourront être utilisés pour préparer un médicament destiné à traiter le cancer, et notamment le cancer du sein, les lymphomes, les cancers du cou ou de la tête, le cancer du poumon, le cancer du colon, le cancer de la prostate ou le cancer du pancréas.

L'invention concerne de plus une méthode de traitement de l'une des maladies / l'un des désordres mentionnés, ladite méthode comprenant l'administration au patient atteint de ladite maladie / dudit désordre d'un quantité thérapeutiquement efficace d'un composé de formule générale **(I)** ou d'un sel pharmaceutiquement acceptable d'un tel composé.

L'invention offre également, à titre de produits industriels nouveaux, les intermédiaires de formule générale **(A)** 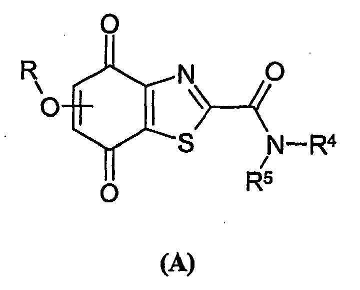 dans laquelle R est un radical alkyle,
R⁵ a la même signification que dans la formule générale **(I)**
et R⁴ représente un radical alkyle, un radical haloalkyle, un radical cycloalkyle, un radical cycloalkylalkyle, un radical alkoxyalkyle, l'un des radicaux aryle carbocyclique ou hétérocyclique ou aralkyle carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂,
ou encore R⁴ représente l'un des radicaux -(CH₂)ₘ-[O-(CH₂)ₚ]_{q}-O-Alk, -(CH₂)ᵣ-[O-(CH₂)ₛ]ₜ-NR²¹R²² ou -(CH₂)ᵥ-A dans lesquels dans lesquels m, p et s sont chacun indépendamment un entier de 2 à 4, q est un entier de 1 à 4, t est un entier de 0 à 4, r est un entier de 2 à 12 (et de préférence un entier de 2 à 8 et notamment un entier de 2 à 6) et v est un entier de 1 à 12 (et de préférence un entier de 1 à 8 et notamment un entier de 1 à 6), Alk est un radical alkyle, R²¹ est un atome d'hydrogène ou un radical alkyle, alkoxycarbonyle ou aralkoxycarbonyle, R²² est un atome d'hydrogène ou un radical alkyle et A est un hétérocycle saturé comptant de 1 à 2 hétéroatomes choisis indépendamment parmi O, N et S et attaché au groupe -(CH₂)ᵥ- par un chaînon N ou CH, ledit hétérocycle saturé comptant par ailleurs de 2 à 6 chaînons supplémentaires choisis indépendamment parmi -CHR²³-, -CO-, -NR²⁴-, -O- et -S-, R²³ représentant un atome d'hydrogène ou un radical alkyle et R²⁴ représentant un atome d'hydrogène, un radical alkyle ou un groupement alkoxycarbonyle ou aralkoxycarbonyle,
ou encore R⁴ représente un radical de formule 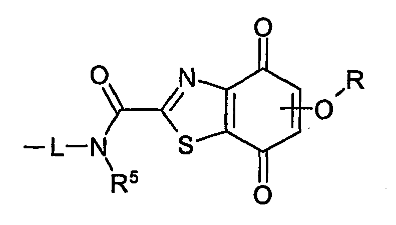 dans laquelle R et R⁵ sont identiques aux radicaux R et R⁵ mentionnés précédemment et L est choisi parmi les radicaux -(CH₂)g-[O-(CH₂)_{w}]ₓ-[O-(CH₂)_{y}]_{z}- et -(CH₂)ₐ-Ω-(CH₂)_{b}- dans lesquels g, w et y sont des entiers de 2 à 4 (et de préférence 2 à 3), x est un entier de 1 à 3 et z est 0 ou 1, a et b sont indépendamment des entiers de 2 à 6 (et de préférence 2 à 4) et Ω est choisi parmi le groupe constitué par -O-, -S-, -NR²⁵-, -CO-,-CO-NR²⁶-,-CR²⁷R²⁸-, un radical cycloalkylène comptant de 3 à 7 atomes de carbone et enfin un radical aryle carbocyclique, R²⁵ représentant un radical alkyle, R²⁶ représentant un atome d'hydrogène ou un radical méthyle, R²⁷ et R²⁸ étant chacun choisis indépendamment parmi un atome d'hydrogène et un groupe méthyle ;
ou bien encore R⁴ représente un radical de formule 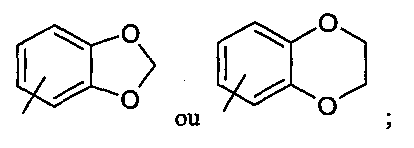 et les sels de tels composés.

Selon une variante de l'invention, lesdits composés de formule générale **(A)** seront tels que R⁴ ne représente pas un radical de formule 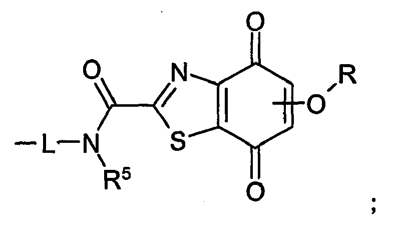 pour la suite de cet exposé, de tels composés seront appelés les « composés de sous-formule générale (**A**)**_{M}** ».

Un aspect particulier de cette variante de l'invention concerne les composés de sous-formule générale (**A**)**_{M5}** 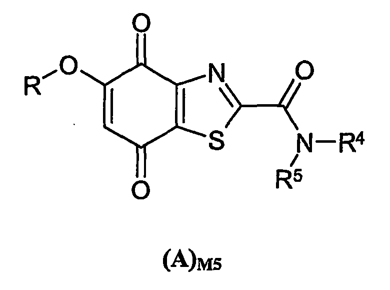 dans laquelle R, R⁴ et R⁵ ont la même signification que dans la formule générale **(A)**.

Un autre aspect de cette variante de l'invention concerne les composés de sous-formule générale **(A)_{M6}** 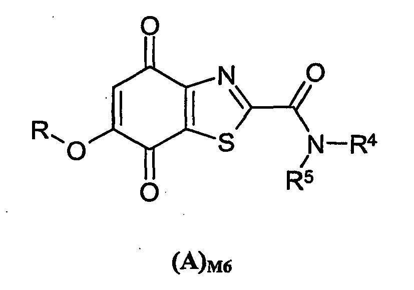 dans laquelle R, R⁴ et R⁵ ont la même signification que dans la formule générale **(A)**.

Selon une autre variante de l'invention, lesdits composés de formule générale **(A)** seront tels que R⁴ représente un radical de formule 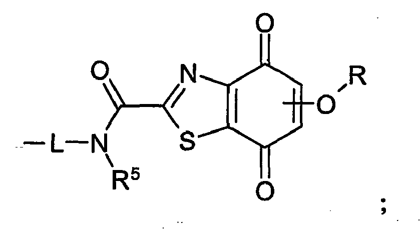 pour la suite de cet exposé, de tels composés seront appelés les "composés de sous-formule générale (**A**)**_{D}**".

Un aspect particulier de cette variante de l'invention concerne les composés de sous-formule générale (**A**)**_{D5}** 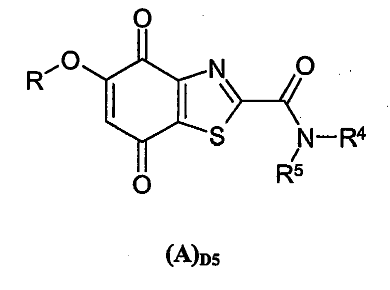 dans laquelle R et R⁵ ont la même signification que dans la formule générale **(A)** tandis que R⁴ représente un radical de formule 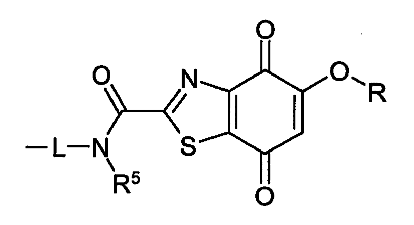 dans laquelle L, R et R⁵ ont la même signification que dans la formule générale **(A)**.

Un autre aspect de cette variante de l'invention concerne les composés de sous-formule générale **(A)_{D6}** 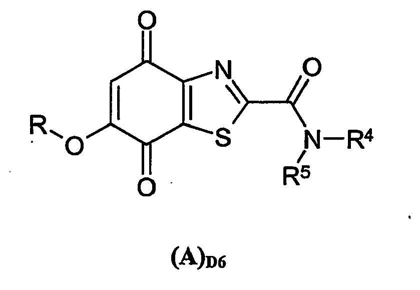 dans laquelle R et R⁵ ont la même signification que dans la formule générale **(A)** tandis que R⁴ représente un radical de formule 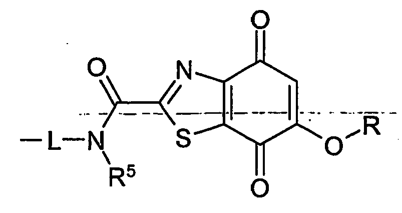 dans laquelle L, R et R⁵ ont la même signification que dans la formule générale **(A)**.

Les préférences indiquées pour les composés de formules générales **(I)**, **(I)_{M}** ou **(I)_{D}** ou leurs sels sont applicables *mutatis mutandis* à ces composés ou leurs sels pharmaceutiquement acceptables en tant que médicaments, aux compositions pharmaceutiques contenant ces composés ou leurs sels pharmaceutiquement acceptables, aux utilisations de ces composés ou de leurs sels pharmaceutiquement acceptables, aux méthodes de traitement selon l'invention ou aux intermédiaires de formules générales **(I)**, **(A)_{M}** ou **(A)_{D}** ou leurs sels.

Les compositions pharmaceutiques contenant un composé de l'invention peuvent se présenter sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par les procédés décrits ci-après.

### Préparation des composés de formule générale (I)

Deux cas sont à distinguer, selon que l'on a affaire aux composés de sous-formule générale **(I)_{M}** ou à ceux de sous-formule générale **(I)_{D}**.

Bien entendu, les procédés de préparation ci-après sont donnés à titre illustratif et l'homme du métier pourra leur faire subir les variations qu'il juge utiles, aussi bien en ce qui concerne les réactifs que les conditions et techniques des réactions.

### Méthode générale :

Les composés de formule générale **(I)_{M}** dans lesquels R³ représente un atome d'hydrogène peuvent être préparés selon la procédure résumée dans le schéma 1 ci-après.

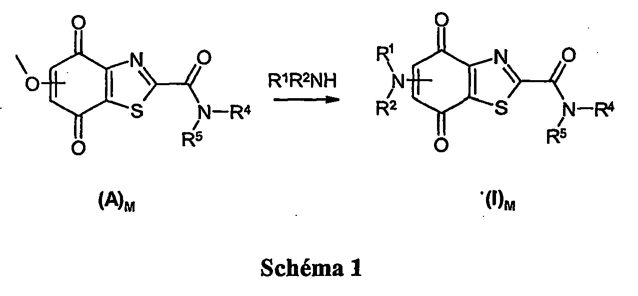

Selon cette méthode, les composés de formule générale **(I)_{M}**, dans lesquels R¹, R², R⁴ et R⁵ sont tels que décrits ci-dessus, sont obtenus par traitement des composés de formule générale **(A)_{M}**, dans lesquels R⁴ et R⁵ ont la même signification que dans la formule générale **(I)_{M}**, avec des amines de formule générale R¹R²NH dans un solvant protique tel que le méthanol ou l'éthanol, à une température comprise entre 20 °C et 80 °C et éventuellement en présence d'une base telle que, par exemple, la diisopropyléthylamine (cf. Yasuyuki Kita et coll., J. Org. Chem. (1996), 61, 223-227).

Les composés de formule générale **(I)_{D}** dans lesquels R³ représente un atome d'hydrogène peuvent être préparés selon la procédure résumée dans le schéma 1*bis* ci-après.

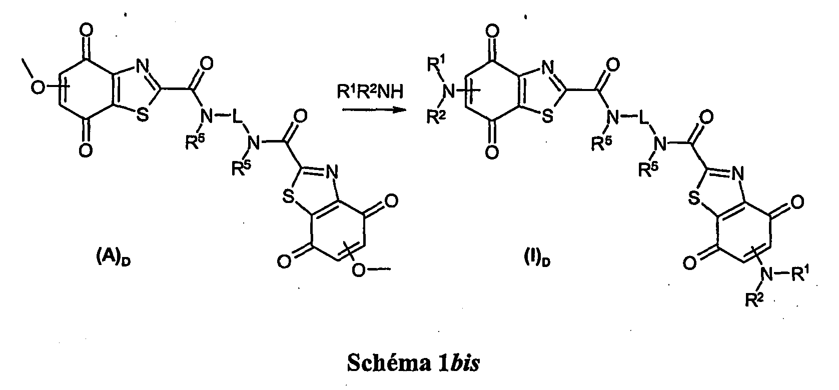

Selon cette méthode, les composés de sous-formule générale **(I)_{D}**, dans lesquels R¹, R², L et R⁵ sont tels que décrits ci-dessus, sont obtenus par traitement des composés de formule générale **(A)_{D}**, dans lesquels L et R⁵ ont la même signification que dans la sous-formule générale **(I)_{D}**, avec des amines de formule générale R¹R²NH dans un solvant protique tel que le méthanol ou l'éthanol, à une température comprise entre 20 °C et 80 °C et éventuellement en présence d'une base telle que, par exemple, la diisopropyléthylamine (cf. Yasuyuki Kita et coll., J. Org. Chem. (1996), 61, 223-227).

D'une façon générale, les composés de sous-formules générales **(I)_{M}** ou **(I)_{D}** dans lesquels R³ représente un atome halogène (Hal) peuvent être obtenus à partir des composés de sous-formules générales **(I)_{M}** ou **(I)_{D}** correspondants dans lesquels R³ représente un atome d'hydrogène, par exemple par action de *N*-chlorosuccinimide ou N-bromosuccinimide dans un solvant aprotique tel que le dichlorométhane ou le tétrahydrofuranne (Paquette et Farley, J. Org. Chem. (1967), 32, 2725-2731), par action d'une solution aqueuse d'hypochlorite de sodium (eau de Javel) dans un solvant tel que l'acide acétique (Jagadeesh et coll., Synth Commun. (1998), 28, 3827-3833), par action de Cu(II) (dans un mélange CuCl₂/HgCl₂) en présence d'une quantité catalytique d'iode dans un solvant tel que l'acide acétique à chaud (Thapliyal, Synth. Commun. (1998), 28, 1123-1126), par action d'un agent tel que le dichloroiodate de benzyltriméthylammonium en présence de NaHCO₃ dans un solvant tel qu'un mélange dichlorométhane / méthanol (Kordik et Reitz, J. Org. Chem. (1996), 61, 5644-5645), ou encore par utilisation de chlore, de brome ou d'iode dans un solvant tel que le dichlorométhane (J. Renault, S. Giorgi-Renault et coll., J. Med. Chem. (1983), 26, 17151719). (cf. schéma 1*ter* où seuls les composés de sous-formule générale **(I)_{M}** sont représentés).

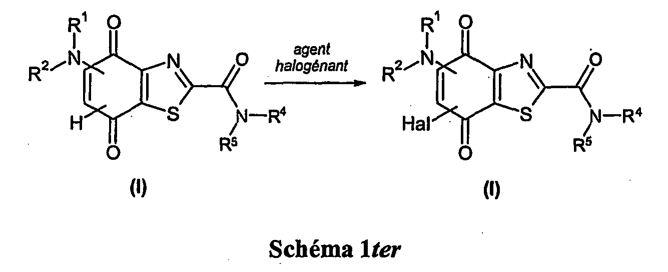

### Préparation des intermédiaires de formule générale (A)_{M} et (A)_{D} :

Les composés de sous-formule générale **(A)**_{M}, dans lesquels R⁴ et R⁵ sont tels que définis ci-dessus, peuvent être obtenus, schéma 2, à partir des composés de formule générale (B)_{M} dans lesquels R⁴ et R⁵ sont tels que définis ci-dessus et l'un de Q et Q' représente un radical amino tandis que l'autre représente un atome d'hydrogène.

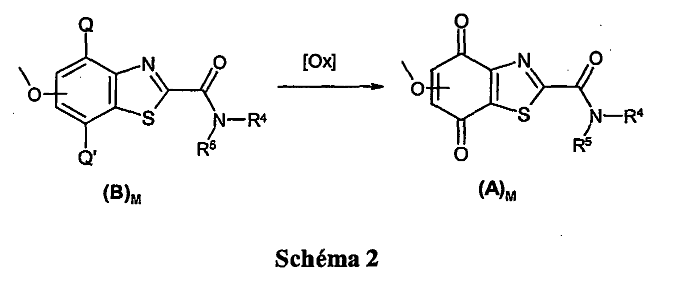

De la même façon, les composés de formule générale (A)_{D}, dans lesquels L et R⁵ sont tels que définis ci-dessus, peuvent être obtenus, schéma *2bis,* à partir des composés de formule générale **(B)_{D}** dans lesquels L et R⁵ sont tels que définis ci-dessus et l'un de Q et Q' représente un radical amino tandis que l'autre représente un atome d'hydrogène.

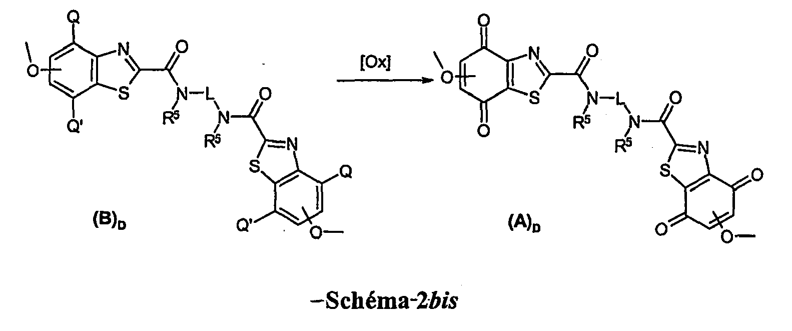

Les composés de formule générale **(A)**_{M} ou (**A**)_{D} sont obtenus par oxydation des composés de formule générale (**B**)_{M} ou (**B**)_{D} respectivement, par exemple par utilisation d'une résine échangeuse d'ion de type Dowex sous forme hypochlorite en milieu anhydre (Hashemi et coll., J. Chem. Res., Synop. (1999), 11, 672-673) ou de sel de Fremy (nitrosodisulfonate de potassium) (Ryu et coll., Bioorg. Med. Chem. Lett. (2000), 10, 461-464), ou par l'utilisation d'un réactif comportant un iode hypervalent tel que le [bis(acétoxy)iodo]benzène ou le [bis(trifluoroacétoxy)iodo]benzène dans l'acétonitrile aqueux à une température de préférence comprise entre -20 °C et la température ambiante (soit environ 25 °C), et de préférence à environ -5 °C (Kinugawa et coll., Synthesis, (1996), 5, 633-636).

### Préparation des intermédiaires de formule générale (B)_{M} et (B)_{D} :

Les composés de formule générale (B)_{M}, dans lesquels Q, Q', R⁴ et R⁵ sont tels que définis ci-dessus, peuvent être obtenus à partir des dérivés nitro de formule (C)_{M} dans lesquels R⁴ et R⁵ sont tels que définis ci-dessus et l'un de Q et Q' représente un radical nitro tandis que l'autre est un atome d'hydrogène, par des méthodes de réduction bien connues de l'homme de l'art telles que, par exemple l'hydrogénation en présence d'un catalyseur palladié ou le traitement par du chlorure d'étain dans l'acide chlorhydrique.

La préparation des composés de formule générale (B)_{M} dans lesquels le groupe méthoxy est en position 5 est représentée dans le schéma 3 ci-dessous pour le cas où, par exemple, Q = NH₂ et Q' = H.

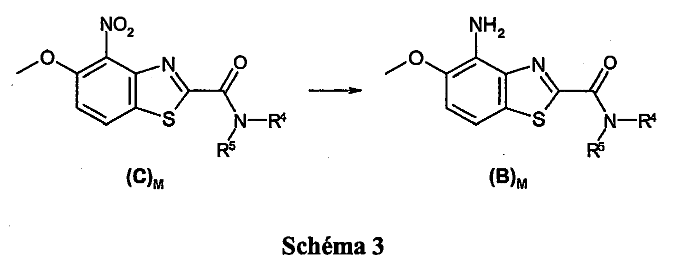

La préparation des composés de formule générale (**B**)_{M} dans lesquels le groupe méthoxy est en position 6 est représentée dans le schéma 3*bis* ci-dessous pour le cas où, par exemple, Q = H et Q' = NH₂.

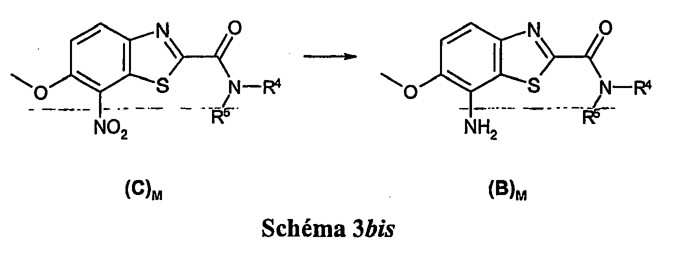

De la même façon, les composés de formule générale (**B**)_{D}, dans lesquels Q, Q', L et sont tels que définis ci-dessus, peuvent être obtenus à partir des dérivés nitro de formule (**C**)_{D} dans lesquels L et R⁵ sont tels que définis ci-dessus et l'un de Q et Q' représente un radical nitro tandis que l'autre représente un atome d'hydrogène (voir les schémas *3ter* et *3quater* ci-dessous).

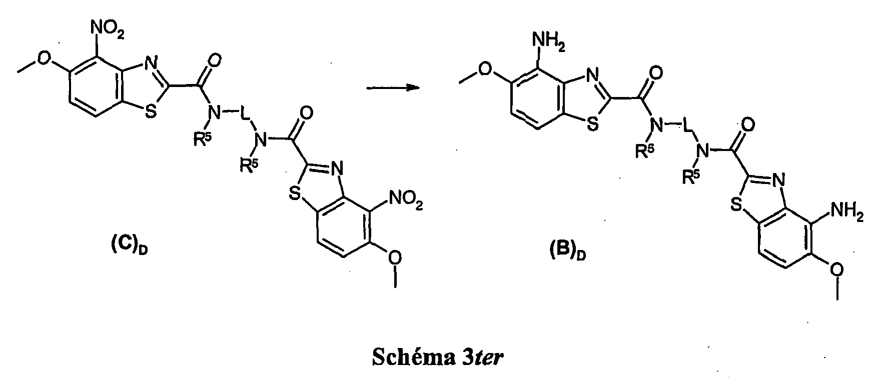

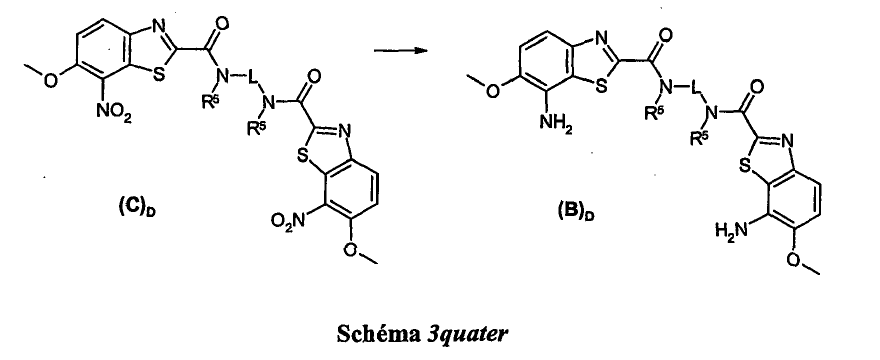

### Préparation des intermédiaires de formule générale (C)_{M} et(C)_{XD} :

Les composés de formule générale (**C**)_{M} dans lesquels R⁴ et R⁵ sont tels que définis ci-dessus et l'un de Q et Q' représente un radical nitro tandis que l'autre représente un atome d'hydrogène, peuvent être obtenus, schéma 4, à partir des composés de formule générale (**D**), dans lesquels Q et Q' sont tels que définis ci-dessus, et des aminés de formule générale R⁴R⁵NH.

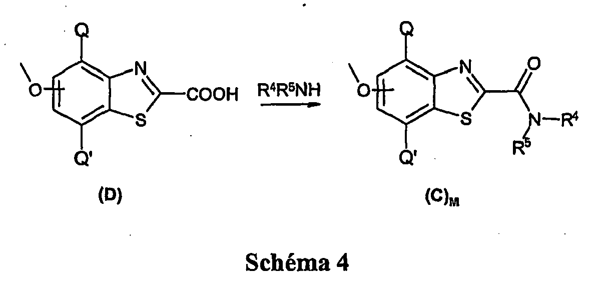

De le même façon, les composés de formule générale (**C**)_{D} dans lesquels L et R⁵ sont tels que définis ci-dessus et l'un de Q et Q' représente un radical nitro tandis que l'autre représente un atome d'hydrogène, peuvent être obtenus, schéma *4bis,* à partir des composés de formule générale (**D**), dans lesquels Q et Q' sont tels que définis ci-dessus, et des diamines de formule générale R⁵HN-L-NHR⁵.

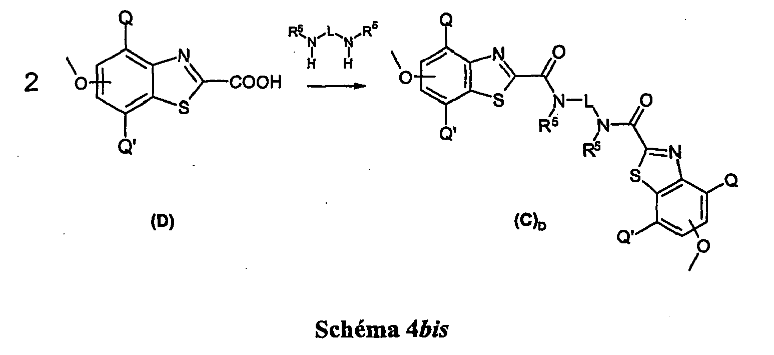

Les composés de formule générale (**C**)_{M} ou (**C**)_{D} sont obtenus comme indiqué ci-dessus en utilisant les conditions classiques de la synthèse peptidique (M. Bodansky, The Practice of Peptide Synthesis, 145 (Springer-Verlag, 1984)), par exemple dans le dichlorométhane en présence d'un réactif de couplage tel que l'hexafluorophosphate de bromo-tris-pyrrolidino-phosphonium (PyBroP) en présence de diméthylaminopyridine (DMAP) (Coste et coll., Tetrahedron Lett. (1990), 31, 669), ou dans un mélange (diméthylformamide/dichlorométhane/dioxane : 1/1/1) en présence de chlorhydrate de 1-(3-diméthylaminopropyl)-3-éthylcarbodiimide, d'une quantité catalytique de diméthylaminopyridine et de diisopropyléthylamine, ou encore par formation d'un chlorure d'acide intermédiaire obtenu par addition de chlorure d'oxalyle en solution dans du dichlorométhane.

### Préparation des intermédiaires de formule générale (D) :

Les composés de formule générale (**D**), dans lesquels Q et Q' sont tels que définis ci-dessus, peuvent être obtenus, schéma 5, par oxydation des carboxaldéhydes de formule générale (**E.i**) par action d'un oxydant tel que, par exemple du chlorite de sodium dans une solution tamponnée d'hydrogénophosphate de sodium (pH 3,5) et dans une solution aqueuse de tert-butanol en présence de 2-méthyl-2-butène ; ces aldéhydes de formule générale (**E.i**) étant eux-mêmes obtenus par oxydation des composés de formule générale (**E**) par action, par exemple, d'oxyde de sélénium dans du 1,4-dioxane à 80 °C (Bu et coll., J. Med Chem. (2001), 44, 2004-2014).

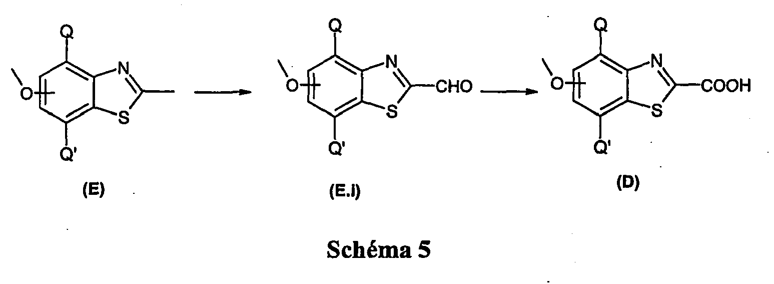

### Préparation des intermédiaires de formule générale (E) :

Les composés de formule générale (**E**), dans lesquels Q et Q' sont tels que définis ci-dessus, peuvent être obtenus à partir des composés de formule générale (F), dans lesquels les positions correspondant aux radicaux Q et Q' sont substituées par des atomes d'hydrogène, par des méthodes de nitration bien connues de l'homme de l'art telles que, par exemple, le traitement par un mélange d'acide nitrique et d'acide sulfurique.

La préparation des composés de formule générale (E) dans lesquels le groupe méthoxy est en position 5 est représentée dans le schéma 6 ci-dessous pour le cas où, par exemple, Q = NO₂ et Q' = H.

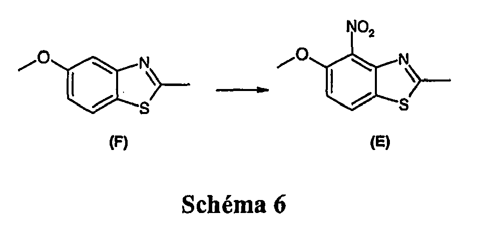

La préparation des composés de formule générale (**E**) dans lesquels le groupe méthoxy est en position 6 est représentée dans le schéma *6bis* ci-dessous pour le cas où, par exemple, Q = H et Q' = NO₂.

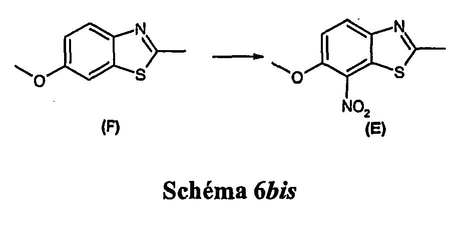

### Composés de formule générale (F) :

Les composés de formule générale (**F**) sont des produits industriels connus disponibles chez les fournisseurs usuels ou peuvent être synthétisés à partir de tels produits selon des méthodes courantes pour l'homme du métier.

En ce qui concerne les températures auxquelles il est fait référence dans le présent texte, le terme "environ *XX°*C" indique que la température en question correspond à un intervalle de plus ou moins 10 °C autour de la température de *XX* °C*,* et de préférence à un intervalle de plus ou moins 5 °C autour de la température de *XX* °C.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

Les points de fusion ont été mesurés à l'aide d'un appareil à capillaire Büchi 535.

Les spectres de RMN ont été enregistrés à l'aide d'un spectromètre Brücker ARX 400. Les déplacements chimiques sont exprimés en partie par million (ppm) par rapport au trétraméthylsilane (TMS) et la multiplicité des signaux est donnée sous forme de s (singulet), d (doublet), t (triplet), m (multiplet).

### EXEMPLES

### Méthode employée pour la mesure du temps de rétention (t.r.) et du pic moléculaire (MH+).

Les composés sont caractérisés par leur temps de rétention (t.r.), exprimé en minutes, déterminé par chromatographie liquide (CL), et leur pic moléculaire (MH+) déterminé par spectrométrie de masse (SM), un spectromètre de masse simple quadripôle (Micromass, modèle Platform) équipé d'une source électrospray est utilisé avec une résolution de 0,8 da à 50 % de vallée.

Pour les exemples 1 à 15 ci-après, les conditions d'élution correspondant aux résultats indiqués sont les suivantes : élution avec le mélange acétonitrile-eau-acide trifluoroacétique 50-950-0,2 (A) pendant 1 minute puis passage du mélange (A) à un mélange acétonitrile-eau 950-50 (B) par un gradient linéaire sur une période de 7,5 minutes avant élution avec le mélange B pur pendant 2 minutes.

### Exemple 1 : 5-{[2-(diméthylamino)éthyl]amino}-2-(morpholin-4-ylcarbonyl) 1,3-benzothiazole-4,7-dione :

### 1.1) Acide 5-méthoxy-4-nitro-1,3-benzothiazole-2-carboxylique :

### 1.1.1) 5-méthoxy-4-nitro-1, 3-benzothiazole-2-carbaldéhyde :

12,8 g (0,115 mol; 6 équivalents) de dioxyde de sélénium sont ajoutés à 4,34 g (19,3 mmol) de 5-méthoxy 2-méthyl-4-nitro-1,3-benzothiazole en solution dans 180 ml de dioxane anhydre. Le mélange réactionnel est agité à 80 °C pendant 18 heures puis l'insoluble est filtré et le solvant est évaporé sous pression réduite. L'aldéhyde attendu est obtenu sous forme d'une huile jaune et purifié sur colonne de silice (éluant : acétate d'éthyle / heptane : gradient de 30 % à 70 %). Point de fusion : 154-155 °C.
RMN ¹H (DMSO d6, 400 MHz, δ) : 10,06 (s, 1H, CHO) ; 8,52-8,49 (d, 1H, H arom) ; 7,80-7,78 (d, 2H, H arom) ; 4,04 (s, 3H, OCH₃).

### 1.1.2) Acide 5-méthoxy-4-nitro-1,3-benzothiazole-2-carboxylique:

Une solution de 18 g de chlorite de sodium et de 18 g d'hydrogénophosphate de sodium dans 180 ml d'eau est ajoutée goutte à goutte au résidu carbaldéhyde repris dans 420 ml de tert-butanol et 100 ml de 2-méthyl-but-2-ène. Le mélange réactionnel est maintenu sous agitation pendant 18 heures à température ambiante, puis l'insoluble est filtré, repris par de l'eau et la solution aqueuse obtenue est acidifiée par une solution 1*M* d'acide chlorhydrique. Le précipité obtenu est filtré et lavé par de l'eau. L'acide est obtenu sous forme d'une poudre beige. (m = 3,12 g; rendement = 64 %). Point de fusion : 140-142 °C.
RMN ¹H (DMSO d6, 400 MHz, δ) : 8,45-8,43 (d, 1H, H arom) ; 7,74-7,71 (d, 1H, H arom) ; 3,99 (s, 3H, CH₃).
SM-CL : MH+ = 254,99 ; t.r. = 8,20 min.

### 1.2) 5-Méthoxy-2-(morpholin-4 ylcarbonyl)-4-nitro-1,3-benzothiazole :

A 500 mg (1,97 mmol) d'acide 5-méthoxy 4-nitro-1,3-benzothiazole-2-carboxylique et 344 µl (1,97 mmol; 1 éq.) de diisopropyléthylamine en solution dans 40 ml de dichlorométhane, sont ajoutés 980 mg (2,1 mmol ; 1,1 éq.) d'hexafluorophosphate de bromo-tris-pyrrolidino-phosphonium (PyBroP). Le mélange réactionnel est maintenu pendant 15 minutes sous agitation à température ambiante, puis 202 µl (2,3 mmol ; 1,2 éq.) de morpholine et une pointe de spatule de diméthylaminopyridine sont ajoutés au milieu qui est maintenu sous agitation pendant 18 heures à température ambiante. Un insoluble est ensuite filtré et le solvant évaporé sous pression réduite. Le résidu est ensuite purifié sur colonne de silice (éluant : acétate d'éthyle / heptane : gradient de 30 à 70 % sur 40 minutes puis pendant 5 minutes à 70 % d'acétate d'éthyle dans l'heptane) et 210 mg (rendement = 33 %) de produit attendu sont obtenus sous forme de poudre beige.
SM-CL : MH+ = 324,01 ; t.r. = 9,63 min.

### 1.3) 5 Méthoxy-2-(morpholin-4-ylcarbonyl)-1,3-benaothiazol-4-amine :

A 210 mg (0,65 mmol) de 5-méthoxy-2-(morpholin-4-ylcarbonyl)-4-nitro-1,3-benzothiazole en solution dans 10 ml de méthanol sont ajoutés 20 mg de palladium à 10 % sur charbon activé. Le milieu réactionnel est ensuite placé sous agitation sous atmosphère d'hydrogène pendant 18 heures. Le catalyseur est ensuite filtré et le solvant évaporé. 173 mg du produit attendu (rendement brut = 91 %) sont obtenus sous forme d'une huile jaune et sont utilisés dans l'étape suivante sans autre purification.
SM=CL:MH+=294;04 t;r.=9,09 min.

### 1.4) 5-méthoxy-2-(morpholin-4-ylcarbonyl)-1,3-benzothiazole-4,7-dione

Une solution de 570 mg (1,063 mmol ; 1,8 éq.) de sel de Fremy (nitrosodisulfonate de potassium, contenant de 25 à 50 % d'eau et de méthanol) dans une solution aqueuse à 0,3M d'hydrogénophosphate de sodium (12 ml) est ajoutée goutte à goutte à 173 mg (0,6 mmol) de 5-méthoxy-2-(morpholin-4-ylcarbonyl)-1,3-benzothiazol-4-amine en solution dans 5 ml d'acétone. Le mélange réactionnel est maintenu sous agitation à température ambiante pendant 4 heures, puis l'acétone est évaporée et le milieu est repris par 10 ml de dichlorométhane et lavé par 2 fois 7 ml d'une solution aqueuse saturée en chlorure de sodium. Les phases organiques sont réunies, séchées sur sulfate de magnésium et le solvant évaporé sous pression réduite. 175 mg (rendement brut = 99 %) de produit attendu est obtenu sous forme d'une poudre jaune et est utilisé dans l'étape suivante sans autre purification.
SM-CL : MH+ = 308,99 ; t.r. = 8,40 min.

### 1.5) 5-{[2-(diméthylamino)éthyl]amino}-2-(morpholin-4-ylcarbonyl)-1,3-benzothiazole-4,7-dione :

66 µl (0,6 mmol ; 1 éq.) de *N*,*N*-diméthyléthylènediamine sont ajoutés à 175 mg de 5-méthoxy-2-(morpholin-4-ylcarbonyl)-1,3-benzothiazole-4,7-dione en solution dans 10 ml d'éthanol anhydre. Le mélange réactionnel est agité à 80 °C pendant 2 heures puis le solvant est évaporé sous pression réduite. Le résidu est purifié sur colonne de silice (éluant : méthanol dans le dichlorométhane : gradient de 0 à 5 % sur 35 minutes puis pendant 5 minutes à 5 % de méthanol dans le dichlorométhane) et 50 mg (rendement = 23 %) de composé attendu sont obtenus sous forme d'une poudre rouge. Point de fusion = 207-208 °C.
RMN ¹H (DMSO d6, 400 MHz, δ) : 7,56 (t, 1H, NH) ; 5,61 (s, 1H, CH) ; 4,24 (m, 2H) ; 3,69 (m, 6H) ; 3,26 (m, 2H, CH₂) ; 2,49 (m, 2H, CH₂) 2,19 (s, 6H, 2CH₃).
SM-CL : MH+ = 365,06 ; t.r. = 7,19 min.

*Les composés des exemples 2 à 13 sont obtenus selon un protocole analogue à celui employé pour l'exemple 1, les amines adéquates remplaçant la morpholine dans la deuxième étape et la N (2-aminoéthyl)pyrrolidine remplaçant la N,N-diméthyléthylènediamine dans la dernière étape pour l'exemple 6.*

### Exemple 2 : {2-[2-(2-{[(5-{[2-(diméthylamino)éthyl]amino}-4,7-dioxo-4,7-dihydro-1,3-benzothiazol-2-yl)carbonyl]amino}éthoxy)éthoxy]éthyl}carbamate de tert-butyle :

Poudre rouge. Point de fusion: 55-57 °C.
RMN ¹H (DMSO d6, 400 MHz, δ) : 9,10 (t, 1H, NH) ; 7,54 (t, 1H, NH) ; 6,69 (t, 1H, NH) ; 5,62 (s, 1H, CH) ; 3,56-3,43 (m, 8H) ; 3,37-3,35 (m, 2H) ; 3,29-3,25 (m, 2H) ; 3,04-3,02 (m, 2H, CH₂) ; 2,51 (m, 2H, CH₂) ; 2,18 (s, 6H, 2CH₃) ; 1,35 (s, 9H, 3CH₃).
SM-CL : MH+ = 526,34 ; t.r. = 7,80 min.

### Exemple 3 : N,N-dibenzyl-5-{[2-(diméthylamino)éthyl]amino}-4,7-dioxo-4,7-dihydro-1,3-benzothiazole-2-carboxamide :

Poudre rouge.
RMN ¹H (DMSO d6, 400 MHz, δ) : 7,51 (t, 1H, NH) ; 7,36-7,27 (m, 10H, H arom) ; 5,61 (s, 1H, CH) ; 5,26 (s, 2H, CH₂) ; 4,61 (s, 2H, CH₂) ; 3,26-3,23 (m, 2H, CH₂) ; 2,46-2,44 (m, 2H, CH₂) ; 2,17 (s, 6H, 2CH₃).
SM-CL : MH+ = 475,44 ; t.r. = 8,98 min.

### Exemple 4 : 5-{[2-(diméthylamino)éthyl]amino}-N,N-bis(2-méthoxyéthyl)-4,7-dioxo-4,7-dihydro-1,3-benzothiazole-2-carboxamide :

Poudre rouge. Point de fusion: 92-94 °C.
RMN ¹H (DMSO d6, 400 MHz, δ) : 7,53-7,52 (t, 1H, NH) ; 5,60 (s, 1H, CH) ; 4,18-4,15 (m, 2H, CH₂) ; 3,69-3,67 (m, 2H, CH₂); 3,56-3,53 (m, 4H, 2 CH₂) ; 3,29-3,24 (m, 5H) ; 3,19 (m, 3H, CH₃) ; 2,48-2,27 (m, 2H, CH₂) ; 2,19 (s, 6H, 2 CH₃).
SM-CL : MH+ = 411,43 ; t.r. = 7,52 min.

### Exemple 5: 5-{[2-(diméthylamino)éthyl]amino}-4,7-dioxo-N-[3-(2-oxopyrrolidin-1-yl)propyl]-4,7-dihydro-1,3-benzothiazole-2-carboxamide :

Poudre rouge. Point de fusion: 154-156 °C.
RMN ¹H (DMSO d6, 400 MHz, δ) : 9,19 (t, 1H, NH) ; 7,55 (t, 1H, NH) ; 5,61 (s, 1H, CH) ; 3,37-3,18 (m, 10H) ; 2,51-2,49 (m, 2H, CH₂) ; 2,20-2,19 (m, 6H, 2 CH₃) ; 1,93-1,89 (m, 2H, CH₂) ; 1,74-1,69 (m, 2H, CH₂).
LC-MS : MH+ = 420,14 ; t.r. = 7,26 min.

### Exemple 6 : 4,7-dioxo-N-[3-(2-oxopyrrolidin-1-yl)propyl]-5-[(2-pyrrolidin-1-yléthyl)amino]-4,7-dihydro-1,3-benzothiazole-2-carboxamide :

Poudre rouge.
RMN ¹H (DMSO d6, 400 MHz, δ) : 9,18 (t, 1H, NH) ; 7,67 (t, 1H, NH) ; 5,61 (s, 1H, CH) ; 3,37-3,18 (m, 10H) ; 2,68-2,67 (t, 2H, CH₂) ; 2,49 (m, 2H, CH₂) ; 2,20 (t, 2H, CH₂) ; 1,93-1,89 (m, 2H, CH₂) ; 1,74-1,68 (m, 6H).
SM-CL : MH+ = 446,17 ; t.r. = 7,32 min.

### Exemple 7 : 5-{[2-(diméthylamino)éthyl]amino}-N-isobutyl-4,7-dioxo-4,7-dihydro-l,3-benzothiazole-2-carboxamide :

Poudre rouge. Point de fusion: 163-164 °C.
RMN ¹H (DMSO d6, 400 MHz, δ) : 9,21 (t, 1H, NH) ; 7,54 (t, 1H, NH) ; 5,61 (s, 1H, CH) ; 3,28-3,23 (m, 2H, CH₂) ; 3,11-3,08 (m, 2H, CH₂) ; 2,49 (m, 2H, CH₂) ; 2,18 (m, 6H, 2 CH₃) ; 1,92-1,89 (m, 1H, CH) ; 0,88-0,86 (m, 6H, 2 CH₃).
SM-CL : MH+ = 351,16 ; t.r. = 7,71 min.

### Exemple 8 : (4-{[(5-{[2-(diméthylamino)éthyl]amino}-4,7-dioxo-4,7-dihydro-1,3-benzothiazol-2-yl)carbonyl]amino}butyl)carbamate de tert-butyle :

### Poudre rouge.

RMN ¹H (DMSO d6, 400 MHz, δ) : 9,21 (t, 1H, NH) ; 7,54 (t, 1H, NH); 6,77 (t, 1H, NH) ; 5,61 (s, 1H, CH) ; 3,28-3,24 (m, 4H, 2CH₂) ; 2,93-2,90 (m, 2H, CH₂) ; 2,49 (m, 2H, CH₂) ; 2,18 (s, 6H, 2CH₃) ; 1,53-1,49 (m, 2H, CH₂) ; 1,39-1,32 (m, 11H).
SM-CL : MH+ = 466,25 ; t.r. = 7,75 min.

### Exemple 9 : 5-{[2-(diméthylamino)éthyl]amino}-2-{[4-(2-furoyl)pipérazin-1-yl] carbonyl)-1,3-benzothiazole-4,7-dione :

Poudre rouge.
RMN ¹H (DMSO d6, 400 MHz, δ) : 7,86 (m, 1H, H arom) ; 7,58 (t, 1H, NH) ; 7,06 (m, 1H, H arom) ; 6,64 (m, 1H, H arom) ; 5,62 (s, 1H, CH) ; 4,32 (m, 2H) ; 3,82-3,78 (m, 6H) ; 3,26-3,25 (m, 2H, CH₂) ; 2,49 (m, 2H, CH₂) ; 2,18 (s, 6H, 2CH₃).
SM-CL : MH+ = 458,08 ; t.r. = 7,39 min.

### Exemple 10 : 5-{[2-(diméthylamino)éthyl]amino}-N-(2-méthoxyéthyl)-4,7-dioxo-4,7-dihydro-1,3-benzothiazole-2-carboxamide :

Poudre rouge.
RMN ¹H (DMSO d6, 400 MHz, δ) : 9,11 (t, 1H, NH) ; 7,55 (t, 1H, NH) ; 5,61 (s, 1H, CH) ; 3,50-3,44 (m, 4H) ; 3,27-3,24 (m, 5H) ; 2,49 (m, 2H, CH₂) ; 2,18 (s, 6H, 2CH₃).
SM-CL : MH+= 353,11 ; t.r. = 7,27 min.

### Exemple 11 : N-butyl-5-{[2-(diméthylamino)éthyl]amino)-4,7-dioxo-4,7-dihydro-1,3-benzothiazole-2-carboxamide :

Poudre rouge.
RMN ¹H (DMSO d6, 400 MHz, δ) : 9,20 (t, 1H, NH) ; 7,54 (t, 1H, NH) ; 5,61 (s, 1H, CH) ; 3,28-3,25 (m, 4H) ; 2,49 (m, 2H, CH₂) ; 2,18 (s, 6H, 2CH₃) ; 1,54-1,50 (m, 2H, CH₂) ; 1,32-1,27 (m, 2H, CH₂) ; 0,88 (t, 3H, CH₃).
SM-CL : MH+ = 351,10 ; t.r. = 7,78 min.

### Exemple 12 : N-benzyl-5-{[2-(diméthylamino)éthylamino}-4,7-dioxo-4,7-dihydro-1,3-benzothiazole-2-carboxamide :

Poudre rouge. Point de fusion: 188-189 °C.
RMN ¹H (DMSO d6, 400 MHz, δ) : 9,78 (t, 1H, NH) ; 7,55 (t, 1H, NH) ; 7,34-7,23 (m, 5H, H arom) ; 5,62 (s, 1H, CH) ; 4,46-4,45 (m, 2H, CH₂) ; 3,27-3,24 (m, 2H, CH₂) ; 2,49 (m, 2H, CH₂) ; 2,19 (m, 6H, 2 CH₃)..
SM-CL : MH+= 385,09 ; t.r. = 7,89 min.

### Exemple 13: N-(cyclohexylméthyl)-5-{[2-(diméthylamino)éthyl]amino}-4,7-dioxo-4,7-dihydro-1,3-benzothiazole-2-carboxamide :

Poudre rouge. Point de fusion: 171-172 °C.
RMN ¹H (DMSO d6, 400 MHz, δ) : 9,17 (t, 1H, NH) ; 7,54 (t, 1H, NH) ; 5,61 (s, 1H, CH) ; 3,27-3,24 (m, 2H, CH₂) ; 3,13-3,10 (m, 2H, CH₂) ; 2,49 (m, 2H, CH₂) ; 2,18 (m, 6H, 2 CH₃) ; 1,67-1,60 (m, 6H) ; 1,22-1,13 (m, 3H) ; 0,95-0,89 (m, 2H).
SM-CL : MH+ = 391,13 ; t.r. = 8,23 min.

### Exemple 14: N,N'-(oxydiéthane-2,1-diyl)bis(5-{[2-(diméthylamino)éthyl]amino)-4,7-dioxo-4,7-dihydro-1,3-benzothiazole-2-carboxamide) :

### 14.1) Acide 5-méthoxy-4-nitro-1,3-benzothiazole-2-carboxylique :

### 14.1.1) 5-méthoxy-4-nitro-1,3-benzothiazole-2-carbaldéhyde :

12,8 g (0,115 mol; 6 équivalents) de dioxyde de sélénium sont ajoutés à 4,34 g (19,3 mmol) de 5-méthoxy-2-méthyl-4-nitro-1,3-benzothiazole en solution dans 180 ml de dioxane anhydre. Le mélange réactionnel est agité à 80 °C pendant 18 heures puis l'insoluble est filtré et le solvant est évaporé sous pression réduite. L'aldéhyde attendu est obtenu sous forme d'une huile jaune et purifié sur colonne de silice (éluant : acétate d'éthyle / heptane : gradient de 30 % à 70 %). Point de fusion : 154-155 °C.
RMN ¹H (DMSO d6, 400 MHz, δ) : 10,06 (s, 1H, CHO) ; 8,52-8,49 (d, 1H, H arom) ; 7,80-7,78 (d, 2H, H arom) ; 4,04 (s, 3H, OCH₃).

### 14.1.2) Acide 5-méthoxy-4-nitro-1,3-benzothiazole-2-carboxylique :

Une solution de 18 g de chlorite de sodium et de 18 g d'hydrogénophosphate de sodium dans 180 ml d'eau est ajoutée goutte à goutte au résidu carbaldéhyde repris dans 420 ml de tert-butanol et 100 ml de 2-méthyl-but-2-ène. Le mélange réactionnel est maintenu sous agitation pendant 18 heures à température ambiante, puis l'insoluble est filtré, repris par de l'eau et la solution aqueuse obtenue est acidifiée par une solution 1M d'acide chlorhydrique. Le précipité obtenu est filtré et lavé par de l'eau. L'acide est obtenu sous forme d'une poudre beige. (m = 3,12 g; rendement = 64 %). Point de fusion : 140-142 °C.
RMN ¹H (DMSO d6, 400 MHz, δ) : 8,45-8,43 (d, 1H, H arom) ; 7,74-7,71 (d, 1H, H arom) ; 3,99 (s, 3H, CH₃).
SM-CL :MH+ =254,99 ;t.r.=8,20 min.

### 14.2) N,N'-(oxydiéthane-2,1-diyl)bis(5-méthoxy-4-nitro-1,3-benzothiazole-2-carboxamide) :

A 1,5 g (5,9 mmol) d'acide 5-méthoxy-4-nitro-1,3-benzothiazole-2-carboxylique et 1,13 ml (6,5 mmol; 1,1 éq.) de diisopropyléthylamine en solution dans 100 ml de dichlorométhane, sont ajoutés 3,03 g (6,5 mmol ; 1,1 éq.) d'hexafluorophosphate de bromo-tris-pyrrolidino-phosphonium (PyBroP). Le mélange réactionnel est maintenu pendant 10 minutes sous agitation à température ambiante, puis 0,31 g (2,9mmol; 0,5 éq.) de 2,2'-oxybis(éthylamine) et une pointe de spatule de diméthylaminopyridine sont ajoutés au milieu qui est maintenu sous agitation pendant 18 heures à température ambiante. Un insoluble est filtré et le solvant évaporé sous pression réduite. Le résidu est ensuite purifié sur colonne de silice (éluant : acétate d'éthyle / heptane : 4/1) et 250 mg de produit attendu (rendement = 8 %) sont obtenus sous forme de poudre beige.
SM-CL : MH+ = 577,11 ; t.r. = 10,27 min.

### 14.3) NN'-(oxydiéthane-2,1-diyl)bis(4-amino-5-méthoxy-1,3-benzothiazole, 2-carboxamide) :

A 250 mg (0,43 mmol) de *N,N*'-(oxydiéthane-2,1-diyl)bis(5-méthoxy-4-nitro-1,3-benzothiazole-2-carboxamide) en solution dans 10 ml d'acide chlorhydrique concentré, sont ajoutés 0,33 g (1,5 mmol ; 3,4 éq.) de chlorure d'étain dihydraté. Le milieu réactionnel est maintenu sous agitation à 60°C pendant 4 heures, puis versé sur de l'eau glacée et neutralisé par une solution de soude 5N. Le produit attendu est ensuite extrait par 3 fois 25 ml de dichlorométhane, puis les phases organiques sont réunies, séchées sur sulfate de magnésium et le solvant évaporé sous pression réduite. 220 mg du produit attendu (rendement brut = 98 %) sont obtenus sous forme d'une huile jaune et sont utilisés dans l'étape suivante sans autre purification.
SM-CL : MH+ = 517,23 ; t.r. = 9,59 min.

### 14.4) N,N'-(oxydiéthane-2,1-diyl)bis(5-méthoxy-4,7-dioxo-4,7-dihydro-1,3-benzothiazole-2-carboxamide) :

Une solution de 0,91 g (1,7 mmol ; 4 éq.) de sel de Fremy (nitrosodisulfonate de potassium, contenant de 25 à 50 %-d'eau et de méthanol) dans une solution aqueuse à 0,3M d'hydrogénophosphate de sodium (35 ml) est ajoutée goutte à goutte à 220 mg (0,43 mmol) de *N,N'*-(oxydiéthane-2,1-diyl)bis(4-amino-5-méthoxy-1,3-benzothinole-2-carboxamide) en solution dans 10 ml d'acétone. Le mélange réactionnel est maintenu sous agitation à température ambiante pendant 2 heures, puis l'acétone est évaporée et le milieu est repris par 25 ml de dichlorométhane et lavé par 2 fois 15 ml d'une solution aqueuse saturée en chlorure de sodium. Les phases organiques sont réunies, séchées sur sulfate de magnésium et le solvant évaporé sous pression réduite. 230 mg de produit attendu (rendement brut = 99 %) est obtenu sous forme d'une poudre jaune et est utilisé dans l'étape suivante sans autre purification.
SM-CL : MH+ = 547,12 ; t.r. = 8,76 min.

### 14.5) N,N'-(oxydiéthane-2,1-diyl)bis(5- {[2-(diméthylamino)éthyl]amino}-4,7-dioxo-4, 7-dihydro-1,3-benzothiazole-2-carboxamide) :

81 µl (0,73 mmol ; 2 éq.) de *N,N*-diméthyléthylènediamine sont ajoutés à 200 mg de *NN*'-(oxydiéthane-2,1-diyl)bis(5-métlloxy-4,7-dioxo-4,7-dihydro-1,3-benzothinole-2-carboxamide) en solution dans 300 ml d'éthanol anhydre. Le mélange réactionnel est agité à 60 °C pendant 4 heures puis le solvant est évaporé sous pression réduite. Le résidu est purifié sur colonne de silice (éluant : méthanol dans le dichlorométhane : gradient de 0 à 20 % sur 30 minutes puis pendant 15 minutes à 20 % de méthanol dans le dichlorométhane) et 12 mg (rendement = 5 %) de composé attendu sont obtenus sous forme d'une poudre rouge.
RMN ¹H (DMSO d6, 400 MHz, δ) : 9,04 (t, 2H, 2NH) ; 7,49 (t, 2H, 2NH) ; 5,56 (s, 2H, 2CH) ;3,64-3,58 (m, 4H, 2CH₂) ; 3,45-3,41 (m, 4H, 2CH₂) ; 3,26-3,22 (m, 4H, 2CH₂) ; 2,51-2,49 (m, 4H, 2CH₂) ; 2,19 (s, 12H, 4CH₃).
SM-CL : MH+ = 659,20 ; t.r. = 6,99 min.

*Le composé de l'exemple 15 est obtenu selon un protocole analogue à celui employé pour l'exemple 14, l'amine adéquate remplaçant la 2,2'-oxybis(éthylamine) dans la* deuxième *étape.*

### Exemple 15 : N,N'-[éthane-1,2-diylbis(oxyéthane-2,1-diyl)]-bis(5-{[2-(diméthylamino)éthyl]amino}-4,7-dioxo-4,7-dihydro-1,3-benzothiazole-2-carboxamide) :

Poudre rouge.
RMN ¹H (DMSO d6, 400 MHz, δ) : 9,07 (t, 2H, 2NH) ; 7,55 (t, 2H, 2NH) ; 5,59 (s, 2H, 2CH) ; 3,57-3,54 (m, 8H, 4CH₂) ; 3,43-3,37 (m, 4H, 2CH₂) ; 3,26-3,22 (m, 4H, 2CH₂) ; 2,50-2,49 (m, 4H, 2CH₂) ; 2,18 (m, 12H, 4CH₃). SM-CL : MH+ = 703,24 ; t.r. = 7,14 min.

*Les composés des exemples 16 à 20 sont obtenus selon un protocole analogue à celui employé pour l'exemple 1, les amines adéquates remplaçant la morpholine dans la deuxième étape et la N-(2-aminoéthyl)pyrrolidine remplaçant la N,N diméthyléthylènediamine dans la dernière étape pour l'exemple 17.*

### Exemple 16: 5-{[2-(diméthylamino)éthyl]amino}-2-{[4-(morpholin-4-ylcarbonyl)pipérazin-1-yl]carbonyl}-1,3-benzothiazole-4,7-dione :

Poudre rouge. Point de fusion : 211-212°C.
RMN ¹H (DMSO d6, 400 MHz, δ) : 7,57 (t, 1H, NH) ; 5,62 (s, 1H, CH) ; 4,24 (m, 2H) ; 3,69 (m, 2H) ; 3,59-3,56 (m, 4H ; 3,30 (m, 4H) ; 3,19-3,16 (m, 4H) ; 2,52 (m, 4H) ; 2,20 (s, 6H, 2CH₃).
SM-LC : MH+ = 477,20 ; t.r. = 7,23 min.

### Exemple 17 : 2-{[4-(morpholin-4-ylcarbonyl)pipérazin-1-yl]carbonyl}-5-[(2-pyrrolidin-1-yléthyl)amino]-1,3-benzothiazole-4,7-dione :

Poudre rouge. Point de fusion : 193-194°C.
RMN ¹H (DMSO d6, 400 MHz, δ) : 7,72 (t, 1H, NH) ; 5,64 (s, 1H, CH) ; 4,24 (m, 2H) ; 3,69 (m, 4H) ; 3,59-3,56 (m, 6H) ; 3,19-3,16 (m, 6H) ; 2,66-2,60 (m, 6H) ; 1,73 (m, 4H).
SM-LC : MH+ = 503,26 ; t.r. = 7,29 min.

### Exemple 18 : 5-{[2-(diméthylamino)éthyl]amino}-N-(4-méthozyphényl)-4,7-dioxo-4,7-dihydro-1,3-benzothiazole-2-carboxamide :

Poudre rouge. Point de fusion : 207-208°C.
RMN ¹H (DMSO d6, 400 MHz, δ) : 10,96 (s, 1H, NH) ; 7,78-7,75 (m, 2H, arom) ; 7,58 (t, 1H, NH) ; 6,96-6,93 (m, 2H, arom) ; 5,65 (s, 1H, CH) ; 3,75 (s, 3H, OCH₃) ; 3,27-3,26 (m, 2H) ; 2,53-2,50 (m, 2H) ; 2,20 (s, 6H, 2CH₃).
SM-LC : MH+ = 401,11; t.r: = 7,98 min

Ce composé est obtenu sous forme salifiée, (le sel préparé étant un méthanesulfonate) selon des méthodes classiques connues de l'homme de l'art.

Poudre orangée.
SM-LC : MH+ = 401,17 ; t.r. = 8,07 min.

### Exemple 19 : 5-{[2-(diméthylamino)éthyl]amino}-2-{[4-(méthylsulfonyl)pipérazin-1-yl] carbonyl)-1,3-benzothiazole-4,7-dione :

Poudre rouge. Point de fusion : 201-202°C.
RMN ¹H (DMSO d6, 400 MHz, δ) : 7,57 (t, 1H, NH) ; 5,62 (s, 1H, CH) ; 4,32 (m, 2H) ; 3,78 (m, 2H) ; 3,28-3,25 (m, 6H) ; 2,91 (s, 3H, CH₃) ; 2,53-2,50 (m, 2H) ; 2,18 (s, 6H, 2CH₃).
SM-LC : MH+ = 442,15 ; t.r. = 7,31 min.

### Exemple 20 : 5-{[2-(diméthylamino)éthyl]amino}-2-{[4-(tétrahydrofuran- . 2-ylcarbonyl)pipérazin-1-yl]carbonyl}-1,3-benzothiazole-4,7-dione :

Poudre rouge. Point de fusion : 168-169°C.
RMN ¹H (DMSO d6, 400 MHz, δ) : 7,57 (t, 1H, NH) ; 5,62 (s, 1H, CH) ; 4,73-4,69 (m, 1H) ; 4,25-4,21 (m, 2H) ; 3,79-3,72 (m, 2H) ; 3,68-3,57 (m, 6H) ; 3,28-3,25 (m, 2H) ; 2,51-2,49 (m, 2H) ; 2,19 (s, 6H, 2CH₃) ; 2,05-2,01 (m, 2H) ; 1,85-1,80 (m, 2H).
SM-LC : MH+ = 462,19 ; t.r. = 7,22 min.

### Exemple 21 : 6-bromo-5-{[2-(diméthylamino)éthyl]amino}-2-{[4-(tétrahydrofuran-2-ylcarbonyl)pipérazin-1-yl)carbonyl}-1,3-benzothiazole-4,7-dione :

78 mg (0,17 mmol) du composé de l'exemple 20 sont mis en solution dans 10 mL d'acide acétique. 33 mg (0,19 mmol ; 1,1 éq.) de *N*-bromosuccinimide sont ajoutés et le mélange réactionnel est agité pendant 3 heures à température ambiante. Après concentration sous pression réduite, le résidu est purifié par chromatographie sur colonne de silice (éluant : dichlorométhane/méthanol 90/10) et le produit attendu est obtenu, après reprise dans de l'éther éthylique, sous forme d'une poudre violette.

Point de fusion : 161-162° C.
RMN ¹H (DMSO d6, 400 MHz, δ): 7,47 (t, 1H,NH); 4,73-4,69 (m, 1H) 4,25-4,21 (m, 2H) ; 3,79-3,72 (m, 2H) ; 3,68-3,57 (m, 6H) ; 3,28-3,25 (m, 2H) ; 2,51-2,49 (m, 2H) ; 2,19 (s, 6H, 2CH₃) ; 2,05-2,01 (m, 2H) ; 1,85-1,80 (m, 2H).
SM-LC : MH+ = 540,09 ; t.r. = 7,39 min.

*Les composés des exemples 22 à 26 sont obtenus selon un protocole analogue à celui employé pour l'exemple 1, les amines adéquates remplaçant la morpholine dans la deuxième étape.*

### Exemple 22 : 5-{[2-(diméthylamino)éthyl]amino}-2-(pyrrolidin-1-ylcarbonyl)-1,3-benzothiazole-4,7-dione :

Poudre rouge. Point de fusion : 207-208°C.
RMN ¹H (DMSO d6, 400 MHz, δ) : 7,54 (t, 1H, NH) ; 5,61 (s, 1H, CH) ; 4,01 (t, 2H, CH₂) ; 3,55 (t, 2H, CH₂) ; 3,29-3,25 (m, 2H) ; 2,52-2,49 (m, 2H) ; 2,19 (s, 6H, 2CH₃) ; 1,98-1,93 (m, 2H) ; 1,89-1,84 (m, 2H).
SM-LC : MH+ = 349,16 ; t.r. = 7,45 min.

### Exemple 23 : 5-{[2-(diméthylamino)éthyl]amino}-2-{[4-(2-thiénylcarbonyl)pipérazin-1-yl]carbonyl}-1,3-benzothiazole-4,7-dione :

Poudre rouge.
RMN ¹H (DMSO d6, 400 MHz, δ) : 7,79-7,78 (m, 1H) ; 7,57 (t, 1H, NH) ; 7,50-7,49 (m, 1H) ; 7,16-7,13 (m, 1H) ; 5,62 (s, 1H, CH) ; 4,33 (m, 2H) ; 3,79 (m, 6H) ; 3,28-3,25 (m, 2H) ; 2,50-2,48 (m, 2H) ; 2,19 (s, 6H, 2CH₃).
SM-LC : MH+ = 474,15 ; t.r. = 7,55 min.

### Exemple 24 : 5-{[2-(diméthylamino)éthyl]amino}-N-éthyl-4,7-dioxo-4,7-dihydro-1,3-benzothiazole-2-carboxamide :

Poudre rouge. Point de fusion : 174-175°C.
RMN ¹H (DMSO d6, 400 MHz, δ) : 9,21 (t, 1H, NH) ; 7,54 (t, 1H, NH) ; 5,61 (s, 1H, CH) ; 3,27-3,24 (m, 4H) ; 2,52-2,49 (m, 2H) ; 2,19 (s, 6H, 2CH₃) ; 1,13 (t, 3H, CH₃).
SM-LC : MH+ = 323,12 ; t.r. = 7,29 min.

### Exemple 25 : N-(4-chlorophényl)-5-([2-(diméthylamino)éthyl] amino)-4,7-dioxo-4,7-dihydro1,3-benzothiazole-2-carboxamide :

Poudre rouge.
RMN ¹H (DMSO d6, 400 MHz, δ) : 11,22 (s, 1H, NH) ; 7,95-7,85 (m, 3H) ; 7,46-7,39 (m, 2H) ; 5,79 (s, 1H, CH) ; 3,50 (m, 2H) ; 2,66 (m, 2H) ; 2,19 (s, 6H, 2CH₃).
SM-LC : MH+ = 405,03 ; t.r. = 8,35 min.

### Exemple 26 : N-1,3-benzodioxol-5-yl-5-([2-(diméthylamino)éthyl]amino)-4,7-dioxo-4,7-dihydro-1,3-benzothiazole-2-carboxamide :

Poudre rouge. Point de fusion : 220-22 1 °C.
RMN ¹H (DMSO d6, 400 MHz, δ) : 10,98 (s, 1H, NH) ; 7,58 (t, 1H, NH) ; 7,47 (s, 1H) ; 7,37-7,34 (m, 1H) ; 6,93-6,91 (m, 1H) ; 6,02 (s, 2H, CH₂) ; 5,65 (s, 1H, CH) ; 3,27-3,25 (m, 2H) ; 2,53-2,51 (m, 2H) ; 2,19 (s, 6H, 2CH₃).
SM-LC : MH+ = 415,07 ; t.r. = 8,00 min.

*Les composés des exemples 27 à 32 sont obtenus selon un protocole analogue à celui employé pour l'exemple 20, les amines adéquates remplaçant la N,N diméthyléthylènediamine dans la dernière étape.*

### Exemple 27 : 5-[(2-méthoxyéthyl)amino]-2-{[4-(tétrahydrofuran-2-ylcarbonyl)pipérazin-1-yl]carbonyl}-1,3-benzothiazole-4,7-dione :

Poudre rouge.
SM-LC : MH+ = 449,16 ; t.r. = 8,16 min.

### Exemple 28 : 5-(4-méthylpipérazin-1-yl)-2-{[4-(tétrahydrofuran-2-ylcarbonyl)pipérazin-1-yl]carbonyl}-1,3-benzothiazole-4,7-dione :

Poudre rouge.
SM-LC : MH+ = 474,20 ; t.r. = 7,31 min.

### Exemple 29 : 5-[(2-pyridin-2-yléthyl)amino]-2-{[4-(tétrahydrofuran-2-ylcarbonyl)pipérazin-1-yl]carbonyl}-1,3-benzothiazole-4,7-dione :

Poudre rouge.
SM-LC : MH+ = 496,20 ; t.r. = 7,42 min.

### Exemple 30 : 5-[(3-morpholin-4-ylpropyl)amino]-2-{[4-(tétrahydrofuran-2-ylcarbonyl)pipérazin-1-yl]carbonyl}-1,3-benzothiazole-4,7-dione :

Poudre rouge.
SM-LC : MH+ = 518,22 ; t.r. = 7,34 min.

### Exemnle 31 : 5-[(1-benzylpyrrolidin-3-yl)amino]-2-([4-(tétrahydrofuran-2-ylcarbonyl)pipérazin-1-yl]carbonyl}-1,3-benzothiazole-4,7-dione :

Poudre rouge.
SM-LC : MH+ = 550,22 ; t.r. = 7,70 min.

### Exemple 32 : 5-[(2-cyclohexyléthyl)amino]-2-{[4-(tétrahydrofuran-2-ylcarbonyl)pipérazin-1-yl]carbonyl)-1,3-benzothiazole-4,7-dione :

Poudre rouge.
SM-LC : MH+ = 501,21 ; t.r. =10,83 min.

*Le composé de l'exemple 33 est obtenu selon un protocole analogue à celui employé pour l'exemple 1, la 4-fluoroaniline remplaçant la morpholine dans la deuxième étape.*

### Exemple 33 : 5-([2-(diméthylamino)éthyl]amino)-N-(4-fluorophényl)-4,7-dioxo-4,7-dihydro-1,3-benzothiazole-2-carboxamide :

Poudre rouge. Point de fusion : 208-209° C.
RMN ¹H (DMSO d6, 400 MHz, δ) : 11,15 (s, 1H, NH) ; 7,90-7,85 (m, 2H) ; 7,60 (t, 1H, NH) ; 7,26-7,20 (m, 1H) ; 5,66 (s, 1H, CH) ; 3,27-3,25 (m, 2H) ; 2,53-2,51 (m, 2H) ; 2,19-(s, 6H, 2CH₃).
SM-LC : MH+ = 389,14 ; t.r. = 8,11 min.

*Le composé de l'exemple 34 est obtenu selon un protocole analogue à celui employé pour l'exemple 1, le 6-méthoxy-2-méthyl-7-nitro-1,3-benzothiazole remplaçant le 5-méthoxy-2méthyl 4-nitro-1,3-benzothiazole dans la première étape et la pyrrolidine remplaçant la morpholine dans la deuxième étape.*

### Exemple 34 : 6-([2-(diméthylamino)éthyl]amino)-2-pyrrolidin-1-ylcarbonyl)-1,3-benzothiazole-4,7-dione :

Poudre rouge. Point de fusion : 224-225° C.
RMN ¹H (DMSO d6, 400 MHz, δ) : 7,32 (t, 1H, NH) ; 5,55 (s, 1H, CH) ; 4,01 (t, 2H, CH₂) ; 3,55 (t, 2H, CH₂) ; 3,27-3,22 (m, 2H); 2,49-2,47 (m, 2H) ; 2,18 (s, 6H, 2CH₃) ; 1,98-1,93 (m, 2H) ; 1,89-1,84 (m, 2H) .
SM-LC : MH+ = 349,16 ; t.r. = 7,35 min.

*Les composés des exemples 35 à 37 sont obtenus selon un protocole analogue à celui employé pour l'exemple 1, l'amine adéquate remplaçant la morpholine dans la deuxième étape.*

### Exemple 35 : 5-([2-(diméthylamino)éthyl]amino)-N-(3-fluoro-4-méthoxyphényl)-4,7-dioxo-4,7-dihydro-1,3-benzothiazole-2-carboxamide :

Poudre rouge. Point de fusion : 199-200° C.
RMN ¹H (DMSO d6, 400 MHz, δ) : 11,11 (s, 1H, NH) ; 7,80-7,76 (m, 1H) ; 7,68-7,66 (m, 1H) ; 7,60 (t, 1H, NH) ; 7,21-7,16 (m, 1H) ; 5,65 (s, 1H, CH) ; 3,83 (s, 3H, CH₃) ; 3,28-3,26 (m, 2H); 2,53-2,52 (m, 2H) ; 2,20 (s, 6H, 2CH₃).
SM-LC : MH+ = 419,15 ; t.r. = 8,11 min.

### Exemple 36 : 5-([2-(diméthylamino)éthyl]amino)-2-[(6-méthoxy-3,4-dihydroquinolin-1(2H)-yl)carbonyll-1,3-benzothiazole-4,7-dione :

Poudre rouge. Point de fusion : 192-193 °C.
RMN.¹H (DMSO d6, 400 MHz, δ): 7,54 _(m, 2H) ; 6,80 (m,1H); 6,70 (m, 1H); 5,60 (s, 1H, CH) ; 4,05-4,15 (m, 2H) ; 3,73 (s, 3H, CH₃) ; 3,27-3,24 (m, 2H); 2,82-2,79 (m, 2H) ; 2,51-2,49 (m, 2H) ; 2,18 (s, 6H, 2CH₃) ; 1,98-1,96 (m, 2H).
SM-LC : MH+ = 441,22 ; t.r. = 8,01 min.

### Exemple 37 : 5-{[2-(diméthylamino)éthyl]amino)-2-{[4-(4-méthoxybenzoyl) pipérazin-1-yl]carbonyl}-1,3-benzothiazole-4,7-dione :

Poudre rouge. Point de fusion : 209-210 °C.
RMN ¹H (DMSO d6, 400 MHz, δ) : 7,60 (t, 1H, NH) ; 7,44-7,42 (m, 2H) ; 7,01-6,99 (m, 2H) ; 5,61 (s, 1H, CH) ; 4,29-4,31 (m, 2H) ; 3,80 (s, 3H, CH₃) ; 3,73-3,71 (m, 2H); 3,63-3,61 (m, 4H) ; 3,28-3,26 (m, 2H) ; 2,50-2,48 (m, 2H) ; 2,19 (s, 6H, 2CH₃).
SM-LC : MH+ = 498,30 ; t.r. = 7,73 min.

*Les composés des exemples 38 à 39 sont obtenus selon un protocole analogue à celui employé pour l'exemple 24, les amines adéquates remplaçant la, N,N-diméthyléthylènediamine dans la dernière étape.*

### Exemple 38 : 5-[[2-(diméthylamino)éthyl](méthyl)amino]-N-éthyl-4,7-dioxo-4,7-dihydro-1,3-benzothiazole-2-carboxamide :

Poudre rouge. Point de fusion : 163-164 °C.
RMN ¹H (DMSO d6, 400 MHz, δ) : 9,10 (t, 1H, NH) ; 5,65 (s, 1H, CH) ; 3,77-3,74 (m, 2H) ; 3,34-3,32 (m, 2H) ; 2,98 (s, 3H, CH₃) ; 2,35-2,33 (m, 2H); 1,93 (s, 6H, 2CH₃) ; 1,13 (t, 3H, CH₃).
SM-LC : MH+ = 337,19 ; t.r. = 7,36 min.

### Exemple 39 : N-éthyl-5-{[2-(4-fluorophényl)éthyl]amino}-4,7-dioxo-4,7-dihydro-1,3-benzothiazole-2-carboxamide :

Poudre rouge. Point de fusion : 260-261 °C.
RMN ¹H (DMSO d6, 400 MHz, δ): 9,20 (t, 1H, NH) ; 7,96 (t, 1H, NH) ; 7,33-7,29 (m, 2H) ; 7,13-7,09 (m, 2H) ; 5,66 (s, 1H, CH) ; 3,44-3,42 (m, 2H) ; 3,30-3,29 (m, 2H) ; 2,89 (t, 2H, CH₂) ; 1,13 (t, 3H, CH₃).
SM-LC : MH+ = 374,13 ; t.r. = 10,27 min.
et les sels de ces composés.

### Etude pharmacologique des composés de l'invention

### Protocoles des tests

### i) Mesure de l'activité phosphatase de l'enzyme recombinante Cdc25C purifiée

L'activité phosphatase de la protéine MBP-Cdc25C est évaluée par la déphosphorylation du 3-O-méthylfluorescéine-phosphate (OMFP) en 3-O-méthylfluorescéine (OMF) avec une détermination de la fluorescence à 475 nm du produit de la réaction. Cet essai permet d'identifier des inhibiteurs de l'enzyme recombinante cdc25. La préparation de la protéine de fusion MBP-Cdc25C est décrite dans la demande de brevet PCT WO 01/44467.

La réaction est réalisée en format de plaque 384 puits sous un volume final de 50 µl. La protéine MBP-Cdc25C (préparée comme décrit ci-dessus) est conservée dans le tampon d'élution suivant : 20 mM Tris-HCl pH 7,4 ; 250 mM NaCl ; 1mM EDTA ; 1 mM de dithiothréitol (DTT) ; 10 mM maltose. Elle est diluée à à concentration de 60 µM dans le tampon de réaction suivant : 50 mM Tris-HCl pH 8,2 ; 50 mM NaCl ; 1 mM DTT ; 20% glycérol. La mesure du bruit de fond est effectuée avec le tampon sans addition de l'enzyme. Les produits sont testés à des concentrations décroissantes à partir de 40 µM. La réaction est initiée par l'ajout d'une solution OMFP à 500 µM finale (préparée extemporanément à partir d'une solution stock 12,5 mM dans du DMSO 100 % (Sigma #M2629)) . Après 4 heures à 30 °C dans une plaque 384 puits à usage unique, la fluorescence mesurée à DO 475 nm est lue à l'aide d'un lecteur de plaque Victor² (EGG-Wallac). La détermination de la concentration inhibant de 50 % la réaction enzymatique est calculée à partir de trois expériences indépendantes. Seules les valeurs contenues dans la partie linéaire de la sigmoïde sont retenues pour l'analyse de régression linéaire.

### ii) Caractérisation de l'activité anti-proliférative :

A titre d'exemple, on étudiera l'effet d'un traitement sur deux lignées de cellules humaines Mia-Paca2 et DU145 par les composés des exemples décrits précédemment. Les lignées cellulaires DU145 (cellules humaines de cancer de la prostate) et Mia-PaCa2 (cellules humaines de cancer du pancréas) ont été acquises auprès de American Tissue Culture Collection (Rockville, Maryland, USA). Les cellules placées dans 80 µl de milieu Eagle modifié de Dulbecco (Gibco-Brl, Cergy-Pontoise, France) complété avec 10 % de sérum foetal de veau inactivé par chauffage (Gibco-Brl, Cergy-Pontoise, France), 50000 unités/l de pénicilline et 50 mg/l streptomycine (Gibco-Brl, Cergy-Pontoise, France), et 2 mM de glutamine (Gibco-Brl, Cergy-Pontoise, France) ont été ensemencées sur une plaque de 96 puits au jour 0. Les cellules ont été traitées au jour 1 pendant 96 heures avec des concentrations croissantes de chacun des composés à tester jusqu'à 10 µM. A la fin de cette période, la quantification de la prolifération cellulaire est évaluée par test colorimétrique en se basant sur le clivage du sel de tétrazolium WST1 par les déhydrogénases mitochondriales dans les cellules viables conduisant à la formation de formazan (Boehringer Mannheim, Meylan, France). Ces tests sont effectués en double avec 8 déterminations par concentration testée. Pour chaque composé à tester, les valeurs incluses dans la partie linéaire de la sigmoïde ont été retenues pour une analyse en régression linéaire et utilisées pour estimer la concentration inhibitrice CI₅ₒ. Les produits sont solubilisés dans le diméthylsulfoxide (DMSO) à 10⁻² M et utilisés en culture avec 0,1 % DMSO en final.

### Résultats des tests

a) Les composés des exemples 1 à 39 présentent une CI₅₀ inférieure ou égale à 1,1 µM sur l'activité phosphatase de l'enzyme recombinante Cdc25-C purifiée.
b) Les composés des exemples 1 à 39 présentent une CI₅₀ inférieure ou égale à 10 µM, plus particulièrement inférieure ou égale à 4 µM sur la prolifération cellulaire des lignées Mia-Paca2.
c) Les composés des exemples 1 à 39 présentent une CI₅₀ inférieure ou égale à 10 µM, plus particulièrement inférieure ou égale à 6 µM sur la prolifération cellulaire des lignées DU-145.

## Revendications

1. Composé répondant à la formule générale (I) 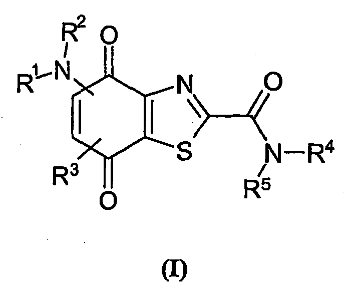 sous forme racémique, d'énantiomère ou toute combinaison de ces formes; dans laquelle :
R¹ représente un atome d'hydrogène ou un radical alkyle, alkoxyalkyle, alkylthioalkyle; cycloalkyle, -(CH₂)-X-Y, -(CH₂)-Z-NR⁶R⁷ ou un radical -CHR⁸R⁹,
X représentant une liaison ou un radical alkylène linéaire ou ramifié comptant de 1 à 5 atomes de carbone,
Y représentant un système cyclique carboné saturé comptant de 1 à 3 cycles condensés choisis indépendamment parmi des cycles de 3 à 7 chaînons, ou bien Y représentant un hétérocycle saturé comptant de 1 à 2 hétéroatomes choisis indépendamment parmi O, N et S et attaché au radical X par un chaînon N ou CH, ledit hétérocycle saturé comptant par ailleurs de 2 à 6 chaînons supplémentaires choisis indépendamment parmi -CHR¹⁰-, -CO-, -NR¹¹-, -O- et -S-, R¹⁰ représentant un atome d'hydrogène ou un radical alkyle et R¹¹ représentant un atome d'hydrogène ou un radical alkyle ou aralkyle, ou encore Y représentant un radical aryle carbocyclique ou hétérocyclique éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR¹² et un radical NR¹³R¹⁴, R¹² représentant un atome d'hydrogène ou un radical alkyle ou phényle, et R¹³ et R¹⁴ représentant indépendamment des radicaux alkyle,
Z représentant une liaison ou un radical alkylène linéaire ou ramifié comptant de 1 à 5 atomes de carbone,
R⁶ et R⁷ étant choisis indépendamment parmi un atome d'hydrogène, un radical alkyle, aralkyle ou -(CH₂)ₙ-OH dans lequel n représente un entier de 1 à 6,
ou R⁶ représentant un radical alkoxycarbonyle, haloalkoxycarbonyle ou aralkoxycarbonyle et R⁷ représentant un atome d'hydrogène ou un radical méthyle,
ou encore R⁶ et R⁷ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR¹⁵R¹⁶-, -O-, -S- et -NR¹⁷-, R¹⁵ et R¹⁶ représentant indépendamment à chaque fois qu'ils interviennent un atome d'hydrogène ou un radical alkyle, et R¹⁷ représentant un atome d'hydrogène ou un radical alkyle ou aralkyle, ou encore R¹⁷ représentant un radical phényle éventuellement substitué de 1 à 3 fois par des substituants choisis indépendamment parmi un atome halogène et un radical alkyle ou alkoxy,
R⁸ et R⁹ formant ensemble avec l'atome de carbone qui les porte un radical indanyle ou tétralinyle, ou encore R⁸ et R⁹ formant ensemble avec l'atome de carbone qui les porte un hétérocycle saturé comptant de 5 à 7 chaînons et de 1 à 2 hétéroatomes choisis parmi O, N et S, les atomes d'azote dudit hétérocycle étant éventuellement substitués par des radicaux choisis parmi les radicaux alkyle et le radical benzyle ;
R² représentant un atome d'hydrogène ou un radical alkyle ou aralkyle ;
ou encore R¹ et R² formant ensemble avec l'atome d'azote un hétérocycle de 4 à 8 chaînons comportant de 1 à 2 hétéroatomes, les chaînons nécessaires pour compléter l'hétérocycle étant choisis indépendamment parmi les radicaux -CR¹⁸R¹⁹-, -O-, -S- et -NR²⁰-, R¹⁸ et R¹⁹ représentant indépendamment à chaque fois qu'ils interviennent un atome d'hydrogène ou un radical alkyle, et R²⁰ représentant un atome d'hydrogène ou un radical alkyle ou aralkyle ;
R³ représente un atome d'hydrogène ou un atome halogène ;
R⁴ représente un radical alkyle, un radical haloalkyle, un radical cycloalkyle, un radical cycloalkylalkyle, un radical alkoxyalkyle, l'un des radicaux aryle carbocyclique ou hétérocyclique ou aralkyle carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂,
ou enclore R⁴ représente l'un des radicaux -(CH₂)ₘ-[O-(CH₂)p]q-O-Alk, -(CH₂)ᵣ-[O-(CH₂)ₛ]ₜ-NR²¹R²² ou -(CH₂)ᵥ-A dans lesquels dans lesquels m, p et s sont chacun indépendamment un entier de 2 à 4, q est un entier de 1 à 4, t est un entier de 0 à 4, r est un entier de 2 à 12 et v est un entier de 1 à 12, Alk est un radical alkyle, R²¹ est un atome d'hydrogène ou un radical alkyle, alkoxycarbonyle ou aralkoxycarbonyle, R²² est un atome d'hydrogène ou un radical alkyle et A est un hétérocycle saturé comptant de 1 à 2 hétéroatomes choisis indépendamment parmi O, N et S et attaché au groupe -(CH₂)ᵥ- par un chaînon N ou CH, ledit hétérocycle saturé comptant par ailleurs de 2 à 6 chaînons supplémentaires choisis indépendamment parmi -CHR²³-, -CO-, -NR²⁴-, -O- et -S-, R²³ représentant un atome d'hydrogène ou un radical alkyle et R²⁴ représentant un atome d'hydrogène, un radical alkyle ou un groupement alkoxycarbonyle ou aralkoxycarbonyle,
ou encore R⁴ représente un radical de formule 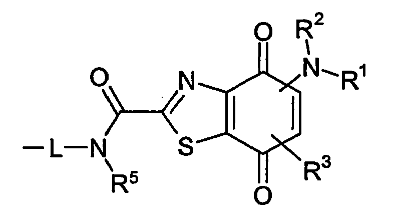 dans laquelle R¹, R², R³ et R⁵ sont identiques aux radicaux R¹, R², R³ mentionnés précédemment et R⁵ mentionnés ci-après, et L est choisi parmi les radicaux -(CH₂)g-[O-(CH₂)_{w}]ₓ-[O-(CH₂)_{y}]_{z}- et -(CH2)ₐ-Ω-(CH₂)_{b}- dans lesquels g, w et y sont des entiers de 2 à 4, x est un entier de 1 à 3 et z est 0 ou 1, a et b sont indépendamment des entiers de 2 à 6 et Ω est choisi parmi le groupe constitué par -O-, -S-, -NR²⁵-, -CO-, -CO-NR²⁶-,-CR²⁷R²⁸-, un radical cycloalkylène comptant de 3 à 7 atomes de carbone et enfin un radical aryle carbocyclique, R²⁵ représentant un radical alkyle, R²⁶ représentant un atome d'hydrogène ou un radical méthyle, R²⁷ et R²⁸ étant chacun choisis indépendamment parmi un atome d'hydrogène et un groupe méthyle ;
ou bien encore R⁴ représente un radical de formule 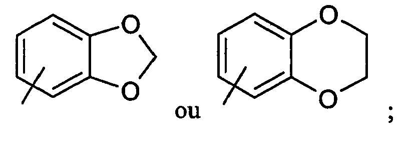
R⁵ représente un atome d'hydrogène ou un radical alkyle ou aralkyle, R⁵ pouvant également représenter un radical identique à R⁴ lorsque R⁴ représente un radical alkyle, haloalkyl, alkoxyalkyle ou aralkyle carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂ ;
ou encore R⁴ et R⁵ forment ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CR²⁹R³⁰-, -O-, -S- et -NR³¹-, R²⁹ et R³⁰ représentant un atome d'hydrogène ou un radical alkyle ou aralkyle et R³¹ représentant -COR³² ou -SO₂R³³,
R³² représentant un radical alkyle, un radical aryle carbocyclique éventuellement substitué de 1 à 3 fois par des substituants choisis indépendamment parmi le groupes constitué par un atome halogène, un radical alkyle et un radical alkoxy, ou encore R³² représentant un radical aryle hétérocyclique ou un hétérocycle saturé comptant de 5 à 7 chaînons et de 1 à 2 hétéroatomes choisis indépendamment parmi O, N et S,
R³³ représentant un atome d'hydrogène ou un radical alkyle,
ou enfin R⁴ et R⁵ forment ensemble avec l'atome d'azote qui les porte un radical aryle hétérocyclique choisi parmi les radicaux 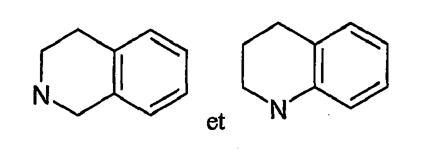 dont le noyau aromatique peut être substitué de 1 à 3 fois par des substituants choisis indépendamment parmi le groupe-constitué-par un radical alkyle et un radical alkoxy ;
ou sel d'un tel composé.

2. Composé selon la revendication 1, **caractérisé en ce que** R⁴ ne représente pas un radical de formule 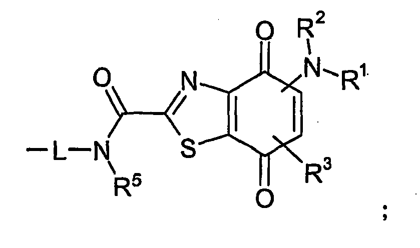 ou sel d'un tel composé.

3. Composé selon la revendication 1, **caractérisé en ce que** R⁴ représente un radical de formule 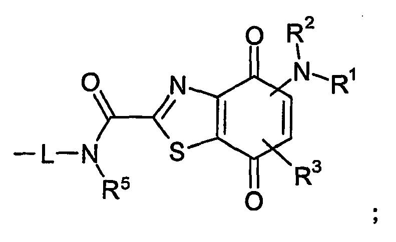 ou sel d'un tel composé.

4. Composé selon la revendication 1, **caractérisé en ce qu'**il est choisi parmi les composés suivants :
- 5-{[2-(diméthylamino)éthyl]amino}-2-(morpholin-4-ylcarbonyl)-1,3-benzothiazole-4,7-dione ;
- {2-[2-(2-{[(5-{[2-(diméthylamino)éthyl]amino}-4,7-dioxo-4,7-dihydro-1,3-benzothiazol-2-yl)carbonyl]amino}éthôxy)éthoxy]éthyl}carbamate de *tert*-butyle ;
- *N*,*N*-dibenzyl-5-{[2-(diméthylamino)éthyl]amino}-4,7-dioxo-4,7-dihydro-1,3-benzothiazole-2-carboxamide ;
- 5-{[2-(diméthylamino)éthyl]amino} -*N,N*-bis(2-méthoxyéthyl)-4,7-dioxo-4,7-dihydro-1,3-benzothiazole-2-carboxamide ;
- 5-{[2-(diméthylainino)éthyl]amino}-4,7-dioxo-*N*-[3-(2-oxopyrrolidin-1-yl)propyl]-4,7-dihydro-1,3-benzathiazole-2-carboxamide;
- 4,7-dioxo-*N*-[3-(2-oxopyrrolidin-1-yl)propyl]-5-[(2-pyrrolidin-1-yléthyl)amino]-4,7-dihydro-1,3-benzothiazole-2-carboxamide ;
- 5-{[2-(diméthylamino)éthyl]amino}-N isobutyl-4,7-dioxo-4,7-dihydro-1,3-benzothiazole-2-carboxamide ;
- (4-{[(5-{[2-(diméthylamino)éthyl]amino}-4,7-dioxo-4,7-dihydro-1,3-benzothiazol-2-yl)carbonyl]amino}butyl)carbamate de *tert*-butyle ;
- 5-{[2-(diméthylamino)éthyl]amino}-2-{[4-(2-furoyl)pipérazin-1-yl]carbonyl}-1,3-benzothiazole-4,7-dione ;
- 5-{[2-(diméthylamino)éthyl]amino}-*N*-(2-méthoxyéthyl)-4,7-dioxo-4,7-dihydro-1,3-benzothiazole-2-carboxamide ;
- *N-*butyl-5-{[2-(diméthylamino)éthyl]amino}-4,7-dioxo-4,7-dihydro-1,3-benzothiazole-2-carboxamide ;
- *N*-benzyl-5-{[2-(diméthylamino)éthyl]amino}-4,7-dioxo-4,7-dihydro-1,3-benzothiazole-2-carboxamide ;
- *N-*(cyclohexylméthyl)-5-{[2-(diméthylamino)éthyl]amino}-4,7-dioxo-4,7-dihydro-1,3-benzothiazole-2-carboxamide ;
- *N,N'*-(oxydiéthane-2,1-diyl)bis(5-{[2-(diméthylamino)éthyl]amino}-4,7-dioxo-4,7-dihydro-1,3-benzothiazole-2-carboxamide) ;
- *N,N'*-[éthane-1,2-diylbis(oxyéthane-2,1-diyl)]bis(5-{[2-(diméthylamino)éthyl]amino}-4,7-dioxo-4,7-dihydro-1,3-benzothiazole-2-carboxamide) ;
- 5-{[2-(diméthylamino)éthyl]amino}-2-{[4-(morpholin-4-ylcarbonyl)pipérazin-1-yl]carbonyl}-1,3-benzothiazole-4,7-dione ;
- 2-{[4-(morpholin-4-ylcarbonyl)pipérazin-1-yl]carbonyl}-5-[(2-pyrrolidin-1-yléthyl)amino]-1,3-benzothiazole-4,7-dione;
- 5-{[2-(diméthylamino)éthyl]alnino}-*N*-(4-méthoxyphényl)-4,7-dioxo-4,7-dihydro-1,3-benzothiazole-2-carboxamide ;
- 5-{[2-(diméthylamino)éthyl]amino}-2-{[4-(méthylsulfonyl)pipérazin-1-yl]carbonyl}-1,3-benzothiazole-4,7-dione ;
- 5-{[2-(diméthylamino)éthyl]amino}-2-{[4-(tétrahydrofuran-2-ylcarbonyl)pipérazin-1--yl]carbonyl}-1,3-benzothiazole-4,7-dione-;
- 6-bromo-5-{[2-(diméthylamino)éthyl]amino}-2-{[4-(tétrahydrofuran-2-ylcarbonyl)pipérazin-1-yl]carbonyl}-1,3-benzothiazole-4,7-dione ;
- 5-{[2-(diméthylainino)éthyl]amino)-2-(pyrrolidin-1-ylcarbonyl)-1,3-benzothiazole-4,7-dione ;
- 5-{[2-(diméthylamino)éthyl]amino}-2-{[4-(2-thiénylcarbonyl)pipérazin-1-yl]carbonyl}-1,3-benzothiazole-4,7-dione ;
- 5-{[2-(diméthylamino)éthyl]amino}-*N*-éthyl-4,7-dioxo-4,7-dihydro-1,3-benzothiazole-2-carboxamide ;
- *N*-(4-chlorophényl)-5-{[2-(diméthylamino)éthyl]amino}-4,7-dioxo-4,7-dihydro-1,3-benzothiazole-2-carboxamide ;
- *N*-1,3-benzodioxol-5-yl-5-{[2-(diméthylamino)éthyl]amino}-4,7-dioxo-4,7-dihydro-1,3-benzothiazole-2-carboxamide ;
- 5-[(2-méthoxyéthyl)amino]-2-{[4-(tétrahydrofuran-2-ylcarbonyl)pipérazin-1-yl]carbonyl}-1,3-benzothiazole-4,7-dione ;
- 5-(4-méthylpipérazin-1-yl)-2-{[4-(tétrahydrofuran-2-ylcarbonyl)pipérazin-1-yl]carbonyl}-1,3-benzothiazole-4,7-dione ;
- 5-[(2-pyridin-2-yléthyl)amino]-2-{[4-(tétrahydrofuran-2-ylcarbonyl)pipérazin-1-yl]carbonyl}-1,3-benzothiazole-4,7-dione;
- 5-[(3-morpholin-4-ylpropyl)amino]-2-{[4-(tétrahydrofuran-2-ylcarbonyl)pipérazin-1-yl]carbonyl}-1,3-benzothiazole-4,7-dione ;
- 5-[(1-benzylpyrrolidin-3-yl)amino]-2-{[4-(tétrahydrofuran-2-ylcarbonyl)pipérazin-1-yl]carbonyl}-1,3-benzothiazole-4,7-dione ;
- 5-[(2-cyclohexyléthyl)amino]-2-{[4-(tétrahydrofuran-2-ylcarbonyl)pipérazin-1-yl]carbonyl}-1,3-benzothiazole-4,7-dione ;
- 5-{[2-(diméthylamino)éthyl]amino}-*N*-(4-fluorophényl)-4,7-dioxo-4,7-dihydro-1,3-benzothiazole-2-carboxamide ;
- 6-{[2-(diméthylamino)éthyl]amino}-2-(pyrrolidin-1-ylcarbonyl)-1,3-benzothiazole-4,7-dione ;
- 5-{[2-(diméthylamino)éthyl]amino}-*N*-(3-fluoro-4-méthoxyphényl)-4,7-dioxo-4,7-dihydro-1,3-benzothiazole-2-carboxamide ;
- 5-{[2-(diméthylamino)éthyl]amino}-2-[(6-méthoxy-3,4-dihydroquinolin-1(2*H*)-yl)carbonyl]-1,3-benzothiazole-4,7-dione ;
- 5-{[2-(diméthylamino)éthyl]amino}-2-{[4-(4-méthoxybenzoyl)pipérazin-1--yl]carbonyl}-1,3-benzothiazole-4,7-dione;
- 5-[[2-(diméthylamino)éthyl](méthyl)amino]-*N*-éthyl-4,7-dioxo-4,7-dihydro-1,3-benzothiazole-2-carboxamide ;
- *N-*éthyl-5-{[2-(4-fluorophényl)éthyl]amino}-4,7-dioxo-4,7-dihydro-1,3-benzothiazole-2-carboxamide ;
ou sel d'un tel composé.

5. Composé de formule générale (**A**) 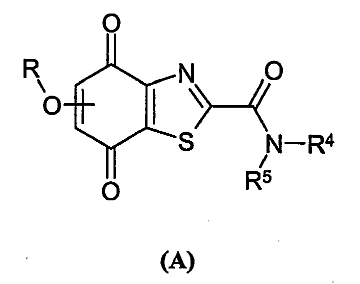 dans laquelle :
R est un radical alkyle ;
R⁵ a la même signification que dans la formule générale (**I**) de la revendication 1 ;
et R⁴ représente un radical alkyle, un radical haloalkyle, un radical cycloalkyle, un radical cycloalkylalkyle, un radical alkoxyalkyle, l'un des radicaux aryle carbocyclique ou hétérocyclique ou aralkyle carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂
ou encore R⁴ représente l'un des radicaux -(CH₂)ₘ-[O-(CH₂)ₚ]_{q}-O-Alk, -(CH₂)ᵣ-[O-(CH2)ₛ]ₜ-NR²¹R²² ou -(CH₂)ᵥ-A dans lesquels m, p et s sont chacun indépendamment un entier de 2 à 4, q est un entier de 1 à 4, t est un entier de 0 à 4, r est un entier de 2 à 12 et v est un entier de 1 à 12, Alk est un radical alkyle, R²¹ est un atome d'hydrogène ou un radical alkyle, alkoxycarbonyle ou aralkoxycarbonyle, R²² est un atome d'hydrogène ou un radical alkyle et A est un hétérocycle saturé comptant de 1 à 2 hétéroatomes choisis indépendamment parmi O, N et S et attaché au groupe -(CH₂)ᵥ- par un chaînon N ou CH, ledit hétérocycle saturé comptant par ailleurs de 2 à 6 chaînons supplémentaires choisis indépendammment parmi -CHR²³-, -CO-, NR²⁴-, -O- et -S-, R²³ représentant un atome d'hydrogène ou un radical alkyle et R²⁴ représentant un atome d'hydrogène, un radical alkyle ou un groupement alkoxycarbonyle ou aralkoxycarbonyle,
ou encore R⁴ représente un radical de formule 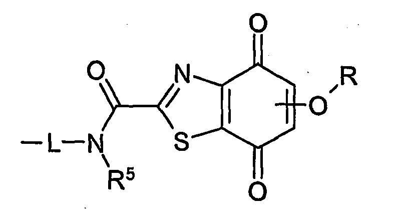 dans laquelle R et R⁵ sont identiques aux radicaux R et R⁵ mentionnés précédemment et L est choisi parmi les radicaux -(CH₂)_{g}-[O-(CH₂)_{w}]ₓ-[O-(CH₂)_{y}]_{z}- et -(CH₂)ₐ-Ω-(CH₂)_{b}- dans lesquels g, w et y sont des entiers de 2 à 4, x est un entier de 1 à 3 et z est 0 ou 1, a et b sont indépendamment des entiers de 2 à 6 et Ω est choisi parmi le groupe constitué par -O-, -S-, -NR²⁵-, -CO-, -CO-NR²⁶-, -CR²⁷R²⁸-, un radical cycloalkylène comptant de 3 à 7 atomes de carbone et enfin un radical aryle carbocyclique, R²⁵ représentant un radical alkyle, R²⁶ représentant un atome d'hydrogène ou un radical méthyle, R²⁷ et R²⁸ étant chacun choisis indépendamment parmi un atome d'hydrogène et un groupe méthyle ;
ou bien encore R⁴ représente un radical de formule 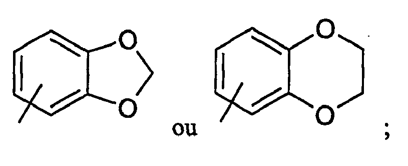
ou un sel d'un tel composé.

6. A titre de médicament, un composé de formule générale **(I)** selon la revendication 1 ou un sel pharmaceutiquement acceptable d'un tel composé.

7. Composition pharmaceutique comprenant, à titre de principe actif, un composé de formule générale **(I)** selon la revendication 1 ou un sel pharmaceutiquement acceptable d'un tel composé, avec au moins un excipient pharmaceutiquement acceptable.

8. Utilisation d'un composé de formule générale **(I)** selon la revendication 1, ou d'un sel pharmaceutiquement acceptable d'un tel composé, pour préparer un médicament destiné à traiter_ une maladie ou un désordre choisi parmi les maladies suivantes ou les désordres suivants : les maladies prolifératives tumorales, les maladies prolifératives non tumorales, les maladies neurodégénératives, les maladies parasitaires, les infections virales, l'alopécie spontanée, l'alopécie induite par des produits exogènes, l'alopécie radio-induite, les maladies auto-immunes, les rejets de greffes, les maladies inflammatoires ou les allergies.

9. Utilisation selon la revendication 8, **caractérisée en ce que** le médicament préparé est destiné à traiter le cancer.

10. Utilisation selon la revendication 9, **caractérisée en ce que** le cancer destiné à être traité est choisi parmi le cancer, et notamment le cancer du sein, les lymphomes, les cancers du cou ou de la tête, le cancer du poumon, le cancer du colon, le cancer de la prostate ou le cancer du pancréas.

## Claims

1. Compound corresponding to general formula (I) 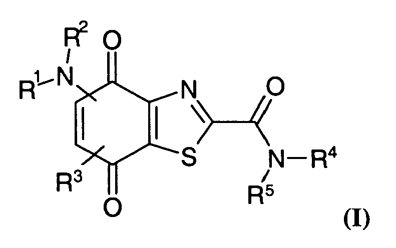 in racemic, enantiomeric form or any combination of these forms, in which:
R¹ represents a hydrogen atom or an alkyl, alkoxyalkyl, alkylthioalkyl, cycloalkyl, -(CH₂)-X-Y, -(CH₂)-Z-NR⁶R⁷ radical or a-CHR⁸R⁹ radical,
X representing a bond or a linear or branched alkylene radical containing 1 to 5 carbon atoms,
Y representing a saturated cyclic carbon system containing 1 to 3 condensed rings chosen independently from rings with 3 to 7 members, or Y representing a saturated heterocycle containing 1 to 2 heteroatoms chosen independently from O, N and S and attached to the X radical by an N or CH member, said saturated heterocycle moreover containing 2 to 6 additional members chosen independently from -CHR¹⁰-, -CO-, -NR¹¹-, -O- and -S-, R¹⁰ representing a hydrogen atom or an alkyl radical and R¹¹ representing a hydrogen atom or an alkyl or aralkyl radical, or also Y representing a carbocyclic or heterocyclic aryl radical optionally substituted 1 to 3 times by substituents chosen independently from the group constituted by a halogen atom, an alkyl radical, a haloalkyl radical, an alkoxy radical, a haloalkoxy radical, a hydroxy radical, a nitro radical, a cyano radical, the phenyl radical, an SO₂NHR¹² radical and an NR¹³R¹⁴ radical, R¹² representing a hydrogen atom or an alkyl or phenyl radical, and R¹³ and R¹⁴ independently representing alkyl radicals,
Z representing a bond or a linear or branched alkylene radical containing 1 to 5 carbon atoms,
R⁶ and R⁷ being chosen independently from a hydrogen atom, an alkyl, aralkyl or -(CH₂)ₙ-OH radical in which n represents an integer from 1 to 6,
or R⁶ representing an alkoxycarbonyl, haloalkoxycarbonyl or aralkoxycarbonyl radical and R⁷ representing a hydrogen atom or a methyl radical,
or also R⁶ and R⁷ forming together with the nitrogen atom a heterocycle with 4 to 7 members comprising 1 to 2 heteroatoms, the members necessary for completing the heterocycle being chosen independently from the -CR¹⁵R¹⁶-, -O-, -S- and -NR¹⁷- radicals, R¹⁵ and R¹⁶ independently representing each time that they occur a hydrogen atom or an alkyl radical, and R¹⁷ representing a hydrogen atom or an alkyl or aralkyl radical, or also R¹⁷ representing a phenyl radical optionally substituted 1 to 3 times by substituents chosen independently from a halogen atom and an alkyl or alkoxy radical,
R⁸ and R⁹ forming together with the carbon atom which carries them an indanyl or tetralinyl radical, or also R⁸ and R⁹ forming together with the carbon atom which carries them a saturated heterocycle containing 5 to 7 members and 1 to 2 heteroatoms chosen from O, N and S, the nitrogen atoms of said heterocycle being optionally substituted by radicals chosen from the alkyl radicals and the benzyl radical;
R² representing a hydrogen atom or an alkyl or aralkyl radical;
or also R¹ and R² forming together with the nitrogen atom a heterocycle with 4 to 8 members comprising 1 to 2 heteroatoms, the members necessary for completing the heterocycle being chosen independently from the -CR¹⁸R¹⁹-, -O-, -S- and -NR²⁰- radicals, R¹⁸ and R¹⁹ independently representing each time that they occur a hydrogen atom or an alkyl radical, and R²⁰ representing a hydrogen atom or an alkyl or aralkyl radical;
R³ represents a hydrogen atom or a halogen atom;
R⁴ represents an alkyl radical, a haloalkyl radical, a cycloalkyl radical, a cycloalkylalkyl radical, an alkoxyalkyl radical, one of the carbocyclic or heterocyclic aryl radicals or carbocyclic or heterocyclic aralkyl radicals the aryl nucleus of which is optionally substituted 1 to 3 times by substituents chosen independently from the group constituted by a halogen atom, an alkyl radical, an alkoxy radical, a haloalkyl radical and an -SO₂-NH₂ radical,
or also R⁴ represents one of the -(CH₂)ₘ-[O-(CH₂)ₚ]_{q}-O-Alk, -(CH₂)ᵣ-[O-(CH₂)ₛ]ₜ-NR²¹R²² or -(CH₂)ᵥ-A radicals in which m, p and s are each independently an integer from 2 to 4, q is an integer from 1 to 4, t is an integer from 0 to 4, r is an integer from 2 to 12 and v is an integer from 1 to 12, Alk is an alkyl radical, R²¹ is a hydrogen atom or an alkyl, alkoxycarbonyl or aralkoxycarbonyl radical, R²² is a hydrogen atom or an alkyl radical and A is a saturated heterocycle containing 1 to 2 heteroatoms chosen independently from O, N and S and attached to the -(CH₂)ᵥ- group by an N or CH member, said saturated heterocycle containing moreover 2 to 6 additional members chosen independently from -CHR²³-, -CO-, -NR²⁴-, -O- and -S-, R²³ representing a hydrogen atom or an alkyl radical and R²⁴ representing a hydrogen atom, an alkyl radical or an alkoxycarbonyl or aralkoxycarbonyl group,
or also R⁴ represents a radical of formula 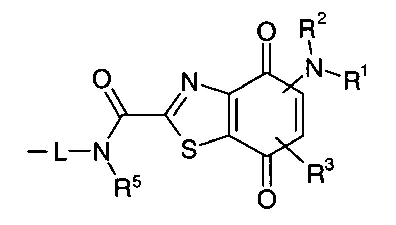 in which R¹, R², R³ and R⁵ are identical to the R¹, R², R³ radicals mentioned previously and R⁵ mentioned hereafter, and L is chosen from the -(CH₂)_{g}-[O-(CK₂)_{w}]ₓ-[O-(CH₂)_{y}]_{z}- and -(CH₂)ₐ-Ω-(CH₂)_{b}-radicals in which g, w and y are integers from 2 to 4, x is an integer from 1 to 3 and z is 0 or 1, a and b are independently integers from 2 to 6 and Ω is chosen from the group constituted by -O-, -S-, -NR²⁵-, -CO-, -CO-NR²⁶-, -CR²⁷R²⁸-, a cycloalkylene radical containing 3 to 7 carbon atoms and finally a carbocyclic aryl radical, R²⁵ representing an alkyl radical, R²⁶ representing a hydrogen atom or a methyl radical, R²⁷ and R²⁸ each being chosen independently from a hydrogen atom and a methyl group;
or also R⁴ represents a radical of formula 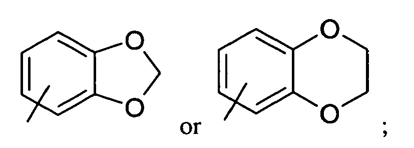
R⁵ represents a hydrogen atom or an alkyl or aralkyl radical, R⁵ also being able to represent a radical identical to R⁴ when R⁴ represents a alkyl, haloalkyl, alkoxyalkyl or carbocyclic or heterocyclic aralkyl radical, the aryl nucleus of which is optionally substituted 1 to 3 times by substituents chosen independently from the group constituted by a halogen atom, an alkyl radical, an alkoxy radical, a haloalkyl radical and an -SO₂-NH₂ radical;
or also R⁴ and R⁵ form together with the nitrogen atom which carries them a saturated heterocycle with 4 to 7 members comprising 1 to 2 heteroatoms in total, the members necessary for completing the heterocycle being chosen independently from the -CR²⁹R³⁰-, -O-, -S- and -NR³¹- radicals, R²⁹ and R³⁰ representing a hydrogen atom or an alkyl or aralkyl radical and R³¹ representing -COR³² or -SO₂R³³,
R³² representing an alkyl radical, a carbocyclic aryl radical optionally substituted 1 to 3 times by substituents chosen independently from the group constituted by a halogen atom, an alkyl radical and an alkoxy radical, or also R³² representing a heterocyclic aryl radical or a saturated heterocycle containing 5 to 7 members and 1 to 2 heteroatoms chosen independently from O, N and S,
R³³ representing a hydrogen atom or an alkyl radical,
or finally R⁴ and R⁵ form together with the nitrogen atom which carries them a heterocyclic aryl radical chosen from the radicals 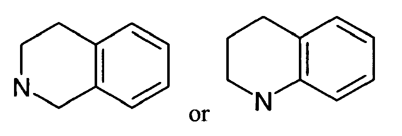 the aromatic ring of which can be substituted 1 to 3 times by substituents chosen independently from the group constituted by an alkyl radical and an alkoxy radical;
or salt of such a compound.

2. Compound according to claim 1, **characterized in that** R⁴ does not represent a radical of formula 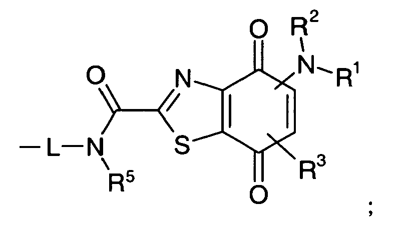 or salt of such a compound.

3. Compound according to claim 1, **characterized in that** R⁴ represents a radical of formula 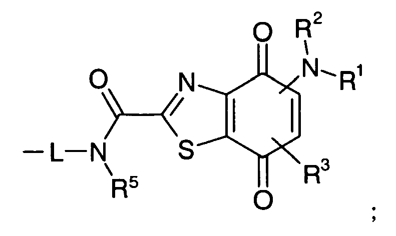 or salt of such a compound.

4. Compound according to claim 1, **characterized in that** it is chosen from the following compounds:
- 5-{[2-(dimethylamino)ethyl]amino}-2-(morpholin-4-ylcarbonyl)-1,3-benzothiazole-4,7-dione;
- *tert-*butyl {2-[2-(2-{[(5-{[2-(dimethylamino)ethyl]amino}-4,7-dioxo-4,7-dihydro-1,3-benzothiazol-2-yl)carbonyl]amino}ethoxy)ethoxy]ethyl}carbamate;
- *N,N*-dibenzyl-5-{[2-(dimethylamino)ethyl]amino}-4,7-dioxo-4,7-dihydro-1,3-benzothiazole-2-carboxamide;
- 5-{[2-(dimethylamino)ethyl]amino}-*N,N*-bis(2-methoxyethyl)-4,7-dioxo-4,7-dihydro-1,3-benzothiazole-2-carboxamide;
- 5-{[2-(dimethylamino)ethyl]amino}-4,7-dioxo-*N*-[3-(2-oxopyrrolidin-1-yl)propyl]-4,7-dihydro-1,3-benzothiazole-2-carboxamide;
- 4,7-dioxo-*N*-[3-(2-oxopyrrolidin-1-yl)propyl]-5-[(2-pyrrolidin-1-ylethyl)amino]-4,7-dihydro-1,3-benzothiazole-2-carboxamide;
- 5-{[2-(dimethylamino)ethyl]amino}-*N*-isobutyl-4,7-dioxo-4,7-dihydro-1,3-benzothiazole-2-carboxamide;
- *tert*-butyl (4-{[(5-{[2-(dimethylamino)ethyl]amino}-4,7-dioxo-4,7-dihydro-1,3-benzothiazol-2-yl)carbonyl]amino}butyl)carbamate;
- 5-{[2-(dimethylamino)ethyl]amino}-2-{[4-(2-furoyl)piperazin-1-yl]carbonyl}-1,3-benzothiazole-4,7-dione;
- 5-{[2-(dimethylamino)ethyl]amino}-*N*-(2-methoxyethyl)-4,7-dioxo-4,7-dihydro-1,3-benzothiazole-2-carboxamide;
- *N*-butyl-5-{[2-(dimethylamino)ethyl]amino}-4,7-dioxo-4,7-dihydro-1,3-benzothiazole-2-carboxamide;
- *N*-benzyl-5-{[2-(dimethylamino)ethyl]amino }-4,7-dioxo-4,7-dihydro-1,3-benzothiazole-2-carboxamide;
- *N*-(cyclohexylmethyl)-5-{[2-(dimethylamino)ethyl]amino}-4,7-dioxo-4,7-dihydro-1,3-benzothiazole-2-carboxamide;
- *N,N'*-(oxydiethane-2,1-diyl)bis(5-{[2-(dimethylamino)ethyl]amino}-4,7-dioxo-4,7-dihydro-1,3-benzothiazole-2-carboxamide);
- *N*,*N'*-[ethane-1,2-diylbis(oxyethane-2,1-diyl)]bis(5-{[2-(dimethylamino)ethyl]amino}-4,7-dioxo-4,7-dihydro-1,3-benzothiazole-2-carboxamide);
- 5-{[2-(dimethylamino)ethyl]amino}-2-{[4-(morpholin-4-ylcarbonyl)piperazin-1-yl]carbonyl }-1,3-benzothiazole-4,7-dione;
- 2-{[4-(morpholin-4-ylcarbonyl)piperazin-1-yl]carbonyl}-5-[(2-pyrrolidin-1-ylethyl)amino]-1,3-benzothiazole-4,7-dione;
- 5-{[2-(dimethylamino)ethyl]amino}-*N*-(4-methoxyphenyl)-4,7-dioxo-4,7-dihydro-1,3-benzothiazole-2-carboxamide;
- 5-{[2-(dimethylamino)ethyl]amino}-2-{[4-(methylsulphonyl)piperazin-1-yl]carbonyl}-1,3-benzothiazole-4,7-dione;
- 5-{[2-(dimethylamino)ethyl]amino}-2-{[4-(tetrahydrofuran-2-ylcarbonyl)piperazin-1-yl]carbonyl}-1,3-benzothiazole-4,7-dione;
- 6-bromo-5-{[2-(dimethylamino)ethyl]amino}-2-{[4-(tetrahydrofuran-2-ylcarbonyl)piperazin-1-yl]carbonyl}-1,3-benzothiazole-4,7-dione;
- 5-{[2-(dimethylamino)ethyl]amino}-2-(pyrrolidin-1-ylcarbonyl)-1,3-benzothiazole-4,7-dione;
- 5-{[2-(dimethylamino)ethyl]amino}-2-{[4-(2-thienylcarbonyl)piperazin-1-yl]carbonyl }-1,3-benzothiazole-4,7-dione;
- 5-{[2-(dimethylamino)ethyl]amino}-N-ethyl-4,7-dioxo-4,7-dihydro-1,3-benzothiazole-2-carboxamide;
- *N*-(4-chlorophenyl)-5-{[2-(dimethylamino)ethyl]amino}-4,7-dioxo-4,7-dihydro-1,3-benzothiazole-2-carboxamide;
- *N*-1,3-benzodioxol-5-yl-5-{[2-(dimethylamino)ethyl]amino}-4,7-dioxo-4,7-dihydro-1,3-benzothiazole-2-carboxamide;
- 5-[(2-methoxyethyl)amino]-2-{[4-(tetrahydrofuran-2-ylcarbonyl)piperazin-1-yl]carbonyl}-1,3-benzothiazole-4,7-dione;
- 5-(4-methylpiperazin-1-yl)-2-{[4-(tetrahydrofuran-2-ylcarbonyl)piperazin-1-yl]carbonyl}-1,3-benzothiazole-4,7-dione;
- 5-[(2-pyridin-2-ylethyl)amino]-2-({[4-(tetrahydrofuran-2-ylcarbonyl)piperazin-1-yl]carbonyl}-1,3-benzothiazole-4,7-dione;
- 5-[(3-morpholin-4-ylpropyl)amino]-2-{[4-(tetrahydrofuran-2-ylcarbonyl)piperazin-1-yl]carbonyl}-1,3-benzothiazole-4,7-dione;
- 5-[(1-benzylpyrrolidin-3-yl)amino]-2-{[4-(tetrahydrofuran-2-ylcarbonyl)piperazin-1-yl]carbonyl}-1,3-benzothiazole-4,7-dione;
- 5-[(2-cyclohexylethyl)amino]-2-{[4-(tetrahydrofuran-2-ylcarbonyl)piperazin-1-yl]carbonyl}-1,3-benzothiazole-4,7-dione;
- 5-{[2-(dimethylamino)ethyl]amino}-*N*-(4-fluorophenyl)-4,7-dioxo-4,7-dihydro-1,3-benzothiazole-2-carboxamide;
- 6-{ [2-(dimethylamino)ethyl]amino }-2-(pyrrolidin-1-ylcarbonyl)-1,3-benzothiazole-4,7-dione;
- 5-{[2-(dimethylamino)ethyl]amino}-*N*-(3-fluoro-4-methoxyphenyl)-4,7-dioxo-4,7-dihydro-1,3-benzothiazole-2-carboxamide;
- 5-{[2-(dimethylamino)ethyl]amino}-2-[(6-methoxy-3,4-dihydroquinolin-1(2*H*)-yl)carbonyl]-1,3-benzothiazole-4,7-dione;
- 5-{[2-(dimethylamino)ethyl]amino}-2-{[4-(4-methoxybenzoyt)piperazin-1-yl]carbonyl}-1,3-benzothiazole-4,7-dione;
- 5-[[2-(dimethylamino)ethyl](methyl)amino]-*N*-ethyl-4,7-dioxo-4,7-dihydro-1,3-benzothiazole-2-carboxamide;
- *N*-ethyl-5-{[2-(4-fluorophenyl)ethyl]amino}-4,7-dioxo-4,7-dihydro-1,3-benzothiazole-2-carboxamide;
or salt of such a compound.

5. Compound of general formula (A) 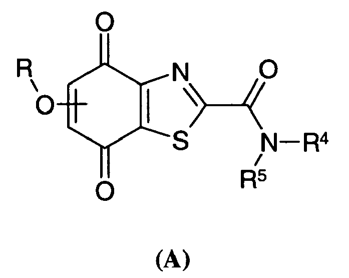 in which:
R is an alkyl radical;
R⁵ has the same meaning as in general formula (I) of claim 1;
and R⁴ represents an alkyl radical, a haloalkyl radical, a cycloalkyl radical, a cycloalkylalkyl radical, an alkoxyalkyl radical, one of the carbocyclic or heterocyclic aryl radicals or carbocyclic or heterocyclic aralkyl radicals the aryl nucleus of which is optionally substituted 1 to 3 times by substituents chosen independently from the group constituted by a halogen atom, an alkyl radical, an alkoxy radical, a haloalkyl radical and an -SO₂-NH₂ radical,
or also R⁴ represents one of the -(CH₂)ₘ-[O-(CH₂)ₚ]_{q}-O-Alk, -(CH₂)ᵣ-[O-(CH₂)ₛ]ₜ-NR²¹R²² or -(CH₂)ᵥ-A radicals in which m, p and s are each independently an integer from 2 to 4, q is an integer from 1 to 4, t is an integer from 0 to 4, r is an integer from 2 to 12 and v is an integer from 1 to 12, Alk is an alkyl radical, R²¹ is a hydrogen atom or an alkyl, alkoxycarbonyl or aralkoxycarbony radical, R²² is a hydrogen atom or an alkyl radical and A is a saturated heterocycle containing 1 to 2 heteroatoms chosen independently from O, N and S and attached to the group -(CH₂)ᵥ- group by an N or CH member, said saturated heterocycle moreover containing 2 to 6 additional members chosen independently from -CHR²³-, -CO-, -NR²⁴-, -O- and -S-, R²³ representing a hydrogen atom or an alkyl radical and R²⁴ representing a hydrogen atom, an alkyl radical or an alkoxycarbonyl or aralkoxycarbonyl group,
or also R⁴ represents a radical of formula 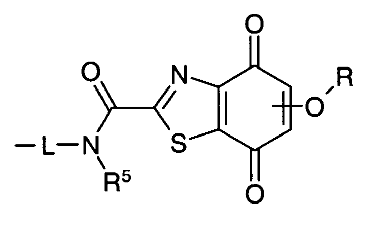
in which R and R⁵ are identical to the R and R⁵ radicals mentioned previously and L is chosen from the -(CH₂)_{g}-[O-(CH₂)_{w}]ₓ-[O-(CH₂)_{y}]_{z}- and -(CH₂)ₐ-Ω-(CH₂)_{b}- radicals in which g, w and y are integers from 2 to 4, x is an integer from 1 to 3 and z is 0 or 1, a and b are independently integers from 2 to 6 and Ω is chosen from the group constituted by -O-, -S-, -NR²⁵-, -CO-, -CO-NR²⁶-, -CR²⁷R²⁸-, a cycloalkylene radical containing 3 to 7 carbon atoms and finally a carbocyclic aryl radical, R²⁵ representing an alkyl radical, R²⁶ representing a hydrogen atom or a methyl radical, R²⁷ and R²⁸ each being chosen independently from a hydrogen atom and a methyl group;
or also R⁴ represents a radical of formula 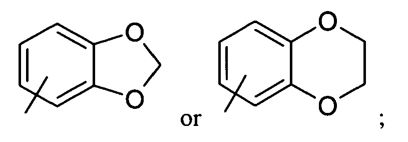
or a salt of such a compound.

6. As a medicament, a compound of general formula (I) according to claim 1 or a pharmaceutically acceptable salt of such a compound.

7. Pharmaceutical composition comprising, as active ingredient, a compound of general formula (I) according to claim 1 or a pharmaceutically acceptable salt of such a compound, with at least one pharmaceutically acceptable excipient.

8. Use of a compound of general formula (I) according to claim 1, or of a pharmaceutically acceptable salt of such a compound, for preparing a medicament intended to treat a disease or a disorder chosen from the following diseases or the following disorders: tumorous proliferative diseases, non-tumorous proliferative diseases, neurodegenerative diseases, parasitic diseases, viral infections, spontaneous alopecia, alopecia induced by exogenous products, radiation-induced alopecia, autoimmune diseases, transplant rejections, inflammatory diseases or allergies.

9. Use according to claim 8, **characterized in that** the medicament prepared is intended to treat cancer.

10. Use according to claim 9, **characterized in that** the cancer intended to be treated is chosen from cancer, and in particular breast cancer, lymphomas, cancers of the neck or head, lung cancer, cancer of the colon, cancer of the prostate or cancer of the pancreas.

## Patentansprüche

1. Verbindung, die der allgemeinen Formel (I) entspricht 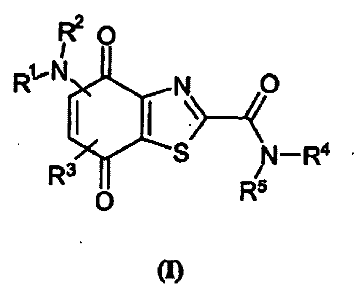 in racemischer Form, Enantiomerenform oder jeder Kombination dieser Formen, in der:
R¹ ein Wasserstoffatom oder einen der Reste Alkyl, Alkoxyalkyl, Alkylthioalkyl, Cycloalkyl, -(CH₂)-X-Y, -(CH₂)-Z-NR⁶R⁷ oder einen Rest -CHR⁸R⁹ darstellt,
X eine Bindung oder einen linearen oder verzweigten Alkylenrest mit 1 bis 5 Kohlenstoffatomen darstellt,
Y ein gesättigtes cyclisches Kohlenstoffsystem mit 1 bis 3 kondensierten Ringen darstellt, unabhängig voneinander aus Ringen von 3 bis 7 Gliedern ausgewählt, oder Y einen gesättigten Heterocyclus mit 1 bis 2 Heteroatomen, unabhängig voneinander aus O N und S ausgewählt, darstellt und an dem Rest X durch ein Glied N oder CH gebunden ist, wobei dieser gesättigte Heterocyclus außerdem 2 bis 6 zusätzliche Glieder zählt, unabhängig voneinander ausgewählt aus -CHR¹⁰-, -CO-, 1+NR¹¹-, -O- und -S-, wobei R¹⁰ ein Wasserstoffatom oder einen Alkylrest darstellt und R¹¹ ein Wasserstoffatom oder einen Alkyl- oder Aralkylrest darstellt, oder Y einen carbocyclischen oder heterocyclischen Arylrest darstellt, der gegebenenfalls 1 bis 3 mal mit Substituenten substituiert ist, unabhängig ausgewählt aus der Gruppe, die besteht aus einem Halogenatom, einem Alkylrest, einem Halogenalkylrest, einem Alkoxyrest, einem Halogenalkoxyrest, einem Hydroxyrest, einem Nitrorest, einem Cyanrest, dem Phenylrest, einem Rest SO₂NHR¹² und einem Rest NR¹³R¹⁴, wobei R¹² ein Wasserstoffatom oder einen Alkyl- oder Phenylrest darstellt und R¹³ und R¹⁴ unabhängig voneinander Alkylreste darstellen,
Z eine Bindung oder einen linearen oder verzweigten Alkylenrest mit 1 bis 5 Kohlenstoffatomen darstellt,
R⁶ und R⁷ unabhängig voneinander aus einem Wasserstoffatom, einem der Reste Alkyl, Aralkyl oder -(CH₂)ₙ-OH ausgewählt ist, in dem n eine ganze Zahl von 1 bis 6 darstellt,
oder R⁶ einen der Reste Alkoxycarbonyl, Halogenalkoxycarbonyl oder Aralkoxycarbonyl darstellt und R⁷ ein Wasserstoffatom oder einen Methylrest darstellt,
oder R⁶ und R⁷ zusammen mit dem Stickstoffatom einen Heterocyclus von 4 bis 7 Gliedern mit 1 bis 2 Heteroatomen bilden, wobei die zur Ergänzung des Heterocyclus erforderlichen Glieder unabhängig voneinander ausgewählt sind aus den Resten -CR¹⁵R¹⁶-, -O-, -S- und -NR¹⁷-, wobei R¹⁵ und R¹⁶ jeweils unabhängig voneinander bei jedem Auftreten ein Wasserstoffatom oder einen Alkylrest darstellen und R¹⁷ ein Wasserstoffatom oder einen Alkyl- oder Aralkylrest darstellt oder R¹⁷ einen Phenylrest darstellt, der gegebenenfalls 1 bis 3 mal mit Substituenten substituiert ist, unabhängig voneinander ausgewählt aus einem Halogenatom und einem Alkyl- oder Alkoxyrest,
R⁸ und R⁹ zusammen mit dem sie tragenden Kohlenstoffatom einen Indanyl- oder Tetralinylrest bilden oder R⁸ und R⁹ zusammen mit dem sie tragenden Kohlenstoffatom einen gesättigten Heterocyclus mit 5 bis 7 Gliedern und 1 bis 2 Heteroatomen bilden, ausgewählt aus O, N und S, wobei die Stickstoffatome dieses Heterocyclus gegebenenfalls mit Resten substituiert sind, ausgewählt aus den Alkylresten und dem Benzylrest;
R² ein Wasserstoffatom oder einen Alkyl- oder Aralkylrest darstellt;
oder R¹ und R² zusammen mit dem Stickstoffatom einen Heterocyclus von 4 bis 8 Gliedern mit 1 bis 2 Heteroatomen bilden, wobei die zur Ergänzung des Heterocyclus erforderlichen Glieder unabhängig voneinander ausgewahlt sind aus den Resten -CR¹⁸R¹⁹-, -O-, -S- und -NR²⁰-, wobei R¹⁸ und R¹⁹ jeweils unabhängig voneinander bei jedem Auftreten ein Wasserstoffatom oder einen Alkylrest darstellen und R²⁰ ein Wasserstoffatom oder einen Alkyl- oder Aralkylrest darstellt;
R³ ein Wasserstoffatom oder ein Halogenatom darstellt;
R⁴ darstellt einen Alkylrest, einen Halogenalkylrest, einen Cycloalkylrest, einen Cycloalkylalkylrest, einen Alkoxyalkylrest, einen der Reste carbocyclisches oder heterocyclisches Aryl oder carbocyclisches oder heterocyclisches Aralkyl, dessen Arylring gegebenenfalls 1 bis 3 mal mit Substituenten substituiert ist, unabhängig voneinander ausgewählt aus der Gruppe, die aus einem Halogenatom, einem Alkylrest, einem Alkoxyrest, einem Halogenalkylrest und einem Rest -SO₂-NH₂ besteht,
oder R⁴ einen der Reste -(CH2)ₘ-[O-(CH₂)ₚ]_{q}-O-Alk, -(CH₂)ᵣ-[O-(CH₂)ₛ]ₜ-NR²¹R²² oder -(CH₂)ᵥ-A darstellt, in denen m, p und s jeweils unabhängig voneinander eine ganze Zahl von 2 bis 4 sind, q eine ganze Zahl von 1 bis 4 ist, t eine ganze Zahl von 0 bis 4 ist, r eine ganze Zahl von 2 bis 12 ist und v eine ganze Zahl von 1 bis 12 ist, Alk ein Alkylrest ist, R²¹ ein Wasserstoffatom oder einer der Reste Alkyl, Alkoxycarbonyl oder Aralkoxycarbonyl ist, R²² ein Wasserstoffatom oder ein Alkylrest ist und A ein gesättigter Heterocyclus mit 1 bis 2 Heteroatomen ist, unabhängig voneinander ausgewählt aus O, N und S, der an die -(CH₂)ᵥ-Gruppe durch ein Glied N oder CH gebunden ist, wobei dieser gesättigte Heterocyclus ferner 2 bis 6 zusätzliche Glieder zählt, unabhängig voneinander ausgewählt aus -CHR²³-, -CO-, -NR²⁴-, -O- und -S-, wobei R²³ ein Wasserstoffatom oder einen Alkylrest darstellt und R²⁴ ein Wasserstoffatom, einen Alkylrest oder eine Alkoxycarbonyl- oder Aralkoxycarbonylgruppe darstellt,
oder R⁴ einen Rest der Formel darstellt 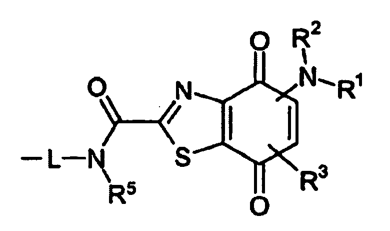
in der R¹ R², R³ und R⁵ mit den oben genannten Resten R¹ R², R³ und den im Nachstehenden genannten Resten R⁵ identisch sind und L ausgewählt ist aus den Resten -(CH₂)_{g}-[O-(CH₂)_{w}]ₓ[O-(CH₂)_{y}]_{z} und-(CH₂)ₐ-Ω-(CH₂)_{b}-, in denen g, w und y ganze Zahlen von 2 bis 4 sind, x eine ganze Zahl von 1 bis 3 ist und z 0 oder 1 ist, a und b unabhängig voneinander ganze Zahlen von 2 bis 6 sind und Ω aus der Gruppe ausgewählt ist, bestehend aus -O-, -S-, -NR²⁵-, -CO-, -CO-NR²⁶-, -CR²⁷R²⁸-, einem Cycloalkylenrest mit 3 bis 7 Kohlenstoffatomen und schließlich einem carbocyclischen Arylrest, wobei R²⁵ einen Alkylrest darstellt, R²⁶ ein Wasserstoffatom oder einen Methylrest darstellt, R²⁷ und R²⁸ jeweils unabhängig voneinander aus einem Wasserstoffatom und einer Methylgruppe ausgewählt sind;
oder R⁴ einen Rest der Formel darstellt 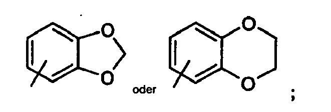
R⁵ ein Wasserstoffatom oder einen Alkyl- oder Aralkylrest darstellt, wobei R₅ auch einen mit R₄ identischen Rest darstellen kann, wenn R₄ einen der Reste Alkyl, Halogenalkyl, Alkoxyalkyl oder carbocyclisches oder heterocyclisches Aryl darstellt, dessen Arylring gegebenenfalls 1 bis 3 mal mit Substituenten substituiert ist, unabhängig voneinander ausgewählt aus der Gruppe, bestehend aus einem Halogenatom, einem Alkylrest, einem Alkoxyrest, einem Halogenalkylrest und einem Rest -SO₂-NH_{2;}
oder auch R⁴ und R⁵ zusammen mit dem sie tragenden Stickstoffatom einen gesättigten Heterocyclus von 4 bis 7 Gliedern mit insgesamt 1 bis 2 Heteroatomen bilden, wobei die zur Ergänzung des Heterocyclus erforderlichen Glieder unabhängig voneinander ausgewählt sind aus den Resten -CR²⁹R³⁰-, -O-, -S- und -NR³¹-, R²⁹ und R³⁰ ein Wasserstoffatom oder einen Alkyl- oder Aralkylrest darstellen und R³¹ -COR³² oder -SO₂R³³ darstellt,
R³² einen Alkylrest, einen carbocyclischen Arylrest darstellt, der gegebenenfalls 1 bis 3 mal mit Substituenten substituiert ist, unabhängig voneinander ausgewählt aus der aus einem Halogenatom, einem Alkylrest und einem Alkoxyrest bestehenden Gruppe, oder R₃₂ einen heterocyclischen Arylrest oder einen gesättigten Heterocyclus mit 5 bis 7 Gliedern und 1 bis 2 Heteroatomen darstellt, unabhängig ausgewählt aus O, N und S,
R³³ ein Wasserstoffatom oder einen Alkylrest darstellt,
oder schließlich R⁴ und R⁵ zusammen mit dem sie tragenden Stickstoffatom einen heterocyclischen Arylrest bilden, ausgewählt aus den Resten 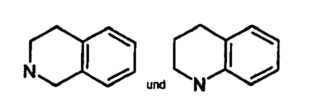
deren aromatischer Ring 1 bis 3 mal mit Substituenten substituiert sein kann, unabhängig voneinander ausgewählt aus der aus einem Alkylrest und einem Alkoxyrest bestehenden Gruppe;
oder ein Salz einer solchen Verbindung.

2. Verbindung nach Anspruch 1, **dadurch gekennzeichnet, dass** R⁴ nicht einen Rest der Formel darstellt 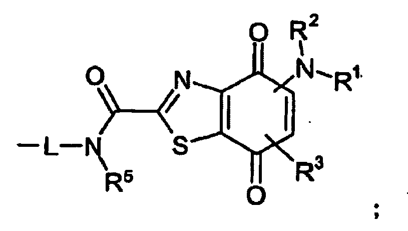 oder Salz einer solchen Verbindung.

3. Verbindung nach Anspruch 1, **dadurch gekennzeichnet, dass** R⁴ einen Rest der Formel 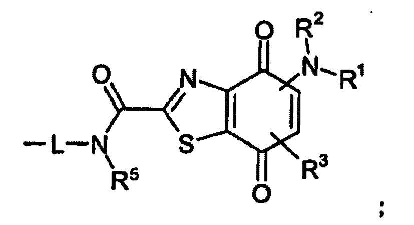 darstellt
oder ein Salz einer solchen Verbindung.

4. Verbindung nach Anspruch 1, **dadurch gekennzeichnet, dass** sie aus den folgenden Verbindungen ausgewählt ist:
- 5-{[2-(Dimethylamino)ethyl]amino}-2-(morpholin-4-ylcarbonyl)-1,3-benzothiazol-4,7-dion;
- {2-[2-(2-{[(5-{[2-(Dimethylamino)ethyl]amino}-4,7-dioxo-4,7-dihydro-1,3-benzothiazol-2-yl)carbonyl]amino}ethoxy)ethoxy]ethyl}tert.-butylcarbamat;
- N,N-Dibenzyl-5-{[2-(dimethylamino)ethyl]amino}-4,7-dioxo-4,7-dihydro-1,3-benzothiazol-2-carboxamid;
- 5-{[2-(Dimethylamino)ethyl]amino}-N,N-bis(2-methoxyethyl)-4,7-dioxo-4,7-dihydro-1,3-benzothiazol-2-carboxamid;
- 5-{[2-(Dimethylamino)ethyl]amino}-4,7-dioxo-N-[3-(2-oxopyrrolidin-1-yl)propyl]-4,7-dihydro-1,3-benzothiazol-2-carboxamid;
- 4,7-Dioxo-*N*-[3-(2-oxopyrrolidin-1-yl)propyl]-5-[(2-pyrrolidin-1-ylethyl)amino]-4,7-dihydro-1,3-benzothiazol-2-carboxamid;
- 5-{[2-(Dimethylamino)ethyl]amino}-*N*-isobutyl-4,7-dioxo-4,7-dihydro-1,3-benzothiazol-2-carboxamid;
- (4-{[(5-{[2-(Dimethylamino)ethyl]amino}-4,7-dioxo-4,7-dihydro-1,3-benzothiazol-2-yl)carbonyl]armino}butyl)tert.-butylcarbamat;
- 5-{[2-(Dimethylamino)ethyl]amino}-2-{[4-(2-furoyl)piperazin-1-yl]carbonyl}-1,3-benzothiazol-4,7-dion;
- 5-{[2-(Dimethylamino)ethyl]amino}-*N*-(2-methoxyethyl)-4,7-dioxo-4,7-dihydro-1,3-benzothiazol-2-carboxamid;
- *N*-Butyl-5-{[2-(dimethylamino)ethyl]amino}-4,7-dioxo-4,7-dihydro-1,3-benzothiazol-2-carboxamid;
- *N*-Benzyl-5-{[2-(dimethylamino)ethyl]amino}-4,7-dioxo-4,7-dihydro-1,3-benzothiazol-2-carboxamid;
- *N*-(Cyclohexylmethyl)-5-{[2-(dimethylamino)ethyl]amino}-4,7-dioxo-4,7-dihydro-1,3-benzothiazol-2-carboxamid;
- *N*,*N'*-(Oxydiethan-2,1-diyl)bis(5-{[2-(dimethylamino)ethyl]amino}-4,7-dioxo-4,7-dihydro-1,3-benzothiazol-2-carboxamid);
- *N*,*N'*-[Ethan-1,2-diylbis(oxyethan-2,1-diyl)]bis(5-{[2-(dimethylamino)ethyl]amino)-4,7-dioxo-4,7-dihydro-1,3-benzothiazol-2-carboxamid),
- 5-{[2-(Dimethylamino)ethyl]amino}-2-{[4-(morpholin-4-ylcarbonyl)piperazin-1-yl]carbonyl}-1,3-benzothiazol-4,7-dion;
- 2-{[4-(Morpholin-4-ylcarbonyl)piperazin-1-yl]carbonyl}-5-[(2-pyrrolidin-1-ylethyl)amino]-1,3-benzothiazol-4,7-dion;
- 5-{[2-(Dimethylamino)ethyl]amino}-*N*-(4-methoxyphenyl)-4,7-dioxo-4,7-dihydro-1,3-benzothiazol-2-carboxamid);
- 5-{[2-(Dimethylamino)ethyl]amino}-2-{[4-(methylsulfonyl)piperazin-1-yl]carbonyl}-1,3-benzothiazol-4,7-dion;
- 5-{[2-(Dimethylamino)ethyl]amino}-2-{[4-(tetrahydrofuran-2-ylcarbonyl)piperazin-1-yl]carbonyl}-1,3-benzothiazol-4,7-dion;
- 6-Brom-5-{[2-(dimethylamino)ethyl]amino}-2-{[4-(tetrahydrofuran-2-ylcarbonyl)piperazin-1-yl]carbonyl}-1,3-benzothiazol-4,7-dion;
- 5-{[2-(Dimethylamino)ethyl]amino}-2-(pyrrolidin-1-ylcarbonyl)-1,3-benzothiazol-4,7-dion;
- 5-{[2-(Dimethylamino)ethyl]amino}-2-{[4-(2-thienylcarbonyl)piperazin-1-yl]carbonyl}-1,3-benzothiazol-4,7-dion;
- 5-{[2-(Dimethylamino)ethyl]amino}-N-ethyl-4,7-dioxo-4,7-dihydro-1,3-benzothiazol-2-carboxamid;
- *N*-(4-Chlorophenyl)-5-{[2-(dimethylamino)ethyl]amino}-4,7-dioxo-4,7-dihydro-1,3-benzothiazol-2-carboxamid;
- *N*-1,3-Benzodioxol-5-yl-5-{[2-(dimethylamino)ethyl]amino}-4,7-dioxo-4,7-dihydro-1,3-benzothiazol-2-carboxamid;
- 5-[(2-Methoxyethyl)amino]-2-{[4-(tetrahydrofuran-2-ylcarbonyl)piperazin-1-yl]carbonyl}-1,3-benzothiazol-4,7-dion;
- 5-(4-Methylpiperazin-1-yl)-2-{[4-(tetrahydrofuran-2-ylcarbonyl)piperazin-1-yl]carbonyl}-1,3-benzothiazol-4,7-dion;
- 5-[(2-Pyridin-2-ylethyl)aminol-2-([4-(tetrahydrofuran-2-ylcarbonyl)piperazin-1-yl]carbonyl}-1,3-benzothiazol-4,7-dion;
- 5-[(3-Morpholin-4-ylpropyl)amino]-2-{[4-(tetrahydrofuran-2-ylcarbonyl)piperazin-1-yl]carbonyl}-1,3-benzothiazol-4,7-dion;
- 5-[(1-Benzylpyrrolidin-3-yl)amino]-2-{[4-(tetrahydrofuran-2-ylcarbonyl)piperazin-1-yl]carbonyl}-1,3-benzothiazol-4,7-dion;
- 5-[(2-Cyclohexylethyl)amino]-2-{[4-(tetrahydrofuran-2-ylcarbonyl)piperazin-1-yl]carbonyl}-1,3-benzothiazol-4,7-dion;
- 5-{[2-(Dimethylamino)ethyl]amino}-*N*-(4-fluorphenyl)-4,7-dioxo-4,7-dihydro-1,3-benzothiazol-2-carboxamid;
- 6-{[2-(Dimethylamino)ethyl]amino}-2-(pyrrolidin-1-ylcarbonyl)-1,3-benzothiazol-4,7-dion;
- 5-{[2-(Dimethylamino)ethyl]amino}-*N*-(3-fluoro-4-methoxyphenyl)-4,7-dioxo-4,7-dihydro-1,3-benzothiazol-2-carboxamid;
- 5-{[2-(Dimethylamino)ethyl]amino}-2-[(6-methoxy-3,4-dihydrochinolin-1(2*H*)-yl)carbonyl]-1,3-benzothiazol-4,7-dion;
- 5-{[2-(Dimethylamino)ethyl]amino}-2-{[4-(4-methoxybenzoyl)piperazin-1-yl]carbonyl}-1,3-benzothiazol-4,7-dion;
- 5-[[2-(Dimethylamino)ethyl](methyl)amino]-*N*-ethyl-4,7-dioxo-4,7-dihydro-1,3-benzothiazol-2-carboxamid;
- *N*-Ethyl-5-{[2-(4-fluorphenyl)ethyl]amino}-4,7-dioxo-4,7-dihydro-1,3-benzothiazol-2-carboxamid;
oder ein Salz einer solchen Verbindung.

5. Verbindung der allgemeinen Formel (A) 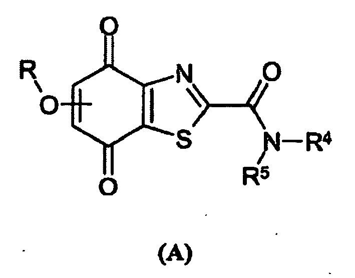 in der:
R ein Alkylrest ist;
R⁵ dieselbe Bedeutung wie in der allgemeinen Formel (1) von Anspruch 1 hat;
und R⁴ einen Alkylrest, einen Halogenalkylrest, einen Cycloalkylrest, einen Cycloalkylalkylrest, einen Alkoxyalkylrest, einen der Reste carbocyclisches oder heterocyclisches Aryl oder carbocyclisches oder heterocyclisches Aralkyl darstellt, dessen Arylring gegebenenfalls 1 bis 3 mal mit Substituenten substituiert ist, unabhängig voneinander ausgewählt aus der Gruppe, bestehend aus einem Halogenatom, einem Alkylrest, einem Alkoxyrest, einem Halogenalkylrest und einem Rest -SO₂-NH₂,
oder R⁴ einen der Reste -(CH2)ₘ-[O-(CH₂)ₚ]_{q}-O-Alk, -(CH₂)ᵣ-[O(-CH₂)ₛ]ₜ-NR²¹R²² oder -(CH₂)ᵥ-A darstellt, in denen m, p und s jeweils unabhängig voneinander einen ganze Zahl von 2 bis 4 sind, q eine ganze Zahl von 1 bis 4 ist, t eine ganze Zahl von 0 bis 4 ist, r eine ganze Zahl von 2 bis 12 ist und v eine ganze Zahl von 1 bis 12 ist, Alk ein Alkylrest ist, R²¹ ein Wasserstoffatom oder ein Alkyl-, Alkoxycarbonyl- oder A-ralkoxycarbonylrest ist, R²² ein Wasserstoffatom oder ein Alkylrest ist und A ein gesättigter Heterocyclus ist, der 1 bis 2 Heteroatome zählt, unabhängig voneinander ausgewählt aus O, N und S, und an die Gruppe -(CH₂)ᵥ- durch ein Glied N oder CH gebunden ist, wobei dieser gesättigte Heterocyclus ferner 2 bis 6 zusätzliche Glieder zählt, unabhängig voneinander ausgewählt aus -CHR²³-, -CO-, -NR²⁴-, -O- und -S-, wobei R²³ ein Wasserstoffatom oder einen Alkylrest darstellt und R²⁴ ein Wasserstoffatom, einen Alkylrest oder eine Alkoxycarbonyl- oder Aralkoxycarbonylgruppe darstellt,
oder R⁴ einen Rest der Formel 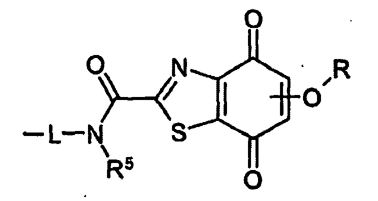
darstellt, in der R und R⁵ mit den oben genannten Resten R und R⁵ identisch sind und L aus den Resten -(CH₂)_{g}-[O-(CH₂)_{w}]ₓ-[O-(CH₂)_{y}]_{z}- und -(CH₂)ₐ-Ω-(CH₂)_{b}- ausgewählt ist, in denen g, w und y ganze Zahlen von 2 bis 4 sind, x eine ganze Zahl von 1 bis 3 ist und z 0 oder 1 ist, a und b unabhängig voneinander ganze Zahlen von 2 bis 6 sind und Ω aus der Gruppe ausgewählt ist, bestehend aus -O-, -S-, -NR²⁵-, -CO-, -CO-NR²⁶-, -CR²⁷R²⁸-, einem Cycloalkylenrest mit 3 bis 7 Kohlenstoffatomen und schließlich einem carbocyclischen Arylrest, wobei R²⁵ einen Alkylrest darstellt, R²⁶ ein Wasserstoffatom oder einen Methylrest darstellt, R²⁷ und R²⁸ jeweils unabhängig voneinander aus einem Wasserstoffatom und einer Methylgruppe ausgewählt sind;
oder auch R⁴ einen Rest der Formel 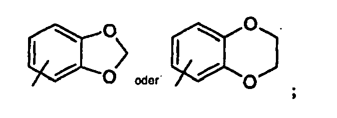
darstellt;
oder ein Salz einer solchen Verbindung.

6. Eine Verbindung der allgemeinen Formel (I) nach Anspruch 1 oder ein pharmazeutisch annehmbares Salz einer solchen Verbindung als Medikament.

7. Pharmazeutische Zusammensetzung, die als Wirkstoff eine Verbindung der allgemeinen Formel (I) nach Anspruch 1 oder ein pharmazeutisch annehmbares Salz einer solchen Verbindung mit mindestens einem pharmazeutisch annehmbaren Hilfsstoff umfasst.

8. Verwendung einer Verbindung der allgemeinen Formel (I) nach Anspruch 1 oder eines pharmazeutisch annehmbaren Salzes einer solchen Verbindung zur Herstellung eines Medikaments, das zur Behandlung einer Krankheit oder einer Störung bestimmt ist, die aus den folgenden Krankheiten oder Störungen ausgewählt ist: proliferative tumorale Krankheiten, proliferative nicht tumorale Krankheiten, neurodegenerative Krankheiten, parasitäre Krankheiten, virale Infektionen, spontane Alopezie, durch exogene Produkte induzierte Alopezie, strahleninduzierte Alopezie, Autoimmunkrankheiten, Abstoßungen von Transplantaten, entzündliche Krankheiten oder Allergien.

9. Verwendung nach Anspruch 8, **dadurch gekennzeichnet, dass** das hergestellte Medikament zur Behandlung von Krebs bestimmt ist.

10. Verwendung nach Anspruch 9, **dadurch gekennzeichnet, dass** der zu behandelnde Krebs ausgewählt ist aus Krebs, und zwar insbesondere Brustkrebs, Lymphonen, Hals- oder Kopfkrebs, Lungenkrebs, Colonkrebs, Prostatakrebs oder Pankreaskrebs.
